# FASCICULE DE BREVET EUROPEEN

(11) **EP 1 275 647 B1**
(45) Date de publication et mention de la délivrance du brevet: **12.11.2003**
(21) Numéro de dépôt: 02291745.4
(22) Date de dépôt: 11.07.2002
(51) Int. Cl.: C07D 471/04, A61K 31/5025, A61P 25/00

(54) **Dérivés d'octahydro-2H-pyrido[1,2-a]pyrazine, leur procédé de préparation et les compositions pharmaceutiques qui les contiennent**
Octahydro-2H-pyrido[1,2-a]pyrazinderivate, Verfahren zu ihrer Herstellung und diese enthaltende pharmazeutische Zubereitungen
Octahydro-2H-pyrido[1,2-a]pyrazine derivatives, process for their preparation and pharmaceutical compositions containing them

(30) Priorité: 12.07.2001 FR 0109260
(43) Date de publication de la demande: 15.01.2003
(73) Titulaire: Les Laboratoires Servier, 92415 Courbevoie Cedex (FR)
(72) Inventeur: Goldstein, Solo, 92150 Suresnes (FR); Poissonnet, Guillaume, 91400 Orsay (FR); Parmentier, Jean-Gilles, 92130 Issy les Moulineaux (FR); Lestage, Pierre, 78170 La Celle Saint Cloud (FR); Lockhart, Brian, 78290 Croissy sur Seine (FR)

(56) Documents cités:
- WO-A-01/05772
- DATABASE CA [en ligne] CHEMICAL ABSTRACTS SERVICE, COLUMBUS, OHIO, US; UESAKA, IKUO ET AL: "Diazabicycloalkanes. I. Pharmacological studies of octahydro-2H-pyrido[1,2-a]pyrazine derivatives and related compounds, especially their antihistaminic activities" retrieved from STN Database accession no. 78:66809 XP002192969 & YAKUGAKU ZASSHI (1972), 92(11), 1339-49,

## Description

La présente invention concerne de nouveaux dérivés d'octahydro-2*H*-pyrido [1,2-*a*]pyrazine, leur procédé de préparation et les compositions pharmaceutiques qui les contiennent.

Les composés de la présente invention sont particulièrement intéressants d'un point de vue pharmacologique pour leur interaction spécifique avec les récepteurs histaminergiques centraux de type H3, trouvant leur application dans le traitement des neuropathologies associées au vieillissement cérébral, des troubles de l'humeur, du comportement alimentaire et du rythme veille-sommeil, ainsi que du syndrome d'hyperactivité avec déficits attentionnels.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence.

Au niveau du système nerveux central, de récentes études neuropharmacologiques ont montré que l'histamine *via* les systèmes histaminergiques centraux jouait un rôle de neurotransmetteur ou neuromodulateur en situations physiologiques ou physiopathologiques (Annu. Rev. Neurosci., 1986, 9, 209-254; Physiol. Rev., 1991, 71, 1-51). Ainsi, il a été montré que l'histamine intervenait dans divers processus physiologiques et comportementaux tels que la thermorégulaton, la régulation neuro-endocrinienne, le rythme circadien, les états cataleptiques, la motricité, l'agressivité, le comportement alimentaire, l'apprentissage et la mémorisation, ainsi que la plasticité synaptique (Hass et coll., histaminergic neurones : morphology and function, Boca Roton, FL : CRC Press, 1991, pp. 196-208 ; Prog. Neurobiology, 2001, 63, 637-672).

Parmi les 3 sous-types de récepteurs (H1, H2 et H3) à l'histamine, il a été initialement montré que le récepteur de type H3 était un autorécepteur pré-synaptique qui contrôlait la libération de l'histamine (Nature, 1987, 327, 117-123). Son activation inhibe la libération et la synthèse d'histamine par un mécanisme de feedback négatif (Neuroscience, 1987, 23, 149-157). Par la suite, il a été montré l'existence d'hétérorécepteurs pré-synaptiques, capables de moduler la libération de quelques neuropeptides et de nombreux neurotransmetteurs tels que noradrénaline, sérotonine, dopamine, GABA, acétylcholine et glutamate (TiPS, 1998, 19, 177-183). Des études, réalisées chez l'animal, ont montré que l'augmentation des taux endogènes extra-synaptiques d'histamine *via* le blocage des récepteurs de type H3 par des antagonistes H3 permettait de promouvoir les états de vigilance, les processus d'apprentissage et de mémoire, de réguler la prise alimentaire, et de s'opposer à des crises convulsives (Prog. Neurobiol., 2000, 63, 637-672; Neurosci. Biobehav. Rev., 2000, 24, 107-113). En conséquence, les indications thérapeutiques potentielles pour des antagonistes H3 sont le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels, de l'obésité et de la douleur.

Plusieurs demandes de brevets ou brevets tels que JP 52012188, WO 94/06794, ou US 3,388,128 décrivent des composés comportant un motif octahydro-2*H*-pyrido[1,2-*a*] pyrazine. Ces composés sont revendiqués pour leur activité vasodilatatrice, ou pour leur utilité dans le traitement de l'hypotension, de l'ischémie cérébrale, des psychoses ou des convulsions. Aucuns de ces documents ne décrivent ou ne suggèrent pour ces composés une activité antagoniste des récepteurs histaminiques centraux H3, propriété originale des composés revendiqués par la Demanderesse.

Plus particulièrement, la présente invention concerne les composés de formule (I) : 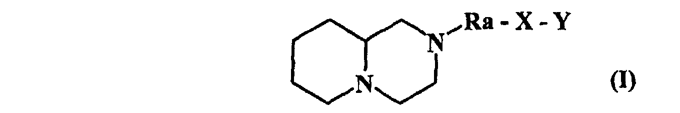 dans laquelle :
- **Ra** représente une chaîne alkylène (C₁-C₆) linéaire ou ramifié,
- **X** représente un groupement choisi parmi W₁, -C(W₁)-W₂-, -W₂-C(W₁)-, -W₂-C(W₁)W₂-, -W₂-Ra- avec Ra tel que défini précédemment, et -CH(OR₁)- dans lesquels :
   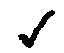 W₁ représente un atome d'oxygène, un atome de soufre, ou un groupement de formule -NR₂ dans laquelle R₂ représente un groupement choisi parmi atome d'hydrogène, alkyle (C₁-C₆) linéaire ou ramifié, aryle, arylalkyle (C₁-C₆) linéaire ou ramifié, et acyle (C₁-C₆) linéaire ou ramifié,
   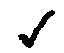 W₂ représente un groupement tel que défini pour W₁,
   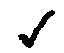 R₁ représente un groupement choisi parmi atome d'hydrogène et groupement alkyle (C₁-C₆) linéaire ou ramifié,
- lorsque **Y** représente un groupement aryle ou hétéroaryle,
   ou
- **X** représente un groupement choisi parmi liaison simple,-C(W₁)-, -W₂-C(W₁)-, -W₂-Ra-, et -CH(OR₁)- dans lesquels W₁, W₂, Ra et R₁ sont tels que définis précédemment,
- lorsque **Y** représente un groupement bicyclique fusionné, de formule : 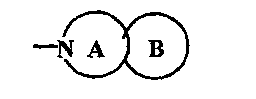 dans laquelle :
   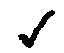 A représente un hétérocycle azoté de 4 à 7 chaînons, insaturé ou partiellement saturé, contenant éventuellement un second hétéroatome choisi parmi oxygène, azote et soufre, et éventuellement substitué par un ou plusieurs groupements choisis parmi oxo et alkyle (C₁-C₆) linéaire ou ramifié,
   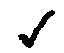 B représente un cycle phényle éventuellement substitué par un ou plusieurs groupements choisis parmi atomes d'halogènes, groupements nitro, cyano, hydroxy, alkoxy (C₁-C₆) linéaire ou ramifié, alkyle (C₁-C₆) linéaire ou ramifié, trihalogénoalkyle (C₁-C₆) linéaire ou ramifié, acylc (C₁-C₆) linéaire ou ramifié, acyl(C₁-C₆)oxy linéaire ou ramifié, carboxy, alkoxy (C₁-C₆) carbonyle linéaire ou ramifié, sulfanyle, alkylsulfanyle (C₁-C₆) linéaire ou ramifié, et amino éventuellement substitué par un ou deux groupements, identiques ou différents, choisis parmi alkyle (C₁-C₆) linéaire ou ramifié, aryle, et arylalkyle (C₁-C₆) linéaire ou ramifié,
leurs énantiomères, diastéréoisomères , ainsi que leurs sels d'addition à un acide ou à une base pharmaceutiquement acceptable,
étant entendu que les composés :
- 2-octahydro-2*H*-pyrido[1,2-a]pyrazin-2-yl-1-phényléthanol,
- 3-octahydro-2*H*-pyrido[1,2-a]pyrazine-2-ylpropyl-3,4,5-triméthoxybenzoate,
- et 2-octahydro-2*H*-pyrido[1,2-a]pyrazine-2-yléthyl-3,4,5-triméthoxybenzoate, ne font pas partie des composés de l'invention.

Par groupement aryle, on comprend un système aromatique monocyclique ou bicyclique, de 5 à 10 atomes de carbone, éventuellement substitué par un ou plusieurs groupements, identiques ou différents, indépendamment l'un de l'autre, choisis parmi atomes d'halogène, groupements nitro, cyano, hydroxy, alkoxy (C₁-C₆) linéaire ou ramifié, aryloxy, arylalkoxy (C₁-C₆) linéaire ou ramifié, alkyle (C₁-C₆) linéaire ou ramifié, alkényle (C₂-C₆) linéaire ou ramifié, alkynyle (C₂-C₆) linéaire ou ramifié, trihalogénoalkyle (C₁-C₆) linéaire ou ramifié, carboxy, alkoxy(C₁-C₆)carbonyle linéaire ou ramifié, acyle (C₁-C₆) linéaire ou ramifié, acyl(C₁-C₆)oxy linéaire ou ramifié, sulfanyle, alkylsulfanyle (C₁-C₆) linéaire ou ramifié, méthylènedioxy, éthylènedioxy, amide-oxime et amino éventuellement substitué par un ou deux groupements, identiques ou différents, indépendamment l'un de l'autre, choisis parmi alkyle (C₁-C₆) linéaire ou ramifié, aryle, arylalkyle (C₁-C₆) linéaire ou ramifié, et acyle (C₁-C₆) linéaire ou ramifié.

Par groupement hétéroaryle, on comprend un système aromatique monocyclique ou bicyclique, de 5 à 12 chaînons, contenant au sein du système cyclique, de 1 à 3 hétéroatomes, identiques ou différents, indépendamment l'un de l'autre, choisis parmi atome d'oxygène, d'azote et de soufre, chacun de cesdits systèmes étant éventuellement substitués par un ou plusieurs groupements, identiques ou différents, indépendamment l'un de l'autre, choisis parmi la liste des substituants précédemment décrits dans le cas d'un groupement aryle.

Par isomère, on comprend les isomères optiques, les énantiomères et les diastéréoisomères.

Parmi les groupements aryles, on peut citer, à titre indicatif, le groupement phényl, naphtyle, et benzocyclobutyle.

Parmi les groupements hétéroaryles, on peut citer, à titre indicatif, le groupement furyle, thiényle, pyrrolyle, pyrazolyle, imidazolyle, oxazolyle, pyridyle, pyrimidinyle, pyrazinyle, benzothiényle, benzofuryle, indolyle, quinolyle, isoquinolyle, ...

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) dans laquelle Ra représente une chaîne alkylène (C₂-C₅) linéaire.

Les substituants R₂ préférés selon l'invention sont l'atome d'hydrogène, le groupement alkyle (C₁-C₆) linéaire ou ramifié, et acyle (C₁-C₆) linéaire ou ramifié.

Selon une variante avantageuse de l'invention, les composés préférés sont les composés de formule (IA) : 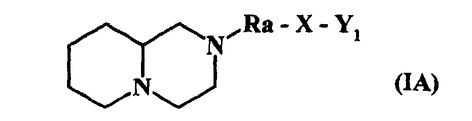 dans laquelle :
- **Ra** représente une chaîne alkylène (C₁-C₆) linéaire ou ramifié,
- **X** représente un groupement choisi parmi W₁, -C(W₁)-W₂-, -W₂-C(W₁)-, -W₂-C(W₁)W₂-, -W₂-Ra- avec Ra tel que défini précédemment, et -CH(OR₁)- dans lesquels :
   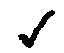 W₁ représente un atome d'oxygène, un atome de soufre, ou un groupement de formule -NR₂ dans laquelle R₂ représente un groupement choisi parmi atome d'hydrogène, alkyle (C₁-C₆) linéaire ou ramifié, aryle, arylalkyle (C₁-C₆) linéaire ou ramifié, et acyle (C₁-C₆) linéaire ou ramifié,
   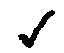 W₂ représente un groupement tel que défini pour W₁,
   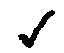 R₁ représente un groupement choisi parmi atome d'hydrogène et groupement alkyle (C₁-C₆) linéaire ou ramifié,
- **Y**_{**1**} représente un groupement aryle ou hétéroaryle.

Les substituants X préférés selon l'invention dans les composés de formule (IA) sont les groupements choisis parmi atome d'oxygène, groupements -C(W₁)-W₂-, -W₂-C(W₁)-, et -N(R₂)- dans lesquels W₁ représente un atome d'oxygène, W₂ représente un atome d'oxygène ou un groupement -NR₂, R₂ étant tel que défini dans la formule (IA).

Le substituant Y₁ préférés selon l'invention est le groupement phényle éventuellement substitué par 1 à 3 groupements choisis parmi halogène, cyano, hydroxy, alkoxy (C₁-C₆) linéaire ou ramifié, aryloxy, arylalkoxy (C₁-C₆) linéaire ou ramifié, alkyle (C₁-C₆) linéaire ou ramifié, trihalogénoalkyle (C₁-C₆) linéaire ou ramifié, méthylènedioxy et éthylènedioxy.

Selon une seconde variante avantageuse de l'invention, les composés préférés sont les composés de formule (IB) : 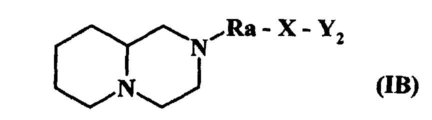 dans laquelle :
- **Ra** représente une chaîne alkylène (C₁-C₆) linéaire ou ramifiée,
- **X** représente un groupement choisi parmi liaison simple, -C(W₁)-, -W₂-C(W₁)-, -W₂-Ra- et -CH(OR₁)- dans lesquels W₁, W₂, Ra et R₁ sont tels que définis dans la formule (I),
- **Y**_{**2**} représente un groupement de formule : 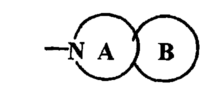 dans laquelle A et B sont tels que définis dans la formule (I).

Les groupement Y₂ préférés sont les groupements de formule : 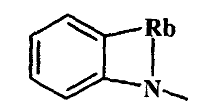 dans laquelle Rb représente une chaîne linéaire saturée ou insaturée, contenant 2 ou 3 atomes choisis parmi carbone, azote et oxygène, et/ou contenant éventuellement un groupement carbonyle.

Plus particulièrement, les groupements Y₂ préférés selon l'invention sont les groupements 2,3-dihydro-1*H*-indol-1-yl, 1*H*-indol-1-yl, 1*H*-indazol-1-yl, 1*H*-benzimidazol-1-yl, 3,4-dihydro-2*H*-quinolin-1-yl, 2,3-dihydro-1*H*-4-quinolinone-1-yl, 3,4-dihydro-2*H*-quinoxalin-1-yl, et 2,3-dihydro-4*H*-1,4-benzoxazin-4-yl.

Le substituant X préféré selon l'invention dans les composés de formule (IB) est la définition liaison simple.

Par énantiomères α et énantiomères β, on entend les isomères optiques du mélange racémique correspondant.

Les composés préférés de l'invention sont le :
- 2-[4-(2,3-dihydro-1*H*-indol-1-yl)butyl]octahydro-2*H*-pyrido[1,2-*a*] pyrazine,
- 2-[4-(3,4,5-triméthoxyphénoxy)butyl]octahydro-2*H*-pyrido[1,2-*a*] pyrazine,
- 2-[4-(3,4,5-triméthoxyphénoxy)butyl]octahydro-2*H*-pyrido[1,2-*a*] pyrazine, énantiomère α,
- 2-[4-(3,4,5-triméthoxyphénoxy)butyl]octahydro-2*H*-pyrido[1,2-*a*] pyrazine, énantiomère β,
- *N*-(4-octahydro-2*H*-pyrido[1,2-*a*]pyrazin-2-ylbutyl)-*N*-(3,4,5-triméthoxyphényl)amine,
- *N*-(4-octahydro-2*H*-pyrido[ 1,2-*a*]pyrazin-2-ylbutyl)-*N*-(3,4,5-triméthoxyphényl)amine, énantiomère α,
- *N*-(4-octahydro-2*H*-pyrido[1,2-*a*]pyrazin-2-ylbutyl)-*N*-(3,4,5-triméthoxyphényl)amine, énantiomère β,
- *N*-(4-trifluorophényl)-*N*-(4-octahydro-2*H*-pyrido[1,2-*a*]pyrazin-2-ylbutyl)amine,
- *N*-(3,4-dichloro-phényl)-*N*-(4-octahydro-2*H*-pyrido[1,2-*a*]pyrazin-2-ylbutyl)amine,
- *N*-(3,5-dichloro-phényl)-*N*-(4-octahydro-2*H*-pyrido[1,2-*a*]pyrazin-2-ylbutyl)amine,
- *N*-(2-chlorophényl)-*N*-(4-octahydro-2*H*-pyrido[1,2-*a*]pyrazin-2-ylbutyl)amine,
- 2-[4-(3,4-dihydro-1(2*H*)-quinolinyl)butyl]octahydro-2*H*-pyrido[1,2-*a*] pyrazine,
- 2 - [4-(1*H*-benzimidazol-1-yl)butyl ]octahydro-2*H*-pyrido[1,2-*a*] pyrazine,
- 2-[4-(1*H*-indol-1-yl)butyl]octahydro-2*H*-pyrido[1,2-*a*]pyrazine,
- 2-[4-(1*H*-indazol-1-yl)butyl]octahydro-2*H*-pyrido[1,2-*a*]pyrazine,
- 2-[4-(2,3-dihydro-1*H*-indol-1-yl)butyl]octahydro-2*H*-pyrido[1,2-*a*]pyrazine, énantiomère α,
- 2-[4-(2,3-dihydro-1*H*-indol-1-yl)butyl] octahydro-2*H*-pyrido[1,2-*a*]pyrazine, énantiomère β,
- 3-(4-octahydro-2*H*-pyrido[1,2-*a*]pyrazin-2-ylbutoxy)benzonitrile,
- 3-methoxy-4-(4-octahydro-2*H*-pyrido[1,2-*a*]pyrazin-2-ylbutoxy) benzonitrile,
- 2-[4-(2,3,4-trimethoxyphenoxy)butyl]octahydro-2*H*-pyrido[1,2-*a*] pyrazine.

De manière encore plus préférentielle, les composés de l'invention sont le :
- 4-[(4-octahydro-2*H*-pyrido[1,2-*a*]pyrazin-2-ylbutyl)amino]benzonitrile,
- 4-(3-octahydro-2*H*-pyrido[1,2-*a*]pyrazin-2-ylpropoxy)benzonitrile,
- 4-(3-octahydro-2*H*-pyrido[1,2-*a*]pyrazin-2-ylpropoxy)benzonitrile, énantiomère α,
- 4-(3-octahydro-2*H*-pyrido[1,2-*a*]pyrazin-2-ylpropoxy)benzonitrile, énantiomère β,
- 4-(4-octahydro-2*H*-pyrido[1,2-*a*]pyrazin-2-ylbutoxy)benzonitrile,
- 4-(4-octahydro-2*H*-pyrido[1,2-*a*]pyrazin-2-ylbutoxy)benzonitrile, énantiomère α,
- 4-(4-octahydro-2*H*-pyrido[1,2-*a*]pyrazin-2-ylbutoxy)benzonitrile, énantiomère β,
- 4-(2-octahydro-2*H*-pyrido[1,2-*a*]pyrazin-2-yléthoxy)benzonitrile.

Les énantiomères, diastéréoisomères, ainsi que les sels d'addition, à un acide ou à une base pharmaceutiquement acceptable des composés préférés, font partie intégrante de l'invention.

La présente invention s'étend également au procédé de préparation des composés de formule (I) caractérisé en ce qu'on utilise comme produit de départ un composé de formule (II) : 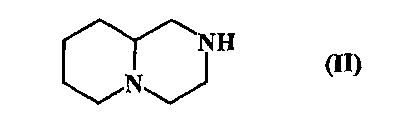 composé de formule (II) qui est mis à réagir, en condition basique :
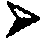 *soit avec un composé de formule (III)* : 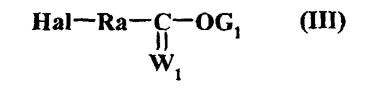 dans laquelle Hal représente un atome d'halogène, Ra est tel que défini dans la formule (I) et G₁ représente un groupement alkylc (C₁-C₆) linéaire ou ramifié,
   pour conduire aux composés de formule (IV) : 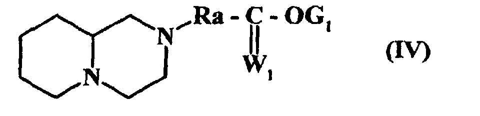 dans laquelle Ra et G₁ sont tels que définis précédemment,
   composés de formule (IV) qui sont saponifiés puis qui sont mis à réagir en présence d'hydrochlorure de 1-(3-diméthylaminopropyl)-3-éthylcarbodiimide, et d'une amine tertiaire, avec un composé de formule (V) :

   **Y**_{**1**}**-W**_{**2**}**H (V)**

   dans laquelle Y₁ représente un groupement aryle, ou hétéroaryle, et W₂ est tel que défini dans la formule (I)
   pour conduire aux composés de formule (i/a), cas particulier des composés de formule (I) : 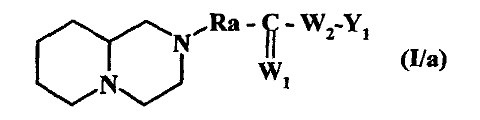 dans laquelle Ra, W₁, W₂ et Y₁ sont tels que définis précédemment,
   composés de formule (I/a), dans le cas particulier où W₁ représente un atome d'oxygène et Ra prend la définition particulière R'a et représente un chaîne alkylène (C₁-C₅) linéaire ou ramifiée, qui peuvent être réduits de façon sélective par action d'un réducteur classique utilisé en synthèse organique, pour conduire aux composés de formule (I/b), cas particulier des composés de formule (I) : 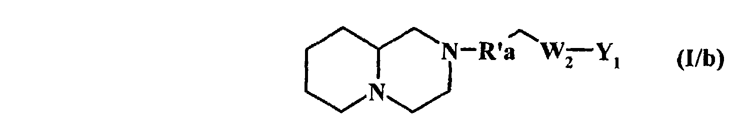 dans laquelle R'a représente une chaîne alkylène (C₁-C₅) linéaire ou ramifiée, W₂ et Y₁ sont tels que définis précédemment,
   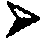 *soit avec un composé de formule (VI) :*

      **Hal-Ra**_{**1**}**-CN (VI)**

      dans laquelle Hal représente un atome d'halogène, et Ra₁ représente un groupement alkyle (C₁-C₅) linéaire ou ramifié ou une liaison,
   pour conduire aux composés de formule (VII) : 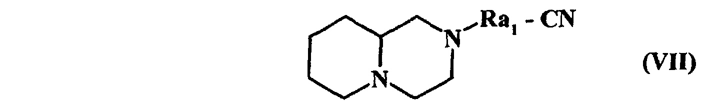 dans laquelle Ra₁ est tel que défini précédemment,
   composés de formule (VII) dont la fonction cyano est réduite selon des conditions classiques en amine primaire, puis qui sont traités :
   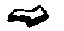 soit dans l'éthanol en présence d'un composé de formule (VIII) :

      **Y**_{**1**}**-N=C=W**_{**3**} **(VIII)**

      dans laquelle Y₁ est tel que défini précédemment et W₃ représente un atome d'oxygène ou de soufre,
      pour conduire aux composés de formule (I/c), cas particulier des composés de formule (I) : 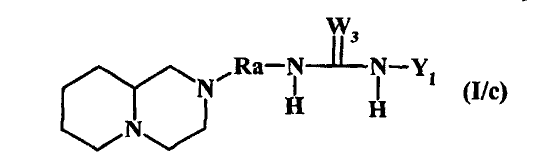 dans laquelle Ra, Y₁ et W₃ sont tels que définis précédemment,
   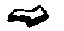 soit en condition de couplage par un composé de formule (IX) : 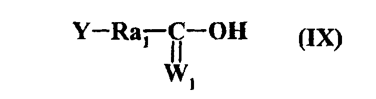 dans laquelle Y et W₁ sont tels que définis dans la formule (I) et Ra₁ est tel que défini précédemment,
   pour conduire :
   - dans le cas où Ra₁ représente unc liaison, aux composés de formule (I/d), cas particulier des composés de formule (I) : 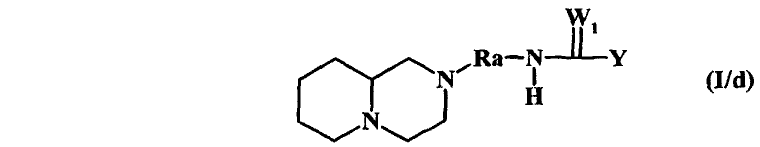 dans laquelle Ra, W₁ et Y sont tels que définis précédemment,
      composés de formule (I/d) qui sont traités en présence d'hydrure de sodium avec un composé de formule (X) :

      **R'**_{**2**}**-Hal (X)**

      dans laquelle Hal représente un atome d'halogène et R'₂ a la même définition que R₂ dans la formule (I) à l'exception de la définition atome d'hydrogène,
      pour conduire aux composés de formule (I/e), cas particulier des composés de formule (I) : 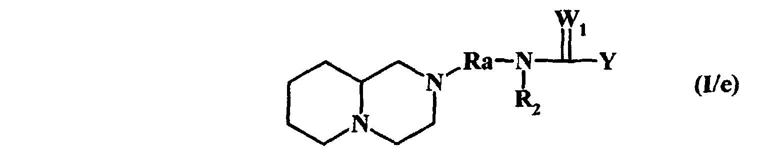 dans laquelle Ra, R₂, W₁ et Y sont tels que définis précédemment,
   - soit dans le cas où Ra₁ représente une chaîne alkylène (C₁-C₅) linéaire ou ramifiée, aux composés de formule (XI) : 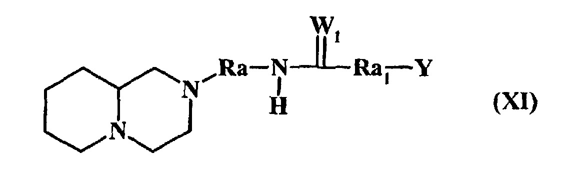 composés de formule (XI) qui sont placés en présence d'un agent réducteur classiquement utilisé en synthèse organique, pour conduire aux composés de formule (I/f), cas particulier des composés de formule (I) : 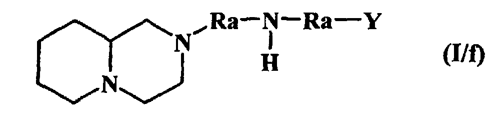 dans laquelle Ra et Y sont tels que définis dans la formule (I),
      composés de formule (I/f) qui peuvent être soumis à l'action d'un composé de formule (X) tel que décrit précédemment, pour conduire aux composés de formule (I/g), cas particulier des composés de formule (I) : 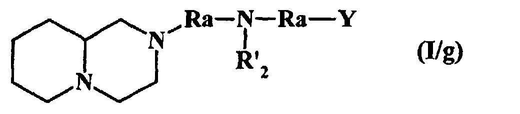 dans laquelle Ra, R'₂ et Y sont tels que décrits précédemment,
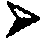 *soit avec un composé de formule (XII) :*

   **Y-Ra-W**_{**3**}**-Ra-OTs (XII)**

   dans laquelle Y et Ra sont tels que définis dans la formule (I) et W₃ représente un atome d'oxygène ou de soufre,
   pour conduire aux composés de formule (I/h), cas particulier des composés de formule (I) : 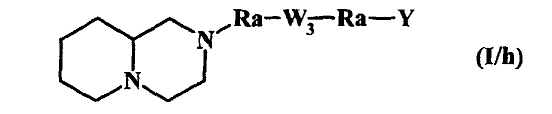 dans laquelle Ra, Y et W₃ sont tels que définis précédemment,
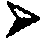 *soit avec un composés de formule (XIII) :* 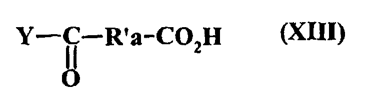 dans laquelle Y et R'a sont tels que définis dans la formule (I),
   pour conduire aux composés de formule (XIV) : 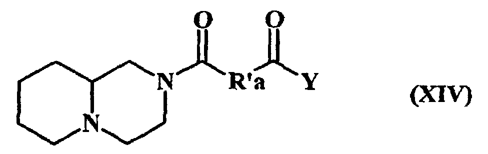 dans laquelle R'a et Y sont tels que définis précédemment,
   composés de formule (XIV) qui sont placés en présence d'un agent réducteur, pour conduire aux composés de formule (I/i), cas particulier des composés de formule (I) : 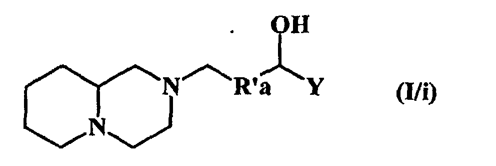 dans laquelle R'a et Y sont tels que définis dans la formule (I),
   composés de formule (I/i), dans le cas où Y représente spécifiquement un groupement Y₂ tel que décrit précédemment, qui sont oxydés en présence de diméthylsulfoxyde, de triéthylamine et de chlorure d'oxalyle, pour conduire aux composés de formule (I/j), cas particulier des composés de formule (I) : 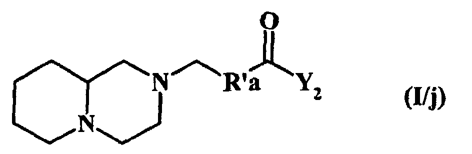 dans laquelle R'a et Y₂ sont tels que définis précédemment,
   ou composé de formule (I/i), qui est mis à réagir avec un composé de formule (XV) :

   **R'**_{**1**}**-Hal (XV)**

   dans laquelle Hal représente un atome d'halogène et R'₁ prend les mêmes définitions que R₁ à l'exception de la définition atome d'hydrogène,
   pour conduire aux composés de formule (I/k), cas particulier des composés de formule (I) : 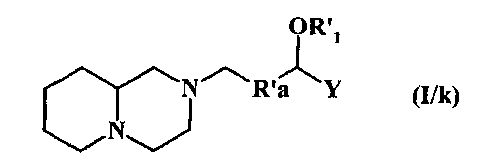 dans laquelle R'a, R'₁ et Y sont tels que définis précédemment,
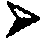 *soit avec un composés de formule (XVI) :*

   **Hal-Ra-W**_{**2**}**H (XVI)**

   dans laquelle Hal représente un atome d'halogène, Ra et W₂ sont tels que définis dans la formule (I)
   pour conduire aux composés de formule (XVII) : 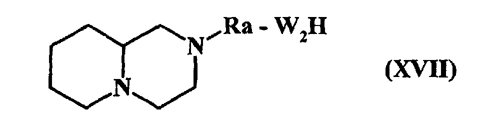 dans laquelle Ra et W₂ sont tels que définis précédemment,
   composés de formule (XVII) qui sont traités par un composé de formule (XVIII) : 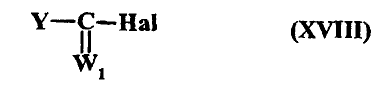 dans laquelle Hal représente un atome d'halogène, Y et W₁ sont tels que définis dans la formule (I) :
   pour conduire aux composés de formule (I/l), cas particulier des composés de formule (I) : 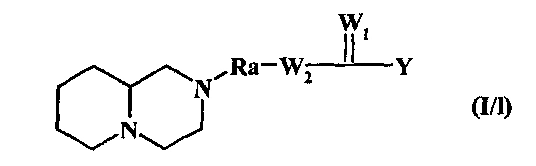 dans laquelle Ra, W₂, W₁ et Y sont tels que définis précédemment,
   les composés de formule (I/a) à (I/l) formant l'ensemble des composés de l'invention, que l'on purifie le cas échéant, selon des techniques classiques de purification, dont on sépare, éventuellement, les isomères, selon une technique classique de séparation, et que l'on transforme, si on le souhaite en leurs sels d'addition à un acide ou à une base pharmaceutiquement acceptable.

Selon une variante de l'invention, les composés de formule (I/b), dans le cas où W₂ prend la définition particulière atome d'oxygène ou de soufre, 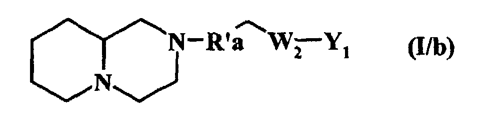 peuvent être préparés à partir d'un composé de formule (II) : 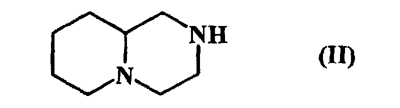 qui est mis à réagir avec un composé de formule (B1) :

**Y**_{**1**}**-W**_{**3**}**-R'a-CO**_{**2**}**H (B1)**

dans laquelle Y₁ représente un groupement aryle ou hétéroaryle, W₃ représente un atome d'oxygène ou de soufre, R'a représente une chaîne alkylène (C₁-C₅) linéaire ou ramifiée,
pour conduire aux composés de formule (B₂) : 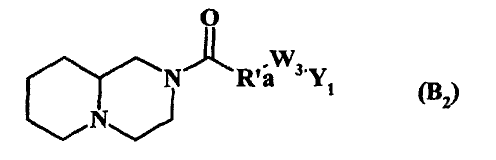 dans laquelle Y₁, W₃, et R'a sont tels que définis précédemment,
composés de formule (B₂) qui sont placés en présence d'un agent réducteur , couramment utilisé en synthèse organique, pour conduire aux composés de formule (I/b₁), cas particulier des composés de formule (I) : 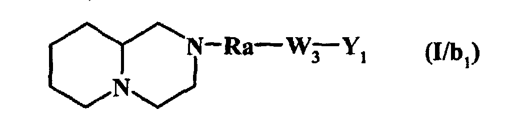 dans laquelle Ra représente une chaîne alkylène (C₁-C₆) linéaire ou ramifiée, W₃ et Y₁ sont tels que définis précédemment.

Les composés de formule (XII) sont soit des composés commerciaux, soit obtenus à partir de composés de formule (A1) :

**Y-Ra**_{**1**}**-CHO (A1)**

dans laquelle Y et Ra₁ sont tels que définis dans la formule (I),
composés de formule (A1) qui sont mis à réagir avec un composé de formule (A2) :

**HO-Ra-W**_{**3**}**H (A2)**

dans laquelle Ra et W₃ sont tels que définis précédemment,
pour conduire aux composés de formule (A3) : 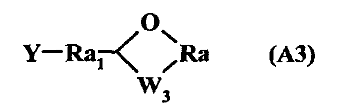 dans laquelle Y, W₃, Ra et Ra₁ sont tels que définis précédemment,
composés de formule (A3) qui sont traités par du DIBAL pour conduire aux composés de formule (A4) :

**Y-Ra-W**_{**3**}**-Ra-OH (A4)**

dans laquelle Y, Ra et W₃ sont tels que définis précédemment,
composés de formule (A4) qui sont mis en présence de chlorure de tosyle en milieu basique pour conduire aux composés de formule (XII) tel que défini précédemment.

Les composés de formule (II), (III), (V), (VI), (VII), (VIII), (IX), (X), (XII), (XIII), (XV), (XVI) et (XVIII) sont soit des produits commerciaux, soit obtenus selon des méthodes classiques de la synthèse organique.

D'une façon générale, on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon des techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur. D'une autre façon, les formes énantiomères pures des composés de l'invention peuvent être séparées par chromatographie sur colonne chirale.

Les composés de l'invention, qui sont présents sous la forme d'un mélange de diastéréoisomères, sont isolés sous forme pure par l'utilisation des techniques classiques de séparation telles que les chromatographies.

Dans certains cas particuliers, le procédé de préparation des composés de l'invention peut conduire à la formation prédominante d'un énantiomère ou d'un diastéréoisomère par rapport à l'autre.

Les composés de la présente invention, de par leurs propriétés pharmacologiques de ligands histaminiques du récepteur H₃, sont utiles dans le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels, de l'obésité et de la douleur.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 10 mg à 1000 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utile pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion donné ainsi que la microanalyse élémentaire correspondent à celui du produit salifié.

### EXEMPLE 1 : Dichlorhydrate de 2-[4-(3,4,5-triméthoxyphénoxy)butyl]octahydro-2H-pyrido[1,2-a]pyrazine

### Stade A : 4-(3,4,5-Triméthoxyphénoxy)butanoate d'éthyle

9,2 g de triméthoxyphénol, 14 g de carbonate de potassium, et 10,7 g de 4-bromobutanoate d'éthyle dans 150 ml de 2-butanone sont portés à reflux 24 heures. Les sels minéraux sont alors filtrés et le solvant éliminé par distillation sous pression réduite permettant d'obtenir le composé attendu.

### Stade B :Acide 4 -(3,4,5-triméthoxyphénoxy)butanoïque

7 g du composé obtenu au stade A sont agités à température ambiante pendant 72 heures dans un mélange de 100 ml d'éthanol et 100 ml de soude aqueuse 1M. Après acidification par 100 ml d'acide chlorhydrique 1M, le milieu réactionnel est extrait au dichlorométhane. Les fractions organiques séchées sur sulfate de sodium sont évaporées sous pression réduite et permettent d'isoler le produit attendu.
*Point de fusion : 58-60°C*

| *Microanalyse élémentaire :* | | |
|---|---|---|
| | *C* | *H* |
| *% calculé* | *57,77* | *6,71* |
| *% trouvé* | *57,95* | *6,72* |

### Stade C : 1-[Octahydro-2H-pyrido[1,2-a]pyrazin-2-yl)-4-(3,4,5-triméthoxyphénoxy-butan-1-one

3,5 g du composé obtenu au stade B, 4,5 g de 2-(1*H*-benzotriazol-1-yl)-1,1,3,3-tétraméthyluronium tétrafluoroborate, 2,6 ml de diisopropyléthylamine et 1,96 g d'octahydro-2*H*-pyrido[1,2-*a*]pyrazine dans 150 ml de tétrahydrofurane sont agités 24 heures à température ambiante. Le solvant est alors éliminé sous pression réduite et le résidu repris dans un mélange eau/dichlorométhane. La phase organique, séchée au sulfate de sodium, évaporée sous pression réduite, est purifiée sur colonne chromatographique de silice éluée par un mélange dichlorométhane/méthanol (98/2) pour conduire au produit attendu.

### Stade D : Dichlorhydrate de 2-[4-(3,4,5-triméthoxyphénoxy)butyl]octahydro-2H-pyrido[1,2-a]pyrazine

4,2 g du composé obtenu au stade C en solution dans 100 ml de tétrahydrofurane sont additionnés de 0,5 g de LiAlH₄, puis laissé 3 heures sous agitation ; après hydrolyse par du sulfate de sodium, les sels minéraux sont filtrés et le solvant évaporé sous vide. Un chlorhydrate du produit attendu est alors préparé dans 50 ml d'éthanol et de l'éther chlorhydrique.
*Point de fusion : 245-246°C*

| *Microanalyse élémentaire :* | | | | |
|---|---|---|---|---|
| | *C* | *H* | *N* | *Cl* |
| *% calculé* | *55,87* | *8,04* | *6,21* | *15,71* |
| *% trouvé* | *55,59* | *8,14* | *6,25* | *15,98* |

### EXEMPLE 2 : Dichlorhydrate de 4-octahydro-2H-pyrido[1,2-a]pyrazin-2-yl-N-(3,4,5-triméthoxyphényl)butanamide

### Stade A : 4-(Octahydro-2H-pyrido[1,2-a]pyrazin-2-yl)-butyracétate d'éthyle

7 g d'octahydro-2*H*-pyrido[1,2-*a*]pyrazine, 14 ml de 4-bromobutyrate d'éthyle et 14 g de carbonate de potassium dans 250 ml d'acétonitrile sont agités 24 heures à température ambiante. Les sels minéraux sont éliminés par filtration et le solvant par distillation sous vide partiel. Le résidu est repris dans 200 ml d'acide chlorhydrique 2N et lavé par 200 ml d'éther diéthylique. La phase aqueuse est alors alcalinisée par du bicarbonate de sodium et extraite au dichlorométhane. Après séchage sur sulfate de sodium et évaporation sous vide partiel, le produit attendu est obtenu.

### Stade B : Acide 4 - (Octahydro -2H-pyrido[1,2-a]pyrazin-2-yl)butyrique

11,2 g du composé obtenu au stade B sont agités dans 100 ml d'eau, 11 ml de soude 1M et 100 ml d'éthanol pendant 24 heures à 60°C. Le solvant est ensuite distillé sous vide partiel et le résidu séché à 60°C sous vide pour conduire au produit attendu.

| *Microanalyse élémentaire :* | | | |
|---|---|---|---|
| | *C* | *H* | *N* |
| *% calculé* | *58*,*05* | *8*,*52* | *11,28* |
| *% trouvé* | *57*,*40* | *8*,*44* | *10,95* |

### Stade C : Dichlorhydrate de 4-octahydro-2H-pyrido[1,2-a]pyrazin-2-yl-N-(3,4,5-triméthoxyphényl)butanamide

1,2 g du composé obtenu au stade B, 1,05 g d'hydrochlorure de 1-(3-diméthylaminopropyl)-3-éthylcarbodiimide et 0,92 g de 3,4,5-triméthoxyaniline en solution dans 50 ml de dichlorométhane et 1,75 ml de diisopropyléthylamine sont agités, sous argon, pendant 24 heures à température ambiante. Le mélange réactionnel est lavé par 50 ml d'eau, décanté, séché sur Na₂SO₄ et concentré sous vide partiel. Le chlorhydrate est alors préparé dans 20 ml d'éthanol et 2 ml d'une solution d'éther chlorhydrique permettant d'obtenir le produit attendu.
*Point de fusion : 230-232°C*

| *Microanalyse élémentaire :* | | | | |
|---|---|---|---|---|
| | *C* | *H* | *N* | *Cl* |
| *% calculé* | *56*,*53* | *7*,*79* | *9*,*42* | *11,92* |
| *% trouvé* | *56*,*63* | *7*,*88* | *9,16* | *11,94* |

### EXEMPLE 3 : Dichlorhydrate de 4-octahydro-2H-pyrido[1,2-a]pyrazin-2-yl-N-phényl butanamide

On procède comme dans l'exemple 2 en utilisant comme substrat l'aniline et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *57*,*75* | *7*,*81* | *11*,*22* | *18,94* |
| *% trouvé* | *57*,*74* | *7*,*71* | *11*,*11* | *19,55* |

### EXEMPLE 4 : Dichlorhydrate de 4-octahydro-2H-pyrido[1,2-a]pyrazin-2-yl-N-(4-bromophényl)butanamide

On procède comme dans l'exemple 2 en utilisant comme substrat la 4-bromoaniline et le composé obtenu lors du stade B de l'exemple 2.
*Point de fusion : >250 °C*

| *Microanalyse élémentaire :* | | | | |
|---|---|---|---|---|
| | *C* | *H* | *N* | *Cl* |
| *% calculé* | *47*,*70* | *6,23* | *9*,*27* | *15,64* |
| *% trouvé* | *47*,*38* | *6*,*34* | *8*,*92* | *15,48* |

### EXEMPLE 5 : Dichlorhydrate de N-(4-fluorophényl)-4-octahydro-2H-pyrido[1,2-a] pyrazin-2-ylbutanamide

On procède comme dans l'exemple 2 en utilisant comme substrat la 4-fluoroaniline et le composé obtenu lors du stade B de l'cxemple 2.
*Point de fusion : 243-244°C*

| *Microanalyse élémentaire :* | | | | |
|---|---|---|---|---|
| | *C* | *H* | *N* | *Cl* |
| *% calculé* | *55,10* | *7,19* | *10,71* | *18,07* |
| *% trouvé* | *54,91* | *7,34* | *10,46* | *18,16* |

### EXEMPLE 6 : Dichlorhydrate de 4-octahydro-2H-pyrido[1,2-a]pyrazin-2-yl-N-(4-méthylphényl)butanamide

On procède comme dans l'exemple 2 en utilisant comme substrat la 4-méthylaniline et le composé obtenu lors du stade B de l'exemple 2.
*Point de fusion : 248-250 °C*

| *Microanalyse élémentaire :* | | | | |
|---|---|---|---|---|
| | *C* | *H* | *N* | *Cl* |
| *% calculé* | *58,76* | *8,05* | *10,82* | *18,26* |
| *% trouvé* | *58,48* | *7,95* | *10,58* | *18,10* |

### EXEMPLE 7 : Dichlorhydrate de 4-octahydro-2H-pyrido[1,2-a]pyrazin-2-yl-N-(4-trifluorométhyl-phényl)butanamide

On procède comme dans l'exemple 2 en utilisant comme substrat la 4-trifluorométhylaniline et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *51,59* | *6,38* | *9,50* | *16,03* |
| *% trouvé* | *51,76* | *6,48* | *9,36* | *16,14* |

### EXEMPLE 8 : Dichlorhydrate de 4-octahydro-2H-pyrido[1,2-a]pyrazin-2-yl-N-(4-méthoxyphényl)butanamide

On procède comme dans l'exemple 2 en utilisant comme substrat la *p*-anisidine et le composé obtenu lors du stade B de l'exemple 2.
*Point de fusion : 248-250°C*

| *Microanalyse élémentaire :* | | | | |
|---|---|---|---|---|
| | *C* | *H* | *N* | *Cl* |
| *% calculé* | *56,43* | *7,73* | *10,39* | *17,53* |
| *% trouvé* | *56,60* | *7,72* | *10,35* | *17,59* |

### EXEMPLE 9 : Dichlorhydrate de 2-[4-(2,3-dihydro-1H-indol-1-yl)-4-oxobutyl]octahydro-2H-pyrido[1,2-a]pyrazine

On procède comme dans l'exemple 2 en utilisant comme substrat le 2,3-dihydroindole et le composé obtenu lors du stade B de l'exemple 2.
*Point de fusion : >250 °C*

| *Microanalyse élémentaire :* | | | | |
|---|---|---|---|---|
| | *C* | *H* | *N* | *Cl* |
| *% calculé* | *60,00* | *7,80* | *10,49* | *17,71* |
| *% trouvé* | *59,72* | *7,77* | *10,28* | *17,85* |

### EXEMPLE 10 : Dichlorhydrate de 4-octahydro-2H-pyrido[1,2-a]pyrazin-2-yl-N-(4-tert-butylphényl)butanamide

On procède comme dans l'exemple 2 en utilisant comme substrat la 4-*tert*-butylaniline et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *61,39* | *8,66* | *9,76* | *16,47* |
| *% trouvé* | *61,09* | *8,58* | *9,64* | *16,41* |

### EXEMPLE 11 : Dichlorhydrate de 4-octahydro-2H-pyrido[1,2-a]pyrazin-2-yl-N-(3,4-diméthoxyphényl)butanamide

On procède comme dans l'cxemple 2 en utilisant comme substrat la 3,4-diméthoxyaniline et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *55,30* | *7,66* | *9,67* | *16,32* |
| *% trouvé* | *55,47* | *7,71* | *9,66* | *16,62* |

### EXEMPLE 12 : Dichlorhydrate de N-(1,3-benzodioxol-5-yl)-4-octahydro-2H-pyrido[1,2-a]pyrazin-2-ylbutanamide

On procède comme dans l'exemple 2 en utilisant comme substrat le benzo[1,3]dioxol-5-ylamine et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *54,55* | *6,99* | *10,04* | *16,95* |
| *% trouvé* | *54,41* | *7,00* | *9,83* | *16,96* |

### EXEMPLE 13 : Dichlorhydrate de 4-octahydro-2H-pyrido[1,2-a]pyrazin-2-yl-N-(3-chlorophényl)butanamide

On procède comme dans l'exemple 2 en utilisant comme substrat la 3-chloroaniline et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *52,89* | *6,90* | *10,28* | *26,02* |
| *% trouvé* | *52,83* | *6,86* | *10,10* | *25,97* |

### EXEMPLE 14 : Dichlorhydrate de N-(3,5-dichlorophényl)-4-octahydro-2H-pyrido[1,2-a]pyrazin-2-ylbutanamide

On procède comme dans l'exemple 2 en utilisant comme substrat la 3,5-dichloroaniline et le composé obtenu lors du stade B de l'exemple 2.
*Point de fusion : >250°C*

| *Microanalyse élémentaire :* | | | | |
|---|---|---|---|---|
| | *C* | *H* | *N* | *Cl* |
| *% calculé* | *48*,*78* | *6*,*14* | *9*,*48* | *31,99* |
| *% trouvé* | *48,55* | *6*,*21* | *9*,*22* | *32,11* |

### EXEMPLE 15 : Dichlorhydrate de 4-octahydro-2H-pyrido[1,2-a]pyrazin-2-yl-N-(2-méthoxyphényl)butanamide

On procède comme dans l'exemple 2 en utilisant comme substrat la 2-méthoxyaniline et le composé obtenu lors du stade B de l'exemple 2.
*Point de fusion :>250 °C*

| *Microanalyse élémentaire :* | | | | |
|---|---|---|---|---|
| | *C* | *H* | *N* | *Cl* |
| *% calculé* | *56*,*43* | *7*,*73* | *10*,*39* | *17,53* |
| *% trouvé* | *56*,*53* | *7*,*78* | *10*,*39* | *17,53* |

### EXEMPLE 16 : Trichlorhydrate de N-(4-octahydro-2H-pyrido[1,2-a]pyrazin-2-ylbutyl)-N-(3,4,5-triméthoxyphényl)amine

1,0 g de composé obtenu lors du stade C de l'exemple 2 est mis en solution dans 50 ml de tétrahydrofurane et additionné sous argon, de 5 ml d'une solution de borane 1M dans le tétrahydrofurane puis porté 6 heures à reflux. Après hydrolyse, par 1 ml de HCl 4N et évaporation à sec, le résidu est dissous dans 5 ml d'éthanol et 1 ml d'éther chlorhydrique pour conduire au produit attendu.
*Point de fusion : 248-250°C*

| *Microanalyse élémentaire :* | | | | |
|---|---|---|---|---|
| | *C* | *H* | *N* | *Cl* |
| *% calculé* | *51*,*80* | *7,88* | *8*,*63* | *21,84* |
| *% trouvé* | *51*,*34* | *7*,*83* | *8*,*43* | *22,24* |

### EXEMPLE 17 : Trichlorhydrate de N-(4-octahydro-2H-pyrido[1,2-a]pyrazin-2-ylbutyl)-N-phénylamine

On procède comme dans l'exemple 16 en utilisant comme substrat le composé obtenu dans l'exemple 3.
*Point de fusion :>250 °C*

| *Microanalyse élémentaire :* | | | | |
|---|---|---|---|---|
| | *C* | *H* | *N* | *Cl* |
| *% calculé* | *54,48* | *8,13* | *10,59* | *26,80* |
| *% trouvé* | *54,60* | *8,08* | *10,44* | *26,40* |

### EXEMPLE 18 : Trichlorhydrate de N-(4-trifluorophényl)-N-(4-octahydro-2H-pyrido[1,2-a]pyrazin-2-ylbutyl)amine

On procède comme dans l'exemple 16 en utilisant comme substrat le composé obtenu dans l'exemple 7.
*Point de fusion : >250°C*

| *Microanalyse élémentaire :* | | | | |
|---|---|---|---|---|
| | *C* | *H* | *N* | *Cl* |
| *% calculé* | *49,10* | *6,72* | *9,04* | *22,88* |
| *% trouvé* | *49,14* | *6,92* | *8,85* | *23,08* |

### EXEMPLE 19 : Trichlorhydrate de N-(4-bromophényl)-N-(4-octahydro-2H-pyrido[1,2-a]pyrazin-2-ylbutyl)amine

On procède comme dans l'exemple 16 en utilisant comme substrat le composé obtenu dans l'exemple 4.
*Point de fusion :>250 °C*

| *Microanalyse élémentaire :* | | | | |
|---|---|---|---|---|
| | *C* | *H* | *N* | *Cl* |
| *% calculé* | *45,45* | *6,57* | *8,83* | *22,36* |
| *% trouvé* | *45,21* | *6,57* | *8,59* | *22,42* |

### EXEMPLE 20 : Trichlorhydrate de N-(4-fluorophényl)-N-(4-octahydro-2H-pyrido[1,2-a]pyrazin-2-ylbutyl)amine

On procède comme dans l'exemple 16 en utilisant comme substrat le composé obtenu dans l'exemple 5.
*Point de fusion : >250°C*

| *Microanalyse élémentaire :* | | | | |
|---|---|---|---|---|
| | *C* | *H* | *N* | *Cl* |
| *% calculé* | *52,12* | *7,53* | *10,13* | *25,64* |
| *% trouvé* | *52,46* | *7,43* | *9,98* | *25,47* |

### EXEMPLE 21 : Trichlorhydrate de N-(4-méthylphényl)-N-(4-octahydro-2H-pyrido[1,2-a]pyrazin-2-ylbutyl)amine

On procède comme dans l'exemple 16 en utilisant comme substrat le composé obtenu dans l'exemple 6.
*Point de fusion : >250 °C*

| *Microanalyse élémentaire :* | | | | |
|---|---|---|---|---|
| | *C* | *H* | *N* | *Cl* |
| *% calculé* | *55,54* | *8,34* | *10,23* | *25,89* |
| *% trouvé* | *55,83* | *8,29* | *9,98* | *25,64* |

### EXEMPLE 22 : Trichlorhydrate de N-(4-methoxyphényl)-N-(4-octahydro-2H-pyrido[1,2-a]pyrazin-2-ylbutyl)amine

On procède comme dans l'exemple 16 en utilisant comme substrat le composé obtenu dans l'exemple 8.
*Point de fusion : >250°C*

| *Microanalyse élémentaire :* | | | | |
|---|---|---|---|---|
| | *C* | *H* | *N* | *Cl* |
| *% calculé* | *53,46* | *8,03* | *9,84* | *24,92* |
| *% trouvé* | *53,14* | *8,15* | *9,49* | *24,11* |

### EXEMPLE 23 : Trichlorhydrate de 2-[4-(2,3-dihydro-1H-indol-1-yl)butyl]octahydro -2H-pyrido[1,2-a] pyrazine

On procède comme dans l'exemple 16 en utilisant comme substrat le composé obtenu dans l'exemple 9.
*Point de fusion : >250°C*

| *Microanalyse élémentaire :* | | | | |
|---|---|---|---|---|
| | *C* | *H* | *N* | *Cl* |
| *% calculé* | *56,94* | *7,88* | *9,96* | *25,21* |
| *% trouvé* | *57*,*81* | *8*,*15* | *9*,*88* | *24,98* |

### EXEMPLE 24 : Trichlorhydrate de N-(4-tert-butyl-phényl)-N-(4-octahydro-2H-pyrido[1,2-a]pyrazin-2-ylbutyl)amine

On procède comme dans l'exemple 16 en utilisant comme substrat le composé obtenu dans l'exemple 10.
*Point de fusion : >250°C*

| *Microanalyse élémentaire :* | | | | |
|---|---|---|---|---|
| | *C* | *H* | *N* | *Cl* |
| *% calculé* | *58*,*34* | *8*,*90* | *9*,*28* | *23,48* |
| *% trouvé* | *58*,*52* | *8*,*94* | *9*,*37* | *23,53* |

### EXEMPLE 25 : Trichlorhydrate de N-(3,4-diméthoxy-phényl)-N-(4-octahydro-2H-pyrido[1,2-a]pyrazin-2-ylbutyl)amine

On procède comme dans l'exemple 16 en utilisant comme substrat le composé obtenu dans l'exemple 11.
*Point de fusion : >250°C*

| *Microanalyse élémentaire :* | | | | |
|---|---|---|---|---|
| | *C* | *H* | *N* | *Cl* |
| *% calculé* | *52*,*58* | *7*,*94* | *9*,*20* | *23,28* |
| *% trouvé* | *52*,*50* | *7*,*84* | *9*,*36* | *23,20* |

### EXEMPLE 26 : Trichlorhydrate de N-(4-octahydro-2H-pyrido[1,2-a]pyrazin-2-ylbutyl)-1,3-benzodioxol-5-amine

On procède comme dans l'exemple 16 en utilisant comme substrat le composé obtenu dans l'exemple 12.
*Point de fusion : 248-250°C*

| *Microanalyse élémentaire :* | | | | |
|---|---|---|---|---|
| | *C* | *H* | *N* | *Cl* |
| *% calculé* | *51,77* | *7,32* | *9,53* | *24,13* |
| *% trouvé* | *51,15* | *7,41* | *9,16* | *24,15* |

### EXEMPLE 27 : Trichlorhydrate de N-(3-chlorophényl)-N-(4-octahydro-2H-pyrido[1,2-a]pyrazin-2-ylbutyl)amine

On procède comme dans l'exemple 16 en utilisant comme substrat le composé obtenu dans l'exemple 13.
*Point de fusion : >250°C*

| *Microanalyse élémentaire :* | | | | |
|---|---|---|---|---|
| | *C* | *H* | *N* | *Cl* |
| *% calculé* | *50,13* | *7,25* | *9,74* | *32,88* |
| *% trouvé* | *50,01* | *7,28* | *9,52* | *32,86* |

### EXEMPLE 28 : Trichlorhydrate de N-(3,5-dichloro-phényl)-N-(4-octahydro-2H-pyrido[1,2-a]pyrazin-2-ylbutyl)amine

On procède comme dans l'exemple 16 en utilisant comme substrat le composé obtenu dans l'exemple 14.
*Point de fusion : >250°C*

| *Microanalyse élémentaire :* | | | | |
|---|---|---|---|---|
| | *C* | *H* | *N* | *Cl* |
| *% calculé* | *46,42* | *6,49* | *9,02* | *38,06* |
| *% trouvé* | *46,67* | *6,53* | *8,91* | *37,49* |

### EXEMPLE 29 : Trichlorhydrate de N-(2-méthoxy-phényl)-N-(4-octahydro-2H-pyrido[1,2-a]pyrazin-2-ylbutyl)amine

On procède comme dans l'exemple 16 en utilisant comme substrat le composé obtenu dans l'exemple 15.
*Point de fusion : >250°C*

| *Microanalyse élémentaire :* | | | | |
|---|---|---|---|---|
| | *C* | *H* | *N* | *Cl* |
| *% calculé* | *53,46* | *8,03* | *9,84* | *24,92* |
| *% trouvé* | *53,31* | *8,15* | *9,65* | *24,92* |

### EXEMPLE 30 : Trichlorhydrate de N-(4-chlorophényl)-N-(4-octahydro-2H-pyrido [1,2-a]pyrazin-2-ylbutyl)amine

### Stade A : N-(4-Chlorophényl)-4-(octahydro -2H-pyrido[1,2-a]pyrazin-2-yl)-butyramide

On procède comme dans l'exemple 2 en utilisant comme substrat la 4-chloroaniline et le composé obtenu lors du stade B de l'exemple 2.

### Stade B : Trichlorhydrate de N-(4-chlorophényl)-N-(4-octahydro-2H-pyrido [1,2-a]pyrazin-2-ylbutyl)amine

On procède comme dans l'exemple 16 en utilisant comme substrat le composé obtenu dans le stade A précédent.
*Point de fusion : >250°C*

| *Microanalyse élémentaire :* | | | | |
|---|---|---|---|---|
| | *C* | *H* | *N* | *Cl* |
| *% calculé* | *50,13* | *7,25* | *9,74* | *32,88* |
| *% trouvé* | *50,19* | *7,42* | *9,66* | *32,89* |

### EXEMPLE 31 : Trichlorhydrate de N-(2-chlorophényl)-N-(4-octahydro-2H-pyrido [1,2-a]pyrazin-2-ylbutyl)amine

### Stade A : N-(2-Chlorophényl)-N-(4-octahydro-2H-pyrido[1,2-a]pyrazin-2-yl)-butyramide

On procède comme dans l'exemple 2 en utilisant comme substrat la 2-chloroaniline et le composé obtenu lors du stade B de l'exemple 2.

### Stade B : Trichlorhydrate de N-(2-chlorophényl)-N-(4-octahydro-2H-pyrido[1,2-a]pyrazin-2 -ylbutyl )amine

On procède comme dans l'exemple 16 en utilisant comme substrat le composé obtenu dans le stade A précédent.
*Point de fusion : >250°C*

| *Microanalyse élémentaire :* | | | | |
|---|---|---|---|---|
| | *C* | *H* | *N* | *Cl* |
| *% calculé* | *52,34* | *7,44* | *10,17* | *30,04* |
| *% trouvé* | *51,92* | *7,69* | *9,86* | *29,93* |

### EXEMPLE 32 : Trichlorhydrate de N-(3,4-dichlorophényl)-N-(4-octahydro-2H-pyrido[1,2-a]pyrazin-2-ylbutyl)amine

### Stade A : N-(3,4-Dichlorophényl)-4-(octahydro-2H-pyrido[1,2-a]pyrazin-2-yl)-butyramide

On procède comme dans l'exemple 2 en utilisant comme substrat la 3,4-dichloroaniline et le composé obtenu lors du stade B de l'exemple 2.

### Stade B : Trichlorhydrate de N-(3,4-dichlorophényl)-N-(4-octahydro-2H-pyrido[1,2-a]pyrazin-2-ylbutyl)amine

On procède comme dans l'exemple 16 en utilisant comme substrat le composé obtenu dans le stade A précédent.
*Point de fusion : >250°C*

| *Microanalyse élémentaire :* | | | | |
|---|---|---|---|---|
| | *C* | *H* | *N* | *Cl* |
| *% calculé* | *46,42* | *6,49* | *9,02* | *38,06* |
| *% trouvé* | *46,50* | *6,51* | *9,01* | *37,40* |

### EXEMPLE 33 : Trichlorhydrate de N-benzyl-N-(4-octahydro-2H-pyrido[1,2-a]pyrazin-2-ylbutyl)-N-(3,4,5-triméthoxyphényl)amine

### Stade A : N-Benzyl-4-(octahydro-2H-pyrido[1,2-a]pyrazin-2-yl)-N-(3,4,5-triméthoxyphényl)-butyramide

On procède comme dans l'exemple 2 en utilisant comme substrat la benzyl-(3,4,5-triméthoxyphényl)amine et le composé obtenu lors du stade B de l'exemple 2.

### Stade B : Trichlorhydrate de N-benzyl-N-(4-octahydro-2H-pyrido[1,2-a]pyrazin-2-ylbutyl)-N-(3,4,5-triméthoxyphényl)amine

On procède comme dans l'exemple 16 en utilisant comme substrat le composé obtenu dans le stade A précédent.
*Point de fusion : 220-222°C*

| *Microanalyse élémentaire :* | | | | |
|---|---|---|---|---|
| | *C* | *H* | *N* | *Cl* |
| *% calculé* | *58,28* | *7,69* | *7,28* | *18,43* |
| *% trouvé* | *59,44* | *7,80* | *7,51* | *17,51* |

### EXEMPLE 34 : Trichlorhydrate de 3,4,5-triméthoxy-N-(4-octahydro-2H-pyrido[1,2-a]pyrazin-2-ylbutyl)-N-phénylaniline

### Stade A : N-Phényl-4-(octahydro-2H-pyrido[1,2-a]pyrazin-2-yl)-N-(3,4,5-triméthoxy-phényl)-butyramide

On procède comme dans l'exemple 2 en utilisant comme substrat la phényl-(3,4,5-triméthoxyphényl)amine et le composé obtenu lors du stade B de l'exemple 2.

### Stade B : 3,4,5-triméthoxy-N-(4-octahydro-2H-pyrido[1,2-a]pyrazin-2-ylbutyl)-N-phénylaniline

On procède comme dans l'exemple 16 en utilisant comme substrat le composé obtenu dans le stade A précédent.
*Point de fusion : 201-202 °C*

| *Microanalyse élémentaire :* | | | | |
|---|---|---|---|---|
| | *C* | *H* | *N* | *Cl* |
| *% calculé* | *59,53* | *7,68* | *7,71* | *16,27* |
| *% trouvé* | *59,96* | *7,72* | *8,10* | *16,20* |

### EXEMPLE 35 : Dichlorhydrate de N-méthyl-4-octahydro-2H-pyrido[1,2-a]pyrazin-2-yl-N-(3,4,5-triméthoxyphényl)butanamide

0,9 g du composé obtenu lors de l'exemple 2 sont solubilisés dans 50 ml de tétrahydrofurane et additionnés sous argon de 0,265 g de terbutoxyde de potassium puis après 1 heure d'agitation de 0,15 ml d'iodure de méthyle. L'agitation est poursuivie 1 heure et le solvant est éliminé sous vide. Le résidu est repris dans un mélange eau/dichlorométhane, extrait, décanté puis séché sur Na₂SO₄ et évaporé à sec sous vide. Le produit est transformé en chlorhydrate dans de l'éthanol chlorhydrique.
*Point de fusion : 185-186°C*

| *Microanalyse élémentaire :* | | | | |
|---|---|---|---|---|
| | *C* | *H* | *N* | *Cl* |
| *% calculé* | *55,23* | *7,79* | *8,78* | *14,82* |
| *% trouvé* | *55,05* | *7,87* | *8,52* | *14,72* |

### EXEMPLE 36 : Trichlorhydrate de N-méthyl-N-(4-octahydro-2H-pyrido[1,2-a]pyrazin-2-ylbutyl)-N-(3,4,5-triméthoxyphényl)amine

0,4 g du composé obtenu lors de l'exemple 35 en solution, sous argon, dans 50 ml de tétrahydrofurane sont additionnés de 10 ml d'une solution de borane 1M dans le tétrahydrofurane et portés à reflux 8 heures. Après hydrolyse par 2 ml d'acide chlorhydrique 4N, évaporation à sec et passage à la base, le chlorhydrate est préparé dans une solution d'éthanol chlorhydrique.
*Point de fusion : 214-215°C*

| *Microanalyse élémentaire :* | | | | |
|---|---|---|---|---|
| | *C* | *H* | *N* | *Cl* |
| *% calculé* | *52,75* | *8,05* | *8,39* | *21,23* |
| *% trouvé* | *53,20* | *8,01* | *8,53* | *21,42* |

### EXEMPLE 37 : Dichlorhydrate de 3,4,5-triméthoxy-N-(3-octahydro-2H-pyrido[1,2-a]pyrazin-2-ylpropyl)benzamide

### Stade A : 2 -(Octahydro-2H-pyrido[1,2-a]pyrazin-2-yl)acétonitrile

8 g de octahydro-2*H*-pyrido[1,2-a]pyrazine et 12 ml d'acrylonitrile sont portés à reflux pendant 48 heures dans 150 ml d'acétonitrile. Le solvant est alors éliminé sous vide pour conduire à 11 g d'une huile jaune pâle.

### Stade B : 3-(Octahydro-2H-pyrido[1,2-a]pyrazin-2-yl)propanamine

4 g du composé obtenu lors du stade A, 0,4 g de PtO₂, 30 ml de méthanol chlorhydrique et 100 ml de méthanol sont hydrogénés dans un autoclave, sous 5 bars de pression d'hydrogène pendant 8 heures à température ambiante. Le catalyseur est alors éliminé par filtration et le filtrat est évaporé puis transformé en base pour conduire au produit attendu.

### Stade C : Dichlorhydrate de 3,4,5-triméthoxy-N-(3-octahydro-2H-pyrido[1,2-a]pyrazin-2-ylpropyl)benzamide

2,1 g de composé obtenu lors du stade B, 2,3 g d'acide 3,4,5 triméthoxybenzoïque, 3,8 ml de diisopropyléthylamine, et 2,1 g d'hydrochlorure de 1-(3-diméthylaminopropyl)-3-éthylcarbodiimide dans 200 ml de dichlorométhane sont agités 48 heures à température ambiante sous argon. Le milieu est ensuite lavé par 100 ml d'eau, séché sur Na₂SO₄, évaporé à sec, purifié sur une colonne chromatographique éluée par un mélange dichlorométhane/méthanol (98/2) et transformé en son chlorhydrate dans l'éthanol.
*Point de fusion : 224-225°C*

| *Microanalyse élémentaire :* | | | | |
|---|---|---|---|---|
| | *C* | *H* | *N* | *Cl* |
| *% calculé* | *54*,*31* | *7*,*61* | *9*,*05* | *15,27* |
| *% trouvé* | *54*,*13* | *7*,*42* | *9*,*10* | *15,37* |

### EXEMPLE 38 : Trichlorhydrate de N-(3-octahydro-2H-pyrido[1,2-a]pyrazin-2-ylpropyl)-N-(3,4,5-triméthoxybenzyl)amine

0,45 g de composé obtenu lors du stade C de l'exemple 37 dans 50 ml de tétrahydrofurane sont additionnés, sous argon, de 8 ml d'une solution 1M de DIBAL dans le toluène et agités pendant 20 heures. Après hydrolyse par 2 ml d'acide chlorhydrique 4N et évaporation sous vide partiel du solvant, le résidu est repris dans 50 ml de dichlorométhane et 50 ml de bicarbonate de sodium à 5 % dans l'eau. Après extraction et décantation, la phase organique est séchée sur Na₂SO₄ puis concentrée à sec. Le résidu est transformé en chlorhydrate dans de l'éthanol chlorhydrique.
*Point de fusion : 247-248°C*

| *Microanalyse élémentaire :* | | | | |
|---|---|---|---|---|
| | *C* | *H* | *N* | *Cl* |
| *% calculé* | *51,80* | *7,87* | *8*,*63* | *21,84* |
| *% trouvé* | *51*,*46* | *7*,*81* | *8,57* | *21,80* |

### EXEMPLE 39 : Dichlorhydrate de 2-[4-(1H-indol-1-yl)butyl]octahydro-2H-pyrido [1,2-a]pyrazine

### Stade A : 1-(4-Bromo-butyl)-1H-indole

Une solution de 6 g d'indole dans 150 ml de diméthylformamide est sodée par 2 g d'hydrure de sodium à 60 % dans l'huile, puis additionnée rapidement de 11 ml de 1,4-dibromobutane et agitée une nuit à température ambiante. Après distillation du diméthylformamide, le résidu est repris dans un mélange eau/dichlorométhane, extrait, et décanté. La phase organique, séchée sur Na₂SO₄ est concentrée sous vide. Le résidu est purifié sur colonne de silice (dichlorométhane/heptane : 5/5) pour conduire au composé attendu.

### Stade B : Dichlorhydrate de 2-[4-(1H-indol-1-yl)butyl]octahydro-2H-pyrido [1,2-a]pyrazine

1,2 g du composé obtenu lors du stade A, 0,7 g de octahydro-2*H*-pyrido[1,2-*a*]pyrazine et 1,4 g de carbonate de potassium sont agités 24 heures dans 60 ml d'éthanol. Les sels minéraux sont ensuite éliminés par filtration et le filtrat concentré à sec sous vide. Le composé obtenu est transformé en chlorhydrate dans un mélange éthanol/acide chlorhydrique/éther diisopropylique permettant d'isoler le produit attendu.
*Point de fusion : >250°C*

| *Microanalyse élémentaire :* | | | | |
|---|---|---|---|---|
| | *C* | *H* | *N* | *Cl* |
| *% calculé* | *62*,*49* | *8*,*13* | *10*,*93* | *18,85* |
| *% trouvé* | *62*,*13* | *8,14* | *10*,*85* | *18,52* |

### EXEMPLE 40 : Dichlorhydrate de N-(4-octahydro-2H-pyrido[1,2-a]pyrazin-2-ylbutyl)-N-(3,4,5-triméthoxyphényl)acetamide

0,44 g du composé obtenu lors de l'exemple 16 en solution dans 50 ml de dichlorométhane et 0,16 ml de triéthylamine sont additionnés à 0°C de 0,16 ml de chlorure d'acétyle et agités 2 heures. La solution est lavée par 25 ml d'une solution de bicarbonate de sodium à 5 %, séchée sur sulfate de sodium, évaporée à sec, puis purifiée sur une colonne de silice éluée par un mélange CH₂Cl₂/MeOH (95/5). Le composé purifié est transformé en chlorhydrate dans une solution d'éthanol chlorhydrique.
*Point de fusion : > 250°C*

| *Microanalyse élémentaire :* | | | | |
|---|---|---|---|---|
| | *C* | *H* | *N* | *Cl* |
| *% calculé* | *56,09* | *7,98* | *8,53* | *14,40* |
| *% trouvé* | *55,71* | *7,96* | *8,27* | *14,44* |

### EXEMPLE 41 : Trichlorhydrate de N-(5-octahydro-2H-pyrido[1,2-a]pyrazin-2-ylpentyl)-N-(3,4,5-triméthoxyphényl)amine

### Stade A : 4-(3,4,5-Triméthoxyphénylcarbamoyl)-butyrate d'éthyle

A une solution refroidie à 0-5°C de 3,4,5-triméthoxyaniline dans 150 ml de dichlorométhane et 7,2 ml de triéthylamine sont additionnés goutte à goutte 8,2 ml de chlorure de l'acide éthyl-glutarique. Après 3 heures d'agitation le mélange réactionnel est lavé par 100 ml d'eau, décanté, séché sur Na₂SO₄ et évaporé à sec pour conduire au produit attendu.

### Stade B : Acide 4-(3,4,5-triméthoxy-phénylcarbamoyl)-butyrique

Une solution composée de 8 g de composé obtenu lors du stade A, 50 ml de soude 1M, 50 ml d'eau et 100 ml d'éthanol est agitée 6 heures à 50°C. L'éthanol est alors éliminé par distillation sous vide partiel. La solution résiduelle est acidifiée par addition d'acide chlorhydrique 1M et extraite au dichlorométhane. Les phases organiques réunies sont séchées par Na₂SO₄ et évaporées à sec pour conduire à une huile incolore (rendement quantitatif).

### Stade C : 5-(Octahydro-pyrido[1,2-a]pyrazin-2-yl)-5-oxo-(3,4,5-triméthoxyphényl)pentanamide

Une solution de 3 g du composé obtenu lors du stade C, 1,4 g de octahydro-2*H*-pyrido[1,2-a]pyrazine, 2 g d'hydrochlorure de 1-(3-diméthylaminopropyl)-3-éthyl carbodiimide, 2 ml de diisopropyléthylamine dans 150 ml de dichlorométhane est agitée 24 heures à température ambiante, puis lavée par 150 ml d'eau, décantée, séchée sur Na₂SO₄ et évaporée à sec pour conduire à une huile incolore.

### Stade D : Trichlorhydrate de N-(5-octahydro -2H-pyrido[1,2-a]pyrazin-2-ylpentyl)-N-(3,4,5-triméthoxyphényl)amine

Une solution de 1,5 g du composé obtenu lors du stade C dans 100 ml de tétrahydrofurane, placée sous argon, est additionnée de 15 ml d'une solution de borane 1M dans le tétrahydrofurane et portée à 50°C pendant 8 heures, puis hydrolysée par 5 ml d'acide chlorhydrique 4N. Après évaporation du solvant, puis passage à la base, le chlorhydrate est obtenu dans une solution d'éthanol chlorhydrique.
*Point de fusion :226-228 °C*

| *Microanalyse élémentaire :* | | | | |
|---|---|---|---|---|
| | *C* | *H* | *N* | *Cl* |
| *% calculé* | *52,75* | *8,05* | *8,39* | *21,23* |
| *% trouvé* | *53,40* | *8,06* | *8,27* | *20,13* |

### EXEMPLE 42 : Dichlorhydrate de 3-octahydro-2H-pyrido[1,2-a]pyrazin-2-yl-N-(3,4,5-triméthoxyphényl)propionamide

### Stade A : 3-(Octahydro-2H-pyrido[1,2-a]pyrazin-2-yl)propionate d'éthyle

Une solution de 7 g de octahydro-2*H*-pyrido[1,2-*a*]pyrazine et 15 ml d'acrylate d'éthyle dans 100 ml d'acétonitrile est portée 24 heures à reflux puis concentrée à sec sous vide pour conduire au produit attendu.

| *Microanalyse élémentaire :* | | | |
|---|---|---|---|
| | *C* | *H* | *N* |
| *% calculé* | *64*,*97* | *10*,*06* | *11,66* |
| *% trouvé* | *64*,*34* | *10*,*08* | *11*,*47* |

### Stade B : Acide 3-(octahydro-2H-pyrido[1,2-a]pyrazin-2-yl)proprionique

Une solution de 6,0 g du composé obtenu lors du stade A dans 50 ml d'éthanol et 25 ml d'une solution 1M de soude est chauffée 5 heures à 50°C puis concentrée à sec sous vide et séchée en présence de P₂O₅ pour conduire au composé attendu.

### Stade C : Dichlorhydrate de 3-octahydro-2H-pyrido[1,2-a]pyrazin-2-yl-N-(3,4,5-triméthoxyphényl)propionamide

Une solution de 6,0 g du composé obtenu lors du stade B, 4,8 g d'hydrochlorure de 1-(3-diméthylaminopropyl)-3-éthylcarbodiimide, 4,8 g de triméthoxyaniline et 4,7 ml de diisopropyléthylamine, dans 200 ml de dichlorométhane est agitée 24 heures, puis lavée par 100 ml d'eau, décantée, séchée sur Na₂SO₄ et enfin évaporée à sec pour conduire au composé attendu sous forme de base. Un chlorhydrate est préparé dans une solution d'éther chlorhydrique
*Point de fusion : 249-250°C*

| *Microanalyse élémentaire :* | | | | |
|---|---|---|---|---|
| | *C* | *H* | *N* | *Cl* |
| *% calculé* | *53*,*33* | *7*,*38* | *9*,*33* | *15,74* |
| *% trouvé* | *53*,*09* | *7*,*39* | *9*,*18* | *16*,*21* |

### EXEMPLE 43 : Trichlorhydrate de N-(3-octahydro-2H-pyrido[1,2-a]pyrazin-2-ylpropyl)-N-(3,4,5-triméthoxyphényl)amine

Une solution de 1,5 g du composé obtenu lors du stade C de l'exemple 42 dans 100 ml de tétrahydrofurane et 15 ml d'une solution de borane 1M dans le tétrahydrofurane est chaufféé 6 heures à reflux, puis hydrolysée par 5 ml d'acide chlorhydrique 4N. Après évaporation du solvant, puis passage à la base, le chlorhydrate est préparé par cristallisation d'une solution d'éthanol chlorhydrique.
*Point de fusion : 226-228°C*

| *Microanalyse élémentaire :* | | | | |
|---|---|---|---|---|
| | *C* | *H* | *N* | *Cl* |
| *% calculé* | *50,80* | *7,67* | *8,89* | *22,49* |
| *% trouvé* | *51,37* | *7,99* | *8,87* | *22,40* |

### EXEMPLE 44 : Dichlorhydrate de 2-[4-(3,4,5-triméthoxy-benzyloxy)butyl] octahydro-2H-pyrido[1,2-a]pyrazine

### Stade A : 3-(Octahydro-2H-pyrido[1,2-a]pyrazin-2-yl)propanoate d'éthyle

Une solution de 3,5 g de octahydro-2*H*-pyrido[1,2-*a*]pyrazine et 8 ml d'acrylate d'éthyle dans 50 ml d'acétonitrile est portée 24 heures à reflux. Le solvant est éliminé sous vide pour conduire au produit attendu.

| *Microanalyse élémentaire :* | | | |
|---|---|---|---|
| | *C* | *H* | *N* |
| *% calculé* | *64*,*97* | *10*,*06* | *11*,*66* |
| *% trouvé* | *64*,*34* | *10*,*08* | *11,47* |

### Stade B : 3-(Octahydro-2H-pyrido[1,2-a]pyrazin-2-yl)propanol

1 g de LiAlH₄ est additionné par portions à une solution de 3 g du composé obtenu lors du stade A, dans 100 ml de tétrhydrofurane puis le mélange réactionnel est agité 8 heures, ensuite hydrolysé, filtré, et enfin évaporé à sec.

### Stade C : 4-Toluènesulfonate de 3-(Octahydro-2H-pyrido[1,2-a]pyrazin-2-yl)propanol

Une solution de 4,5 g du composé obtenu lors du stade B, 2,5 ml de pyridine, 4,8 g de chlorure de tosyle dans 100 ml de dichlorométhane est agitée 24 heures à température ambiante, puis lavée par 100 ml d'eau et enfin purifiée sur une colonne chromatographique de gel de silice, éluée par un mélange CH₂Cl₂/MeOH (95/5) permettant d'isoler le produit attendu.

### Stade D : Dichlorhydrate de 2-[4-(3,4,5-triméthoxy-benzyloxy)butyl]octahydro-2H-pyrido[1,2-a]pyrazine

Une solution de 0,9 g d'alcool 3,4,5-triméthoxybenzylique dans 100 ml de tétrahydrofurane est sodée par 0,25 g d'hydrure de sodium à 60 % dans l'huile. A la fin du dégagement d'hydrogène, 1,6 g du composé obtenu lors du stade C sont additionnés dans 10 ml de tétrahydrofurane et le mélange réactionnel est agité 8 heures, puis lavé par 100 ml d'eau, séché sur Na₂SO₄ et évaporé à sec. Une purification sur gel de silice élué par un mélange CH₂Cl₂/MeOH (98/2) permet d'isoler le produit attendu qui est enfin transformé en chlorhydrate dans une solution d'éthanol chlorhydrique.
*Point de fusion : 182-183°C*

| *Microanalyse élémentaire :* | | | | |
|---|---|---|---|---|
| | *C* | *H* | *N* | *Cl* |
| *% calculé* | *55*,*87* | *8*,*04* | *6,21* | *15,71* |
| *% trouvé* | *55*,*24* | *7*,*94* | *6*,*34* | *16,04* |

### EXEMPLE 45 : Dichlorhydrate de 2-[4-(3,4,5-triméthoxy-phénylsulfanyl)butyl] octahydro-2H-pyrido[1,2-a]pyrazine

### Stade A : Triisopropyl-(3,4,5-triméthoxy-phénylsulfanyl)-silane

Une solution de 5 g de triisopropylsilanethiol dans 100 ml de tétrahydrofurane est additionnée de 1,12 g d'hydrure de sodium. Après la fin du dégagement d'hydrogène, 2,4 g de Pd(PPh₃)₄, 100 ml de toluène et 6,9 g de 1-bromo-3,4,5-triméthoxybenzène sont additionnés et le milieu réactionnel est porté 2 heures à reflux, puis évaporé sous vide. Une chromatographie sur gel de silice éluée par un mélange CH₂Cl₂/heptane (70/30) permet d(isoler le produit attendu.

| *Microanalyse élémentaire :* | | | |
|---|---|---|---|
| | *C* | *H* | *S* |
| *% calculé* | *60*,*63* | *9*,*04* | *8,99* |
| *% trouvé* | *61,12* | *9,19* | *8,38* |

### Stade B : 4-(3,4,5-triméthoxy-phénylsulfanyl)butyrate d'éthyle

Une suspension composée de 1,78 g du composé obtenu au stade A, 0,835 g de CsF et 1,1 g de 4-bromobutyrate d'éthyle dans 80 ml de diméthylformamide est agitée, sous argon, pendant 3 heures. Après distillation du solvant, le résidu est repris dans un mélange eau/dichlorométhane, décanté puis séché sur sulfate de sodium. Une chromatographie sur gel de silice éluée par du dichlorométhane permet d'isoler le produit attendu.

### Stade C : Acide 4-(3,4,5-triméthoxy-phénylsulfanyl)butanoïque

Une solution de 1,6 g du composé obtenu lors du stade B dans 25 ml de soude 1M et 25 ml de méthanol est agitée 4 heures à 50°C, puis distillée sous vide partiel, acidifiée par 25 ml d'acide chlorhydrique 1M et extraite par du dichlorométhane. Les phases organiques réunies sont séchées sur sulfate de sodium puis évaporées à sec permettant d'isoler le produit attendu.
*Point de fusion : 88-90°C*

| *Microanalyse élémentaire :* | | | |
|---|---|---|---|
| | *C* | *H* | *S* |
| *% calculé* | *54,53* | *6,34* | *11,20* |
| *% trouvé* | *54,75* | *6,48* | *11,48* |

### Stade D : 1-(Octahydro-2H-pyrido[1,2-a]pyrazin-2-yl)-4-(3,4,5-triméthoxyphénylsulfanyl)-butan-1-one

Le produit est obtenu selon le procédé du stade C de l'exemple 1 en utilisant comme substrat le composé obtenu au stade C précédent.

### Stade E : Dichlorhydrate de 2-[4-(3,4,5-triméthoxy-phénylsulfanyl)butyl] octahydro-2H-pyrido[1,2-a]pyrazine

Une solution de 0,9 g du composé obtenu lors du stade D, 40 ml de tétrahydrofurane et 5 ml d'une solution de borane 1M dans le tétrahydrofurane est portée 8 heures à reflux, puis hydrolysée par 2 ml d'acide chlorhydrique 4N. Après évaporation et passage à la base, une cristallisation du sel dans une solution d'éthanol chlorhydrique permet d'isoler le produit attendu.
*Point de fusion :215-216 °C*

| *Microanalyse élémentaire :* | | | | | |
|---|---|---|---|---|---|
| | *C* | *H* | *N* | *S* | *Cl* |
| *% calculé* | *53,95* | *7,76* | *5,99* | *6,86* | *15,17* |
| *% trouvé* | *54,29* | *7,91* | *5,69* | *6,64* | *14,97* |

### EXEMPLE 46 : Dichlorhydrate de 3-(octahydro-2H-pyrido[1,2-a]-pyrazin-2-yl)-1-(3,4,5-triméthoxyphényl)-pentan-1-one

### Stade A : Tributyl-(3,4,5-triméthoxyphényl)stannane

Une solution de 8 g de 1-bromo-3,4,5-triméthoxybenzène, 25 ml d'hexabutyldistannane et 1,8 g de Pd(PPh₃)₄ dans 200 ml de toluène est portée à reflux, sous argon, pendant 6 heures puis concentrée à sec. Le résidu est purifié par chromatographie sur gel de silice éluée par du dichlorométhane permettant d'isoler le produit attendu.

### Stade B : 5-Bromo-1-(3,4,5-triméthoxyphényl)-pentan-1-one

Une solution de 8,5 g du composé obtenu au stade A, 3,2 ml de chlorure de 5-bromopentanoyle, 0,3 g de Pd₂(dba)₃, et 250 ml de toluène est portée à reflux pendant 6 heures, puis refroidie et évaporée sous vide. Le résidu est purifié par chromatographie sur gel de silice éluée par du dichlorométhane permettant d'isoler le produit attendu.

### Stade C : Dichlorhydrate de 3-(octahydro-2H-pyrido[1,2-a]-pyrazin-2-yl)-1-(3,4,5-triméthoxyphényl)-pentan-1-one

Une suspension de 1,5 g du composé obtenu lors du stade B, 0,7 g de octahydro-2*H*-pyrido[1,2-*a*]pyrazine et 1,4 g de carbonate de potassium dans 60 ml d'acétonitrile est agitée 24 heures à température ambiante. Les sels minéraux sont éliminés par filtration et le solvant par distillation sous vide. La base obtenue est transformée en sel dans une solution d'éthanol chlorhydrique permettent d'isoler le produit attendu.
*Point de fusion : >250°C*

| *Microanalyse élémentaire :* | | | | |
|---|---|---|---|---|
| | *C* | *H* | *N* | *Cl* |
| *% calculé* | *57,02* | *7,83* | *6,04* | *15,30* |
| *% trouvé* | *56,63* | *7,62* | *6,06* | *15,50* |

### EXEMPLE 47 : Dichlorhydrate de 5-octahydro-2H-pyrido[1,2-a]pyrazin-2-yl-1-(3,4,5-triméthoxyphényl)-1-pentanol

Une solution de 0,5 g du composé obtenu lors du stade C de l'exemple 46 dans 30 ml de méthanol est additionnée de 0,1 g de NaBH₄ en une heure, puis évaporée à sec sous vide. Un sel est cristallisé dans une solution d'éthanol chlorhydrique permettant d'isoler le produit attendu.
*Point de fusion : 184-185°C*

| *Microanalyse élémentaire :* | | | | |
|---|---|---|---|---|
| | *C* | *H* | *N* | *Cl* |
| *% calculé* | *56,77* | *8,23* | *6,02* | *15,23* |
| *% trouvé* | *56,32* | *8,19* | *6,00* | *15,16* |

### EXEMPLE 48 : Trichlorhydrate de N-(4-octahydro-2H-pyrido[1,2-a]pyrazin-2-ylbutyl)-N-(3,4,5-triméthoxyphényl)amine, énantiomère α

### EXEMPLE 49 : Trichlorhydrate de N-(4-octahydro-2H-pyrido[1,2-a]pyrazin-2-ylbutyl)-N-(3,4,5-triméthoxyphényl)amine, énantiomère β

Les composés des exemples 48 et 49 sont obtenus à partir de 0,2 g du composé de l'exemple 16 qui sont séparés sur une colonne chromatographique chirale CHIRALPAK AD. Le chlorhydrate est obtenu par action d'une solution d'éthanol chlorhydrique.

### Enantiomère α

*Pureté optique : > 99 %*
*Point de fusion : 248-250°C*

| *Microanalyse élémentaire :* | | | | |
|---|---|---|---|---|
| | *C* | *H* | *N* | *Cl* |
| *% calculé* | *51,80* | *7,87* | *8,63* | *21,84* |
| *% trouvé* | *52,45* | *7,54* | *8,58* | *20,78* |

### Enantiomère β

*Pureté optique : 98,4 %*
*Point de fusion :248-250 °C*

| *Microanalyse élémentaire :* | | | | |
|---|---|---|---|---|
| | *C* | *H* | *N* | *Cl* |
| *% calculé* | *51,80* | *7,87* | *8,63* | *21,84* |
| *% trouvé* | *52,75* | *7,60* | *8,09* | *20,54* |

### EXEMPLE 50 : Dichlorhydrate de 2-[4-(3,4,5-triméthoxy phénoxy) butyl]octahydro-2H-pyrido[1,2-a]pyrazine, énantiomère α

### EXEMPLE 51 : Dichlorhydrate de 2-[4-(3,4,5-triméthoxy phénoxy) butyl]octahydro-2H-pyrido[1,2-a]pyrazine, énantiomère β

Les composés des exemples 50 et 51 sont obtenus à partir de 0,2 g du composé de l'exemple I qui sont séparés sur une colonne chromatographique chirale CHIRALPAK AD. Le chlorhydrate est obtenu par action d'une solution d'éthanol chlorhydrique.

### Enantiomère α

*Pureté optique : > 99 %*
*Point de fusion : 220-221 °C*

| *Microanalyse élémentaire :* | | | | |
|---|---|---|---|---|
| | *C* | *H* | *N* | *Cl* |
| *% calculé* | *55,87* | *8,04* | *6,21* | *15,71* |
| *% trouvé* | *55,54* | *7,77* | *6,23* | *16,04* |

### Enantiomère β

*Pureté optique : 98,4 %*
*Point de fusion :220-221 °C*

| *Microanalyse élémentaire :* | | | | |
|---|---|---|---|---|
| | *C* | *H* | *N* | *Cl* |
| *% calculé* | *55,87* | *8,04* | *6,21* | *15,71* |
| *% trouvé* | *55,24* | *7,86* | *6,01* | *15,78* |

### EXEMPLE 52 : Trichlorhydrate de 2-[4-(3,4-dihydro-1(2H)-quinolinyl)butyl] octahydro-2H-pyrido[1,2-a]pyrazine

### Stade A : 2-[4-(3,4-dihydro-1(2H)-quinolinyl)-4-oxobutyl]octahydro-2H-pyrido[1,2-a]pyrazine

On procède comme dans l'exemple 2 en utilisant comme substrat la 3,4-dihydro-2*H*-quinoline et le composé obtenu lors du stade B de l'exemple 2.

### Stade B : Trichlorhydrate 2-[4- (3,4-dihydro -1(2H)-quinolinyl)butyl]octahydro-2H-pyrido[1,2-a]pyrazine

On procède comme dans l'exemple 23 en utilisant comme substrat le composé obtenu lors du stade A précédent.
*Point de fusion : 246-247°C*

| *Microanalyse élémentaire :* | | | | |
|---|---|---|---|---|
| | *C* | *H* | *N* | *Cl* |
| *% calculé* | *57,73* | *8,31* | *9,62* | *24,34* |
| *% trouvé* | *58,25* | *8,47* | *9,58* | *24,15* |

### EXEMPLE 53 : Trichlorhydrate de 2-[4-(1H-benzimidazol-1-yl)butyl]octahydro-2H-pyrido[1,2-a]pyrazine

### Stade A : 1-(4-bromobutyl)-1H-benzimidazole

Une solution de 5,9 g de benzimidazole dans 150 ml de diméthylformamide est sodée par 2 g d'hydrure de sodium à 60 % dans l'huile, puis additionnée de 11 ml de 1,4-dibromobutane et agitée une nuit à température ambiante. Après distillation du diméthylformamide, le résidu est repris dans un mélange eau/dichlorométhane, extrait et décanté. La phase organique, séchée sur Na₂SO₄ est concentrée sous vide. Le résidu est purifié sur colonne de silice (dichlorométhane/méthanol : 95/5) pour conduire au composé attendu.

### Stade B : Trichlorhydrate de 2-[4-(1H-benzimidazol-1-yl)butyl]octahydro-2H-pyrido [1,2-a]pyrazine

On procède comme dans l'exemple 39 en utilisant le composé obtenu lors du stade A précédent.
*Point de fusion : 244-245°C*

| *Microanalyse élémentaire :* | | | | |
|---|---|---|---|---|
| | *C* | *H* | *N* | *Cl* |
| *% calculé* | *54,10* | *7,41* | *13,28* | *25,21* |
| *% trouvé* | *54,04* | *7,36* | *13,17* | *25,04* |

### EXEMPLE 54 : Difumarate de 4-[(4-octahydro-2H-pyrido[1,2-a]pyrazin-2-ylbutyl) amino]benzonitrile

0,3 g du composé obtenu lors de l'exemple 19, 45 mg de Zn(CN)₂ et 60 mg de tétrakistriphénylphosphine palladium en solution dans 40 ml de diméthylformamide sont chauffés à 80°C, sous argon, pendant 24 heures. Après distillation du diméthylformamide, le résidu est purifié sur colonne de silice (dichlorométhane/méthanol : 95/5) pour conduire au produit attendu.
*Point de fusion : 188-190°C*

| *Microanalyse élémentaire :* | | | |
|---|---|---|---|
| | *C* | *H* | *N* |
| *% calculé* | *59,55* | *6,66* | *10,29* |
| *% trouvé* | *59,54* | *6,61* | *9,95* |

### EXEMPLE 55 : Dichlorhydrate de 2-[4-(1H-indazol-1-yl)butyl]octahydro-2H-pyrido[1,2-a]pyrazine

### Stade A : Acide 4-(1H-benzimidazol-1-yl )butanoïque

On procède comme dans l'exemple 53, stade A, en utilisant comme substrat l'indazole.

### Stade B : Dichlorhydrate de 2-[4-(1H-indazol-1-yl)butyl]octahydro-2H-pyrido[1,2-a]pyrazine

On procède comme dans l'exemple 53 en utilisant comme substrat le composé obtenu lors du stade A précédent.
*Point de fusion : 237-238°C*

| *Microanalyse élémentaire :* | | | | |
|---|---|---|---|---|
| | *C* | *H* | *N* | *Cl* |
| *% calculé* | *59*,*22* | *7*,*85* | *14*,*54* | *18,40* |
| *% trouvé* | *59*,*80* | *7*,*72* | *13*,*80* | *18,80* |

### EXEMPLE 56 : Difumarate de 4-(3-octahydro-2H-pyrido[1,2-a]pyrazin-2-ylpropoxy) benzonitrile

### Stade A : 4-(3-Bromopropoxy)benzonitrile

On procède comme dans l'exemple 53, stade A, en utilisant comme substrat le 4-hydroxybenzonitrile et le 1,3-dibromopropane.

### Stade B : Difumarate de 4-(3-octahydro-2H-pyrido[1,2-a]pyrazin-2-ylpropoxy) benzonitrile

On procède comme dans l'exemple 53 en utilisant comme substrat le composé obtenu lors du stade A précédent
*Point de fusion : 199-200°C*

| *Microanalyse élémentaire :* | | | |
|---|---|---|---|
| | *C* | *H* | *N* |
| *% calculé* | *58*,*75* | *6*,*26* | *7,91* |
| *% trouvé* | *59*,*19* | *6*,*30* | *7,85* |

### EXEMPLE 57 : Difumarate de 4-(3-octahydro-2H-pyrido[1,2-a]pyrazin-2-ylpropoxy) benzonitrile, énantiomère α

### EXEMPLE 58 : Difumarate de 4-(3-octahydro-2H-pyrido[1,2-a]pyrazin-2-ylpropoxy) benzonitrile, énantiomère β

Les composés des exemples 57 et 58 sont obtenus à partir de 0,5 g du composé de l'exemple 56 qui sont séparés sur une colonne chromatographique chirale CHIRALPAK AD. Le fumarate est obtenu dans l'éthanol.

### Enantiomère α

*Pureté optique : 98,7 %*
*Point de fusion : 180-181°C*

| *Microanalyse élémentaire :* | | | |
|---|---|---|---|
| | *C* | *H* | *N* |
| *% calculé* | *58*,*75* | *6*,*26* | *7,91* |
| *% trouvé* | *58*,*60* | *6*,*13* | *7,81* |

### Enantiomère β

*Pureté optique : > 99 %*
*Point de fusion : 180-181°C*

| *Microanalyse élémentaire :* | | | |
|---|---|---|---|
| | *C* | *H* | *N* |
| *% calculé* | *58*,*75* | *6*,*26* | *7*,*91* |
| *% trouvé* | *58*,*43* | *6*,*22* | *7,79* |

### EXEMPLE 59 : Difumarate de 4-(4-octahydro-2H-pyrido[1,2-a]pyrazin-2-ylbutoxy) benzonitrile

### Stade A : 4-(4-Bromobutoxy)benzonitrile

On procède comme dans l'exemple 53, stade A, en utilisant comme substrat le 4-hydroxybenzonitrile et le 1,4-dibromobutane.

### Stade B : Difumarate de 4-(4-octahydro-2H-pyrido[1,2-a]pyrazin-2-ylbutoxy) benzonitrile

On procède comme dans l'exemple 53 en utilisant le composé obtenu lors du stade A précédent.
*Point de fusion : 208-210°C*

| *Microanalyse élémentaire :* | | | |
|---|---|---|---|
| | *C* | *H* | *N* |
| *% calculé* | *59,44* | *6*,*47* | *7,70* |
| *% trouvé* | *59*,*40* | *6*,*48* | *7,66* |

### EXEMPLE 60 : Difumarate de 4-(4-octahydro-2H-pyrido[1,2-a]pyrazin-2-ylbutoxy) benzonitrile, énantiomère α

### EXEMPLE 61 : Difumarate de 4-(4-octahydro-2H-pyrido[1,2-a]pyrazin-2-ylbutoxy) benzonitrile, énantiomère β

Les composés des exemples 60 et 61 sont obtenus à partir de 0,5 g du composé de l'exemple 59 qui sont séparés sur une colonne chromatographique chirale CHIRALPAK AD. Le fumarate est obtenu dans l'éthanol.

### Enantiomère α

*Pureté optique : > 99 %*
*Point de fusion : 208-210°C*

| *Microanalyse élémentaire :* | | | |
|---|---|---|---|
| | *C* | *H* | *N* |
| *% calculé* | *59*,*44* | *6*,*47* | *7,70* |
| *% trouvé* | *59*,*28* | *6*,*36* | *7,78* |

### Enantiomère β

*Pureté optique : > 99 %*
*Point de fusion : 209-210°C*

| *Microanalyse élémentaire :* | | | |
|---|---|---|---|
| | *C* | *H* | *N* |
| *% calculé* | *59,44* | *6,47* | *7,70* |
| *% trouvé* | *59,28* | *6,43* | *7,75* |

### EXEMPLE 62 : Trichlorhydrate de 2-[4-(2,3-dihydro-1H-indol-1-yl)butyl]octahydro-2H-pyrido[1,2-a]pyrazine, énantiomère α

### EXEMPLE 63 : Trichlorhydrate de 2-[4-(2,3-dihydro-1H-iodol-1-yl)butyl]octahydro-2H-pyrido[1,2-a]pyrazine, énantiomère β

Les composés des exemples 62 et 63 sont obtenus à partir de 1 g du composé de l'exemple 23 qui sont séparés sur une colonne chromatographique chirale CHIRALPAK AD. Le chlorhydrate est obtenu par action d'une solution d'éthanol chlorhydrique.

### Enantiomère α

*Pureté optique : > 99 %*
*Point de fusion : > 250°C*

| *Microanalyse élémentaire :* | | | | |
|---|---|---|---|---|
| | *C* | *H* | *N* | *Cl* |
| *% calculé* | *56.81* | *8,10* | *9,94* | *25,15* |
| *% trouvé* | *57,50* | *8,10* | *9,93* | *25,17* |

### Enantiomère β

*Pureté optique : > 99 %*
*Point de fusion : > 250°C*

| *Microanalyse élémentaire :* | | | | |
|---|---|---|---|---|
| | *C* | *H* | *N* | *Cl* |
| *% calculé* | *56,81* | *8,10* | *9,94* | *25,15* |
| *% trouvé* | *57,29* | *8,24* | *9,89* | *25,29* |

### EXEMPLE 64: Dichlorhydrate de 4-(4-octahydro-2H-pyrido[1,2-a]pyrazin-2-ylbutyl)-2H-1,4-benzoxazin-3(4H)-one

### Stade A : 4-(4-Bromobutyl)-2H-1,4-benzoxazin-3(4H)-one

On procède comme dans l'exemple 53, stade A, en utilisant comme substrat le 4*H*-benzo[1,4]oxazin-3-one et le 1,4-dibromobutane.

### Stade B : Dichlorhydrate de 4-(4-octahydro-2H-pyrido[1,2-a]pyrazin-2 ylbutyl)-2H-1,4-benzoxazin-3(4H)-one

On procède comme dans l'exemple 53 en utilisant le composé obtenu lors du stade A précédent.
*Point de fusion : > 250°C*

| *Microanalyse élémentaire :* | | | | |
|---|---|---|---|---|
| | *C* | *H* | *N* | *Cl* |
| *% calculé* | *57,69* | *7,50* | *10,09* | *17,03* |
| *% trouvé* | *57,59* | *7,85* | *10,01* | *17,43* |

### EXEMPLE 65 : Trichlorhydrate de 4-(4-octahydro-2H-pyrido[1,2-a]pyrazin-2-ylbutyl)-3,4-dihydro-2H-1,4-benzoxazine

On procède comme dans l'exemple 16 en utilisant comme substrat le composé obtenu dans l'exemple 64.
*Point de fusion : 203-205°C*

| *Microanalyse élémentaire :* | | | | |
|---|---|---|---|---|
| | *C* | *H* | *N* | *Cl* |
| *% calculé* | *54,74* | *7,81* | *9,57* | *24,23* |
| *% trouvé* | *54,56* | *7,94* | *9,34* | *23,63* |

### EXEMPLE 66 : Difumarate de 2-(4-octahydro-2H-pyrido[1,2-a]pyrazin-2-ylbutoxy) benzonitrile

### Stade A : 2(-4-Bromobutoxy)benzonitrile

On procède dans l'exemple 53, stade A, en utilisant comme substrat le 2-hydroxybenzonitrile et le 1,4-dibromobutane.

### Stade B : Difumarate de 2-(4-octahydro-2H-pyrido[1,2-a]pyrazin-2-ylbutoxy) benzonitrile

On procède comme dans l'exemple 53 en utilisant le composé obtenu lors du stade A précédent.
*Point de fusion : 154-155°C*

| *Microanalyse élémentaire :* | | | |
|---|---|---|---|
| | *C* | *H* | *N* |
| *% calculé* | *59*,*44* | *6*,*47* | *7,70* |
| *% trouvé* | *56,14* | *6*,*41* | *7,74* |

### EXEMPLE 67 : Difumarate de 3-(4-octahydro-2H-pyrido[1,2-a]pyrazine-2-ylbutoxy) benzonitrile

### Stade A : 3-(4-Bromobutoxy )benzonitrile

On procède comme dans l'exemple 53, stade A, en utilisant comme substrat le 3-hydroxybenzonitrile et le 1,4-dibromobutane.

### Stade B : Difumarate de 3-(4-octahydro-2H-pyrido[1,2-a]pyrazin-2-ylbutoxy) benzonitrile

On procède comme dans l'exemple 53 en utilisant le composé obtenu lors du stade A précédent.
*Point de fusion : 167-168°C*

| *Microanalyse élémentaire :* | | | |
|---|---|---|---|
| | *C* | *H* | *N* |
| *% calculé* | *59*,*44* | *6*,*47* | *7,70* |
| *% trouvé* | *59*,*33* | *6*,*36* | *7,92* |

### EXEMPLE 68 : Difumarate de 4-(2-octahydro-2H-pyrido[1,2-a]pyrazin-2-yléthoxy) benzonitrile

### Stade A : 4-(2-Bromoéthoxy)benzonitrile

On procède comme dans l'exemple 53, stade A, en utilisant comme substrat le 4-hydroxybenzonitrile et le 1,2-dibromoéthane.

### Stade B : Difumarate de 4-(2-octahydro-2H-pyrido[1,2-a]pyrazin-2-yléthoxy) benzonitrile

On procède comme dans l'exemple 53 en utilisant le composé obtenu lors du stade A précédent.
*Point de fusion : 159-160°C*

| *Microanalyse élémentaire :* | | | |
|---|---|---|---|
| | *C* | *H* | *N* |
| *% calculé* | *58,02* | *6,04* | *8,12* |
| *% trouvé* | *57,98* | *5,87* | *8,13* |

### EXEMPLE 69 : Difumarate de 3-méthoxy-4-(4-octahydro-2H-pyrido[1,2-a]pyrazin-2-ylbutoxy)benzonitrile

### Stade A : 4-(4-Bromobutoxy)-3-méthoxybenzonitrile

On procède comme dans l'exemple 53, stade A, en utilisant comme substrat le 4-hydroxy-3-méthoxybenzonitrile et le 1,4-dibromobutane.

### Stade B : Difumarate de 3-méthoxy-4-(4-octahydro-2H-pyrido[1,2-a]pyrazin-2-ylbutoxy)benzonitrile

On procède comme dans l'exemple 53 en utilisant le composé obtenu lors du stade A précédent.
*Point de fusion : 195-196°C*

| *Microanalyse élémentaire :* | | | |
|---|---|---|---|
| | *C* | *H* | *N* |
| *% calculé* | *58,43* | *6,48* | *7,30* |
| *% trouvé* | *58,30* | *6,30* | *7,48* |

### EXEMPLE 70 : Dichlorhydrate de 2-[3-(1H-indol-1-yl)propyl]octahydro-2H-pyrido[1,2-a]pyrazine

### Stade A : 1-(3-Bromopropyl)-1H-benzimidazole

On procède comme dans l'exemple 53, stade A, en utilisant comme substrat l'indole et le 1,3-dibromopropane.

### Stade B : Dichlorhydrate de 2-[3-(1H-indol-1-yl)propyl]octahydro-2H-pyrido]1,2-a]pyrazine

On procède comme dans l'exemple 53 en utilisant le composé obtenu lors du stade A précédent.
*Point de fusion : 246-247°C*

| *Microanalyse élémentaire :* | | | | |
|---|---|---|---|---|
| | *C* | *H* | *N* | *Cl* |
| *% calculé* | *61,62* | *7,89* | *11,35* | *19,14* |
| *% trouvé* | *61,25* | *7,87* | *11,25* | *19,20* |

### EXEMPLE 71 : Dichlorhydrate de 2-[4-(2,3,4-triméthoxyphénoxy)butyl]octahydro-2H-pyrido[1,2-a]pyrazine

### Stade A : 1-(4-Bromobutoxy)-2,3,4-triméthoxybenzene

On procède comme dans l'exemple 53, stade A, en utilisant comme substrat le 2,3,4-triméthoxyphénol et le 1,4-dibromobutane.

### Stade B : Dichlorhydrate de 2-[4-(2,3,4-triméthoxyphenoxy)butyl]octahydro-2H-pyrido[1,2-a]pyrazine

On procède comme dans l'exemple 53 en utilisant le composé obtenu lors du stade A précédent.
*Point de fusion : 211-212°C*

| *Microanalyse élémentaire :* | | | | |
|---|---|---|---|---|
| | *C* | *H* | *N* | *Cl* |
| *% calculé* | *55.87* | *8,04* | *6,21* | *15.71* |
| *% trouvé* | *55,43* | *7,87* | *6,26* | *16,17* |

### EXEMPLE 72 : Difumarate de 2-méthoxy-4-(4-octahydro-2H-pyrido[1,2-a]pyrazin-2-ylbutoxy)benzonitrile

### Stade A : 4-(4-Bromobutoxy)-2-méthoxybenzonitrile

On procède comme dans l'exemple 53, stade A, en utilisant comme substrat le 4-hydroxy-2-méthoxy-benzonitrile et le 1,4-dibromobutane.

### Stade B : Difumarate de 2-méthoxy-4-(4-octahydro-2H-pyrido[1,2-a]pyrazin-2-yl-butoxy)benzonitrile

On procède comme dans l 'exemple 53 en utilisant le composé obtenu lors du stade A précédent.
*Point de fusion : 164-165°C*

| *Microanalyse élémentaire :* | | | |
|---|---|---|---|
| | *C* | *H* | *N* |
| *% calculé* | *64,42* | *4,73* | *9,39* |
| *% trouvé* | *64,27* | *4,64* | *9,46* |

### EXEMPLE 73 : Dichlorhydrate de 2-[4-(1H-indol-1-yl)butyl]octahydro-2H-pyrido[1,2-a]pyrazine, énantiomère α

### EXEMPLE 74 : Dichlorhydrate de 2-[4-(1H-indol-1-yl)butyl]octahydro-2H-pyrido[1,2-a]pyrazine, énantiomère β

Les composés des exemples 73 et 74 sont obtenus à partir de 1 g du composé de l'exemple 39 qui sont séparés sur une colonne chromatographique chirale CHIRALPAK AD. Le chlorhydrate est obtenu dans l'éthanol.

### Enantiomère α

*Pureté optique : > 99 %*

### Enantiomère β

*Pureté optique : > 98 %*

### EXEMPLE 75 : Difumarate de 1-(4-octahydro-2H-pyrido[1,2-a]pyrazin-2-ylbutyl)-1H-indole-5-carbonitrile

### Stade A : 1-(4-Bromobutyl)-1H-indole-5-carbonitrile

On procède comme dans l'exemple 39 en utilisant comme substrat le 1*H*-indole-5-carbonitrile et le 1,4-dibromobutane.

### Stade B : Difumarate de 1-(4-octahydro-2H-pyrido[1,2-a]pyrazin-2-ylbutyl)-1H-indole-5-carbonitrile

On procède comme dans l'exemple 39 en utilisant comme substrat le composé obtenu lors du stade A précédent.

### EXEMPLE 76 : Dichlorhydrate de 2-[4-(5-méthoxy-1H-indol-1-yl)butyl]octahydro-2H-pyrido[1,2-a]pyrazine

### Stade A : 1-(4-Bromobutyl)-5-méthoxy-1H-indole

On procède comme dans l'exemple 39 en utilisant comme substrat le 5-méthoxy-1*H*-indole et le 1,4-dibromobutane.

### Stade B : Dichlorhydrate de 2-[4-(5-méthoxy-1H-indol-1-yl)butyl]octahydro-2H-pyrido[1,2-a]pyrazine

On procède comme dans l'exemple 39 en utilisant comme substrat le composé obtenu lors du stade A précédent.

### EXEMPLE 77 : Dichlorhydrate de 2-[2-(1H-indol-1-yl)éthyl]octahydro-2H-pyrido[1,2-a]pyrazine

### Stade A : 1-(2-Bromoéthyl)-1H-indole

On procède comme dans l'exemple 39 en utilisant comme substrat le 1*H*-indole et le 1,2-dibromoéthane.

### Stade B : Dichlorhydrate de 2-[2-(1H-indol-1-yl)éthyl]octahydro-2H-pyrido[1,2-a] pyrazine

On procède comme dans l'exemple 39 en utilisant comme substrat le composé obtenu lors du stade A précédent.

### EXEMPLE 78 : Dichlorhydrate de 2-[4-(4-bromophénoxy)butyl]octahydro-2H-pyrido[1,2-a]pyrazine

### Stade A : 1-Bromo-4-(4-bromobutoxy)benzène

On procède comme dans l'exemple 53 en utilisant comme substrat le 4-bromophénol et le 1,4-dibromobutane.

### Stade B : Dichlorhydrate de 2-[4-(4-bromophénoxy)butyl]octahydro-2H-pyrido [1,2-a]pyrazine

On procède comme dans l'exemple 53 en utilisant comme substrat le composé obtenu lors du stade A précédent.

### EXEMPLE 79 : Difumarate de 1-[4-(4-octahydro-2H-pyrido[1,2-a]pyrazin-2-ylbutoxy)phényl]éthanone

### Stade A : 2-{4-[4-(Tributylstannyl)phénoxy]butyl}octahydro-2H-pyrido[1,2-a] pyrazine

5 g de composé obtenu lors de l'exemple 78 sont portés à reflux sous atmosphère inerte 2 heures en présence de 10 ml d'hexabutyl distannane, 0,5 g de tétrakistriphénylphosphine palladium et 100 ml de toluène. Après évaporation du solvant et purification sur colonne de silice (éluant : CH₂Cl₂/MeOH, 95/5), nous obtenons le produit attendu.

### Stade B : Difumarate de 1-[4-(4-octahydro-2H-pyrido[1,2-a]pyrazin-2-ylbutoxy)phényl]éthanone

0,6 g du compose obtenu lors du stade A, 85 µl de chlorure d'acétyle, 8 mg de Pd₂dba₃ et 50 ml de toluène sont portés à reflux 1 heure sous atmosphère inerte. Le solvant est alors évaporé et le résidu purifié sur colonne de silice (éluant : CH₂Cl₂/MeOH, 95/5) pour conduire au produit attendu qui sera transformé en fumarate dans un mélange éthanol/éther.

### EXEMPLE 80 : Difumarate de cyclopropyl[4-(4-octahydro-2H-pyrido[1,2-a] pyrazin-2-ylbutoxy)phényl]méthanone

On procède comme dans l'exemple 79, stade B en utilisant le composé obtenu lors du stade A de l'exemple 79 et le chlorure de l'acide cyclopropylcarboxylique.

### EXEMPLE 81 : Difumarate de cyclohexyl[4-(4-octahydro-2H-pyrido[1,2-a]pyrazin-2-ylbutoxy)phényl]méthanone

On procède comme dans l'exemple 79, stade B en utilisant le composé obtenu lors du stade A de l'exemple 79 et le chlorure de l'acide cyclohexanecarboxylique.

### EXEMPLE 82 : Difumarate de 2-methyl-1-[4-(4-octahydro-2H-pyrido[1,2-a] pyrazin-2-ylbutoxy)phényl]-1-propanone

On procède comme dans l'exemple 79, stade B, en utilisant le composé obtenu lors du stade A de l'exemple 79 et le chlorure de l'acide 2-méthylpropanoïque.

### EXEMPLE 83 : Difumarate de [4-(4-octahydro-2H-pyrido[1,2-a]pyrazin-2-ylbutoxy)phényl](phényl)méthanone

On procède comme dans l'esemple 79, stade B en utilisant le composé obtenu lors du stade A de l'exemple 79 et le chlorure de l'acide benzoïque.

### EXEMPLE 84 : Difumarate de (1E)-1-[4-(4-octahydro-2H-pyrido[1,2-a]pyrazin-2-ylbutoxy)phényl]éthanone oxime

0,33 g du composé obtenu lors de l'exemple 79, 0,070 g de chlorhydrate d'hydroxylamine et 0,14 g de carbonate de potassium sont portés à reflux dans 20 ml d'éthanol pendant 1 heure. Le solvant est alors évaporé à sec et le résidu chromatographié sur colonne de silice (CH₂Cl₂/MeOH, 95/5). Le produit attendu est transformé en fumarate dans un mélange éthanol/éther.

### EXEMPLE 85 : Dichlorhydrate de 2-[4-(4-éthynylphénoxy)butyl]octahydro-2H-pyrido[1,2-a]pyrazine

370 mg de composé obtenu lors de l'exemple 78, 10 ml de triéthylamine, 190 mg de iodure cuivreux, 0,28 ml de triméthylsilyl acétylène et 10 mg de tétrakisphénylphosphine sont chauffés 1 heure à 60°C. Le milieu réactionnel est alors évaporé à sec puis repris dans 2 ml d'une solution 1M de TBAF dans le THF et 10 ml de dichlorométhane. Après purification par chromatographie sur silice (éluant : CH₂Cl₂/MeOH, 95/5), le résidu est transformé en chlorhydrate dans une solution d'éthanol chlorhydrique.

Les exemples suivants ont été préparés par purification par chromatographie sur colonne de silice et par chromatographie chirale en utilisant les produits de départ correspondants.

### EXEMPLE 86 : Difumarate de 4-[(4-octahydro-2H-pyrido[1,2-a]pyrazin-2-ylpentyl)oxy]benzonitrile, couple A

### EXEMPLE 86a : Difumarate de 4-[(4-octahydro-2H-pyrido[1,2-a]pyrazin-2-ylpentyl)oxy]benzonitrile, énantiomère α

### EXEMPLE 86b : Difumarate de 4-[(4-octahydro-2H-pyrido[1,2-a]pyrazin-2-ylpentyl)oxy]benzonitrile, énantiomère β

### EXEMPLE 87 : Difumarate de 4-[(4-octahydro-2H-pyrido[1,2-a]pyrazin-2-ylpentyl)oxy]benzonitrile, couple B

### EXEMPLE 87a : Difumarate de 4-[(4-octahydro-2H-pyrido[1,2-a]pyrazin-2-ylpentyl)oxy]benzonitrile, énantiomère γ

### EXEMPLE 87b : Difumarate de (4R, 9aS)-4-[(4-octahydro-2H-pyrido[1,2-a]pyrazin-2-ylpentyl)oxy]benzonitrile, énantiomère δ

### EXEMPLE 88 : Difumarate de 4-(1-méthyl-4-octahydro-2H-pyrido[1,2-a]pyrazin-2-ylbutoxy)benzonitrile, couple A

### EXEMPLE 88a : Difumarate de 4-(1-méthyl-4-octahydro-2H-pyrido[1,2-a]pyrazin-2-ylbutoxy)benzonitrile, énantiomère α

### EXEMPLE 88b : Difumarate de 4-(1-méthyl-4-octahydro-2H-pyrido[1,2-a]pyrazin-2-ylbutoxy)benzonitrile, énantiomère β

### EXEMPLE 89 : Difumarate de 4-(1-méthyl-4-octahydro-2H-pyrido[1,2-a]pyrazin-2-ylbutoxy)benzonitrile, couple B

### EXEMPLE 89a : Difumarate de 4-(1-méthyl-4-octahydro-2H-pyrido[1,2-a]pyrazin-2-ylbutoxy)benzonitrile, énantiomère γ

### EXEMPLE 89b : Difumarate de 4-(1-méthyl-4-octahydro-2H-pyrido[1,2-a]pyrazin-2-ylbutoxy)benzonitrile, énantiomère δ

### EXEMPLE 90 : Difumarate de 4-(2-méthyl-4-octahydro-2H-pyrido[1,2-a]pyrazin-2-ylbutoxy)benzonitrile, couple A

### EXEMPLE 90a : Difumarate de 4-(2-méthyl-4-octahydro-2H-pyrido[1,2-a]pyrazin-2-ylbutoxy)benzonitrile, énantiomère α

### EXEMPLE 90b : Difumarate de 4-(2-méthyl-4-octahydro-2H-pyrido[1,2-a]pyrazin-2-ylbutoxy)benzonitrile, énantiomère β

### EXEMPLE 91 : Difumarate de 4-(2-méthyl-4-octahydro-2H-pyrido[1,2-a]pyrazin-2-ylbutoxy)benzonitrile, couple B

### EXEMPLE 91a : Difumarate de 4-(2-méthyl-4-octahydro-2H-pyrido[1,2-a]pyrazin-2-ylbutoxy)benzonitrile, énantiomère γ

### EXEMPLE 91b : Difumarate de 4-(2-méthyl-4-octahydro-2H-pyrido[1,2-a]pyrazin-2-ylbutoxy)benzonitrile, énantiomère δ

### EXEMPLE 92 : Difumarate de 4-(3-méthyl-4-octahydro-2H-pyrido[1,2-a]pyrazin-2-ylbutoxy)benzonitrile, couple A

### EXEMPLE 92a : Difumarate de 4-(3-méthyl-4-octahydro-2H-pyrido[1,2-a]pyrazin-2-ylbutoxy)benzonitrile, énantiomère α

### EXEMPLE 92b : Difumarate de 4-(3-méthyl-4-octahydro-2H-pyrido[1,2-a]pyrazin-2-ylbutoxy)benzonitrile, énantiomère β

### EXEMPLE 93 : Difumarate de 4-(3-méthyl-4-octahydro-2H-pyrido[1,2-a]pyrazin-2-ylbutoxy)benzonitrile, couple B

### EXEMPLE 93a : Difumarate de 4-(3-méthyl-4-octahydro-2H-pyrido[1,2-a]pyrazin-2-ylbutoxy)benzonitrile, énantiomère γ

### EXEMPLE 93b : Difumarate de 4-(3-méthyl-4-octahydro-2H-pyrido[1,2-a]pyrazin-2-ylbutoxy)benzonitrile, énantiomère δ

### EXEMPLE 94 : Difumarate de 2-[4-(5-bromo-1H-indol-1-yl)butyl]octahydro-2H-pyrido[1,2-a]pyrazine

### Stade A : 4-Bromo-1-(4-bromobutyl)-1H-indole

On procède comme dans l'exemple 39 en utilisant comme substrat le 5-bromo-1*H*-indole et le 1,4-dibromobutane.

### Stade B : Difumarate de 2-[4-(5-bromo-1H-indol-1-yl)butyl]octahydro-2H-pyrido[1,2-a]pyrazine

On procède comme dans l'exemple 39 en utilisant comme substrat le composé obtenu lors du stade A précédent.

### EXEMPLE 95 : Difumarate de 2-{4-[5-(trifluoromethyl)-1H-indol-1-yl]butyl}octahydro-2H-pyrido[1,2-a]pyrazine

### Stade A : 1-(4-Bromobutyl)-4-(trifluoromethyl)-1H-indole

On procède comme dans l'exemple 39 en utilisant comme substrat le 5-trifluorométhyle-1*H*-indole qui est préparé selon le mode opératoire décrit dans Heterocycles, 57, 2, 2002, p. 465 et le 1,4-dibromobutane.

### Stade B : Difumarate de 2-{4-[5-(trifluorométhyl)-1H-indol-1-yl]butyl}octahydro-2H-pyrido[1,2-a]pyrazine

On procède comme dans l'exemple 39 en utilisant comme substrat le composé obtenu lors du stade A précédent.

### EXEMPLE 96 : Difumarate de 2-[4-(4,6-dichloro-1H-indol-1-yl)butyl]octahydro-2H-pyrido[1,2-a]pyrazine

### Stade A : 1-(4-Bromobutyl)-4,6-dichloro-1H-indole

On procède comme dans l'exemple 39 en utilisant comme substrat le 4,6-dichloro-1H-indole et le 1,4-dibromobutane.

### Stade B : Difumarate de 2-[4-(4,6-dichloro-1H-indol-1-yl)butyl]octahydro-2H-pyrido[1,2-a]pyrazine

On procède comme dans l'exemple 39 en utilisant comme substrat le composé obtenu lors du stade A précédent.

### EXEMPLE 97 : Difumarate de 2-[4-(5,7-dichloro-1H-indol-1-yl)butyl]octahydro-2H-pyrido[1,2-a]pyrazine

### Stade A : 1-(4-Bromobutyl)-5,7-dichloro-1H-indole

On procède comme dans l'exemple 39 en utilisant comme substrat le 5,7-dichloro-1*H*-indole et le 1,4-dibromobutane.

### Stade B : Difumarate de 2-[4-(5,7-dichloro-1H-indol-1-yl)butyl]octahydro-2H-pyrido[1,2-a]pyrazine

On procède comme dans l'exemple 39 en utilisant comme substrat le composé obtenu lors du stade A précédent.

### ETUDES PHARMACOLOGIQUES DES COMPOSES DE L'INVENTION

### EXEMPLE 98 : Dosages cérébraux de la N^{t}-Méthylhistamine chez la souris NMRI

Cette étude réalisée selon la méthode de Taylor et Coll. (Biochem. Pharm., 1992, 44, 1261-1267), a pour objectif d'évaluer l'activité *ex vivo* des composés de la présente invention en tant qu'antagonistes des récepteurs histaminergiques centraux de type H3. Cette activité est révélée par la mesure, après traitement par voie intrapéritonéale des composés sous étude, des taux centraux de N^{t}- Méthylhistamine, métabolite principal de l'histamine. Une augmentation des concentrations cérébrales de N^{t}- Méthylhistamine signe une augmentation du turn-over de l'histamine par blocage des récepteurs histaminergiques centraux de type H3.

Des souris NMRI (18-20g) sont traitées par voie intrapéritonéale par les composés de la présente invention ou par leur véhicule (20 ml/kg). Une heure après le traitement pharmacologique, les animaux sont sacrifiés, les cerveaux sont prélevés, congelés dans l'azote liquide, pesés et homogénéisés dans HClO₄ 0,1N à 4°C. Les homogénats sont centrifugés (15000g, 17 min, 4°C). Les surnageants sont récupérés et aliquotés. Les aliquotes sont congelés dans l'azotc liquide et stockés à -80°C jusqu'à leur analyse.
La détermination des taux cérébraux de N^{t}- Méthylhistamine est réalisée par dosage radio-immunologique (RIA) à l'aide d'un kit de dosage. Les taux tissulaires de N'-Méthylhistamine sont exprimés en µg/g de cerveau frais. La comparaison des taux cérébraux de N^{t}-Méthylhistamine entre les animaux traités par le véhicule (témoins) et les animaux traités par les composés de la présente invention est effectuée par une analyse de variance à un facteur suivie si nécessaire par une analyse complémentaire (test de Dunnett).

Les résultats montrent que les composés de la présente invention sont capables, pour des doses comprises entre 3 et 30 mg/kg IP, d'augmenter de 50% les concentrations cérébrales endogènes de N^{t}-Méthylhistamine. A titre indicatif, les composés des exemples 53 et 55, pour des doses de 30 mg/kg IP, augmentent de 89 % et 124 % respectivement les concentrations cérébrales endogènes de N^{t}-Méthylhistamine et, les composés des exemples 56 et 58, pour des doses de 10 mg/kg IP, augmentent de 252 % et 236 % respectivement, les contentrations cérébrales endogènes de N^{t}-Méthylhistamine. Ces résultats indiquent que les composés de la présente invention sont de puissants antagonistes des récepteurs histaminergiques centraux de type H3.

### EXEMPLE 99 : Compositions pharmaceutiques

Formule de préparation pour 1000 comprimés dosés à 100 mg :

| | |
|---|---|
| Composé de l'exemple 1 | 100 g |
| Hydroxypropylcellulose | 20 g |
| Polyvinylpyrrolidone | 20 g |
| Amidon de blé | 150 g |
| Lactose | 900 g |
| Stéarate de magnésium | 30 g |

## Revendications

1. Composés de formule (I) : 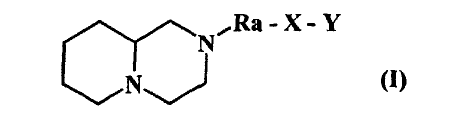 dans laquelle :
• **Ra** représente une chaîne alkylène (C₁-C₆) linéaire ou ramifié,
• **X** représente un groupement choisi parmi W₁, -C(W₁)-W₂-, -W₂-C(W₁)-, -W₂-C(W₁)W₂-, -W₂-Ra- avec Ra tel que défini précédemment, et -CH(OR₁)- dans lesquels :
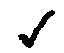 W₁ représente un atome d'oxygène, un atome de soufre, ou un groupement de formule -NR₂ dans laquelle R₂ représente un groupement choisi parmi atome d'hydrogène, alkyle (C₁-C₆) linéaire ou ramifié, aryle, arylalkyle (C₁-C₆) linéaire ou ramifié, et acyle (C₁-C₆) linéaire ou ramifié,
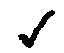 W₂ représente un groupement tel que défini pour W₁,
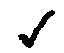 R₁ représente un groupement choisi parmi atome d'hydrogène et groupement alkyle (C₁-C₆) linéaire ou ramifié,
• lorsque **Y** représente un groupement aryle ou hétéroaryle,
ou
• **X** représente un groupement choisi parmi liaison simple,-C(W₁)-, -W₂-C(W₁)-, -W₂-Ra-, et -CH(OR₁)- dans lesquels W₁, W₂, Ra et R₁ sont tels que définis précédemment,
• lorsque **Y** représente un groupement bicyclique fusionné, de formule : 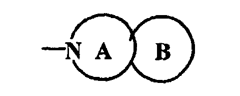 dans laquelle :
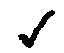 A représente un hétérocycle azoté de 4 à 7 chaînons, insaturé ou partiellement saturé, contenant éventuellement un second hétéroatome choisi parmi oxygène, azote et soufre, et éventuellement substitué par un ou plusieurs groupements choisis parmi oxo et alkyle (C₁-C₆) linéaire ou ramifié,
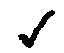 B représente un cycle phényle éventuellement substitué par un ou plusieurs groupements choisis parmi atomes d'halogènes, groupements nitro, cyano, hydroxy, alkoxy (C₁-C₆) linéaire ou ramifié, alkyle (C₁-C₆) linéaire ou ramifié, trihalogénoalkyle (C₁-C₆) linéaire ou ramifié, acyle (C₁-C₆) linéaire ou ramifié, acyl(C₁-C₆)oxy linéaire ou ramifié, carboxy, alkoxy (C₁-C₆) carbonyle linéaire ou ramifié, sulfanyle, alkylsulfanyle (C₁-C₆) linéaire ou ramifié, et amino éventuellement substitué par un ou deux groupements, identiques ou différents, choisis parmi alkyle (C₁-C₆) linéaire ou ramifié, aryle, et arylalkyle (C₁-C₆) linéaire ou ramifié,
leurs énantiomères, distéréoisomères, ainsi que leurs sels d'addition à un acide ou à une base pharmaceutiquement acceptable,
étant entendu que les composés :
- 2-octahydro-2*H*-pyrido[1,2-a]pyrazin-2-yl-1-phényléthanol,
- 3-octahydro-2*H*-pyrido[1,2-a]pyrazine-2-ylpropyl-3,4,5-triméthoxybenzoate,
- et 2-octahydro-2*H*-pyrido[1,2-a]pyrazine-2-yléthyl-3,4,5-triméthoxybenzoate,
ne font pas partie des composés de l'invention,
et étant entendu également que :
• par groupement aryle, on comprend un système aromatique monocyclique ou bicyclique, de 5 à 10 atomes de carbone, éventuellement substitué par un ou plusieurs groupements, identiques ou différents, indépendamment l'un de l'autre, choisis parmi atomes d'halogène, groupements nitro, cyano, hydroxy, alkoxy (C₁-C₆) linéaire ou ramifié, aryloxy, arylalkoxy (C₁-C₆) linéaire ou ramifié, alkyle (C₁-C₆) linéaire ou ramifié, alkényle (C₂-C₆) linéaire ou ramifié, alkynyle (C₂-C₆) linéaire ou ramifié, trihalogénoalkyle (C₁-C₆) linéaire ou ramifié, carboxy, alkoxy(C₁-C₆)carbonyle linéaire ou ramifié, acyle (C₁-C₆) linéaire ou ramifié, acyl(C₁-C₆)oxy linéaire ou ramifié, sulfanyle, alkylsulfanyle (C₁-C₆) linéaire ou ramifié, méthylènedioxy, éthylènedioxy, amide-oxime et amino éventuellement substitué par un ou deux groupements, identiques ou différents, indépendamment l'un de l'autre, choisis parmi alkyle (C₁-C₆) linéaire ou ramifié, aryle, arylalkyle (C₁-C₆) linéaire ou ramifié, et acyle (C₁-C₆) linéaire ou ramifié,
• par groupement hétéroaryle, on comprend un système aromatique monocyclique ou bicyclique, de 5 à 12 chaînons, contenant au sein du système cyclique, de 1 à 3 hétéroatomes, identiques ou différents, indépendamment l'un de l'autre, choisis parmi atome d'oxygène, d'azote et de soufre, chacun de cesdits systèmes étant éventuellement substitués par un ou plusieurs groupements, identiques ou différents, indépendamment l'un de l'autre, choisis parmi la liste des substituants précédemment décrits dans le cas d'un groupement aryle,
• et par isomère, on comprend les isomères optiques, les énantiomères et les diastéréoisomères.

2. Composés de formule (I) selon la revendication 1 **caractérisés en ce que** Ra représente une chaîne alkylène (C₂-C₅) linéaire, leurs isomères, ainsi que leurs sels d'addition à un acide ou à une base pharmaceutiquement acceptable.

3. Composés de formule (I) selon la revendication 1 **caractérisés en ce que** R₂ représente un atome d'hydrogène, un groupement alkyle (C₁-C₆) linéaire ou ramifié, ou acyle (C₁-C₆) linéaire ou ramifié, leurs isomères, ainsi que leurs sels d'addition à un acide ou à une base pharmaceutiquement acceptable.

4. Composés de formule (I) selon la revendication 1 **caractérisés en ce qu'**ils représentent des composés de formule (IA) : 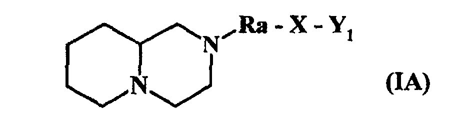 dans laquelle :
• **Ra** représente une chaîne alkylène (C₁-C₆) linéaire ou ramifié,
• **X** représente un groupement choisi parmi W₁, -C(W₁)-W₂-, -W₂-C(W₁)-, -W₂-C(W₁)W₂-, -W₂-Ra- avec Ra tel que défini précédemment, et -CH(OR₁)- dans lesquels :
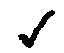 W₁ représente un atome d'oxygène, un atome de soufre, ou un groupement de formule -NR₂ dans laquelle R₂ représente un groupement choisi parmi atome d'hydrogène, alkyle (C₁-C₆) linéaire ou ramifié, aryle, arylalkyle (C₁-C₆) linéaire ou ramifié, et acyle (C₁-C₆) linéaire ou ramifié,
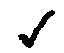 W₂ représente un groupement tel que défini pour W₁,
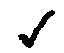 R₁ représente un groupement choisi parmi atome d'hydrogène et groupement alkyle (C₁-C₆) linéaire ou ramifié,
• **Y**_{**1**} représente un groupement aryle ou hétéroaryle.
leurs énantiomères, diastéréoisomères, ainsi que leurs sels d'addition à un acide ou à une base pharmaceutiquement acceptable.

5. Composés de formule (I) selon la revendication 4 **caractérisés en ce que** X représente un groupement choisi parmi atome d'oxygène, groupements -C(W₁)-W₂-, -W₂-C(W₁)-, et -N(R₂)- dans lesquels W₁ représente un atome d'oxygène, W₂ représente un atome d'oxygène ou un groupement -NR₂ et R₂ représente un groupement choisi parmi atome d'hydrogène, alkyle (C₁-C₆) linéaire ou ramifié, aryle, arylalkyle (C₁-C₆) linéaire ou ramifié, et acyle (C₁-C₆) linéaire ou ramifié, leurs isomères, ainsi que leurs sels d'addition à un acide ou à une base pharmaceutiquement acceptable.

6. Composés de formule (I) selon la revendication 4 **caractérisés en ce que** Y₁ représente un groupement phényle éventuellement substitué par 1 à 3 groupements choisis parmi halogène, cyano, hydroxy, alkoxy (C₁-C₆) linéaire ou ramifié, aryloxy, arylalkoxy (C₁-C₆) linéaire ou ramifié, alkyle (C₁-C₆) linéaire ou ramifié, trihalogénoalkyle (C₁-C₆) linéaire ou ramifié, méthylènedioxy et éthylènedioxy, leurs isomères, ainsi que leurs sels d'addition à un acide ou à une base pharmaceutiquement acceptable

7. Composés de formule (I) selon la revendication 1 **caractérisés en ce qu'**ils représentent des composés de formule (IB): 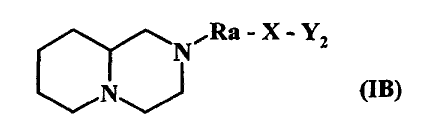 dans laquelle :
• **Ra** représente une chaîne alkylène (C₁-C₆) linéaire ou ramifiée,
• **X** représente un groupement choisi parmi liaison simple, -C(W₁)-, -W₂-C(W₁)-, -W₂-Ra- et -CH(OR₁)- dans lesquels W₁, W₂, Ra et R₁ sont tels que définis dans la formule (I),
• **Y**_{**2**} représente un groupement de formule 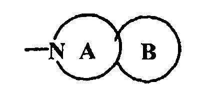
dans laquelle A et B sont tels que définis dans la formule (I),
leurs énantiomères, diastéréoisomères, ainsi que leurs sels d'addition à un acide ou à une base pharmaceutiquement acceptable.

8. Composés de formule (I) selon la revendication 7 **caractérisés en ce que** Y₂ représente un groupemcnt de formule : 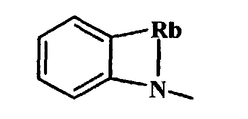 dans laquelle Rb représente une chaîne linéaire saturée ou insaturée, contenant 2 ou 3 atomes choisis parmi carbone, azote et oxygène, et/ou contenant éventuellement un groupement carbonyle, leurs isomères, ainsi que leurs sels d'addition à un acide ou à une base pharmaceutiquement acceptable.

9. Composés de formule (I) selon la revendication 7 **caractérisés en ce que** Y₂ représente un groupement choisi parmi 2,3-dihydro-1*H*-indol-1-yl, 1*H*-indol-1-yl, 1*H*-indazol-1-yl, 1*H*-benzimidazol-1-yl, 3,4-dihydro-2*H*-quinolin-1-yl, 2,3-dihydro-1*H*-4-quinolinone-1-yl, 3,4-dihydro-2*H*-quinoxalin-1-yl, et 2,3-dihydro-4*H*-1,4-benzoxazin-4-yl, leurs isomères, ainsi que leurs sels d'addition à un acide ou à une base pharmaceutiquement acceptable.

10. Composés de formule (I) selon la revendication 7 **caractérisés en ce que** X représente une liaison simple, leurs isomères, ainsi que leurs sels d'addition à un acide ou à une base pharmaceutiquement acceptable.

11. Composés de formule (I) selon la revendication 1 qui sont le
- 2-[4-(2,3-dihydro-1*H*-indol-1-yl)butyl]octahydro-2*H*-pyrido[1,2-*a*] pyrazine,
- 2-[4-(3,4,5-triméthoxyphénoxy)butyl]octahydro-2*H*-pyrido[1,2-*a*] pyrazine,
- 2-[4-(3,4,5-triméthoxyphénoxy)butyl]octahydro-2*H*-pyrido[1,2-*a*] pyrazine, énantiomère α,
- 2-[4-(3,4,5-triméthoxyphénoxy)butyl]octahydro-2*H*-pyrido[1,2-*a*] pyrazine, énantiomère β,
- *N*-(4-octahydro-2*H*-pyrido[1,2-*a*]pyrazin-2-ylbutyl)-*N*-(3,4,5-triméthoxyphényl)amine,
- *N*-(4-octahydro-2*H*-pyrido[1,2-*a*]pyrazin-2-ylbutyl)-*N*-(3,4,5-triméthoxyphényl)amine, énantiomère α,
- *N*-(4-octahydro-2*H*-pyrido[1,2-*a*]pyrazin-2-ylbutyl)-*N*-(3,4,5-triméthoxyphényl)amine, énantiomère β,
- *N*-(4-trifluorophényl)-*N*-(4-octahydro-2*H*-pyrido[1,2-*a*]pyrazin-2-ylbutyl)amine,
- *N*-(3,4-dichloro-phényl)-*N*-(4-octahydro-2*H*-pyrido[1,2-*a*]pyrazin-2-ylbutyl)amine,
- *N*-(3,5-dichloro-phényl)-*N*-(4-octahydro-2*H*-pyrido[1,2-*a*]pyrazin-2-ylbutyl)amine,
- *N*-(2-chlorophényl)-*N*-(4-octahydro-2*H*-pyrido[1,2-*a*]pyrazin-2-ylbutyl)amine,
- 2-[4-(3,4-dihydro-1(2*H*)-quinolinyl)butyl]octahydro-2*H*-pyrido[1,2-*a*] pyrazine,
- 2-[4-(1*H*-benzimidazol-1-yl)butyl]octahydro-2*H*-pyrido[1,2-*a*] pyrazine,
- 2-[4-(1*H*-indol-1-yl)butyl]octahydro-2*H*-pyrido[1,2-*a*]pyrazine,
- 2-[4-(1*H*-indazol-1-yl)butyl]octahydro-2*H*-pyrido[1,2-*a*]pyrazine,
- 2-[4-(2,3-dihydro-1*H*-indol-1-yl)butyl] octahydro-2*H*-pyrido[1,2-*a*]pyrazine, énantiomère α,
- 2-[4-(2,3-dihydro-1*H*-indol-1-yl)butyl] octahydro-2*H*-pyrido[1,2-*a*]pyrazine, énantiomère β,
- 3-(4-octahydro-2*H*-pyrido[1,2-*a*]pyrazin-2-ylbutoxy)benzonitrile,
- 3-methoxy-4-(4-octahydro-2*H*-pyrido[1,2-*a*]pyrazin-2-ylbutoxy) benzonitrile,
- 2-[4-(2,3,4-trimethoxyphenoxy)butyl]octahydro-2*H*-pyrido[1,2-*a*] pyrazine.
leurs énantiomères, diastéréoisomères, ainsi que leurs sels d'addition à un acide ou à une base pharmaceutiquement acceptable.

12. Composés de formule (I) selon la revendication 1 qui sont le :
- 4-[(4-octahydro-2*H*-pyrido[1,2-*a*]pyrazin-2-ylbutyl)amino]benzonitrile,
- 4-(3-octahydro-2*H*-pyrido[1,2-*a*]pyrazin-2-ylpropoxy)benzonitrile,
- 4-(3-octahydro-2*H*-pyrido[1,2-*a*]pyrazin-2-ylpropoxy)benzonitrile, énantiomère α,
- 4-(3-octahydro-2*H*-pyrido[1,2-*a*]pyrazin-2-ylpropoxy)benzonitrile, énantiomère β,
- 4-(4-octahydro-2*H*-pyrido[1,2-*a*]pyrazin-2-ylbutoxy)benzonitrile,
- 4-(4-octahydro-2*H*-pyrido[1,2-*a*]pyrazin-2-ylbutoxy)benzonitrile, énantiomère α,
- 4-(4-octahydro-2*H*-pyrido[1,2-*a*]pyrazin-2-ylbutoxy)benzonitrile, énantiomère β,
- 4-(2-octahydro-2*H*-pyrido[1,2-*a*]pyrazin-2-yléthoxy)benzonitrile.
leurs énantiomères, diastéréoisomères, ainsi que leurs sels d'addition à un acide ou à une base pharmaceutiquement acceptable.

13. Procédé de préparation des composés de formule (I) selon la revendication 1 **caractérisé en ce qu'**on utilise comme produit de départ un composé de formule (II) : 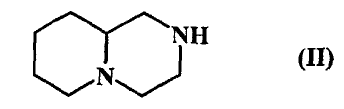 composé de formule (II) qui est mis à réagir, en condition basique :
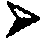 *soit avec un composé de formule (III)* : 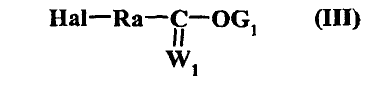 dans laquelle Hal représente un atome d'halogène, Ra est tel que défini dans la formule (I) et G₁ représente un groupement alkyle (C₁-C₆) linéaire ou ramifié,
pour conduire aux composés de formule (IV) : 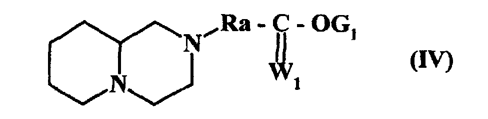 dans laquelle Ra et G₁ sont tels que définis précédemment,
composés de formule (IV) qui sont saponifiés puis qui sont mis à réagir en présence d'hydrochlorure de 1-(3-diméthylaminopropyl)-3-éthylcarbodiimide, et d'une amine tertiaire, avec un composé de formule (V) :
**Y**_{**1**}**-W**_{**2**}**H (V)**
dans laquelle Y₁ représente un groupement aryle, ou hétéroaryle, et W₂ est tel que défini dans la formule (I)
pour conduire aux composés de formule (I/a), cas particulier des composés de formule (I) : 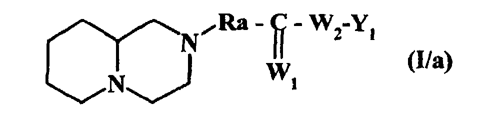 dans laquelle Ra, W₁, W₂ et Y₁ sont tels que définis précédemment,
composés de formule (I/a), dans le cas particulier où W₁ représente un atome d'oxygène et Ra prend la définition particulière R'a et représente un chaîne alkylène (C₁-C₅) linéaire ou ramifiée, qui peuvent être réduits de façon sélective par action d'un réducteur classique utilisé en synthèse organique, pour conduire aux composés de formule (I/b), cas particulier des composés de formule (I) : 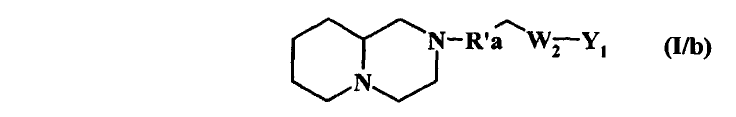 dans laquelle R'a représente une chaîne alkylène (C₁-C₅) linéaire ou ramifiée, W₂ et Y₁ sont tels que définis précédemment,
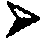 *soit avec un composé de formule (VI) :*
**Hal-Ra**_{**1**}**-CN (VI)**
dans laquelle Hal représente un atome d'halogène, et Ra₁ représente un groupement alkyle (C₁-C₅) linéaire ou ramifié ou une liaison,
pour conduire aux composés de formule (VII) : 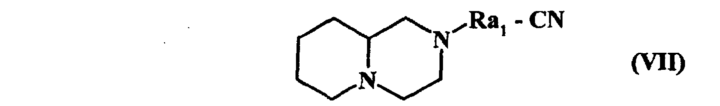 dans laquelle Ra₁ est tel que défini précédemment, composés de formule (VII) dont la fonction cyano est réduite selon des conditions classiques en amine primaire, puis qui sont traités :
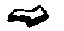 soit dans l'éthanol en présence d'un composé de formule (VIII) :
**Y**_{**1**}**-N=C=W**_{**3**} **(VIII)**
dans laquelle Y₁ est tel que défini précédemment et W₃ représente un atome d'oxygène ou de soufre,
pour conduire aux composés de formule (I/c), cas particulier des composés de formule (I) : 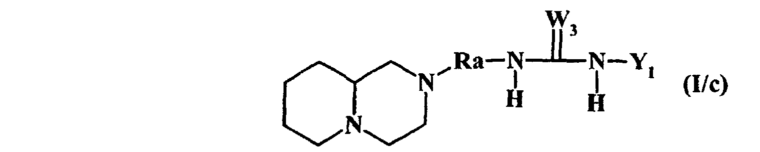 dans laquelle Ra, Y₁ et W₃ sont tels que définis précédemment,
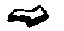 soit en condition de couplage par un composé de formule (IX) : 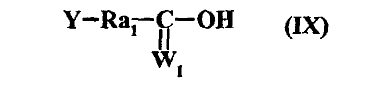 dans laquelle Y et W₁ sont tels que définis dans la formule (I) et Ra₁ est tel que défini précédemment, pour conduire :
• dans le cas où Ra₁ représente une liaison, aux composés de formule (I/d), cas particulier des composés de formule (I) : 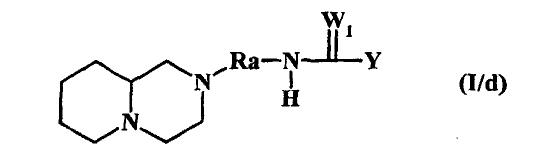 dans laquelle Ra, W₁ et Y sont tels que définis précédemment,
composés de formule (I/d) qui sont traités en présence d'hydrure de sodium avec un composé de formule (X) :
**R'**_{**2**}**-Hal (X)**
dans laquelle Hal représente un atome d'halogène et R'₂ a la même définition que R₂ dans la formule (I) à l'exception de la définition atome d'hydrogène,
pour conduire aux composés de formule (I/e), cas particulier des composés de formule (I): 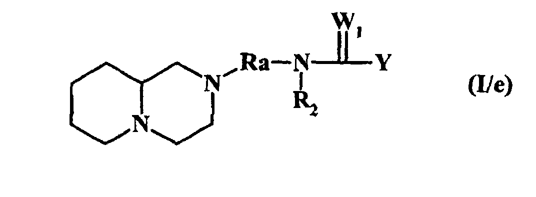 dans laquelle Ra, R₂, W₁ et Y sont tels que définis précédemment,
• soit dans le cas où Ra₁ représente une chaîne alkylène (C₁-C₅) linéaire ou ramifiée, aux composés de formule (XI) : 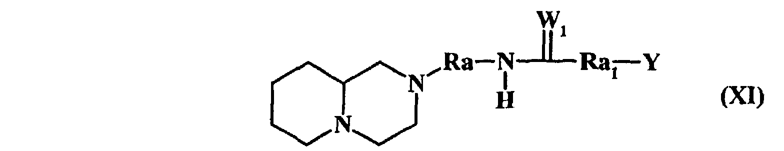 composés de formule (XI) qui sont placés en présence d'un agent réducteur classiquement utilisé en synthèse organique, pour conduire aux composés de formule (I/f), cas particulier des composés de formule (I) : 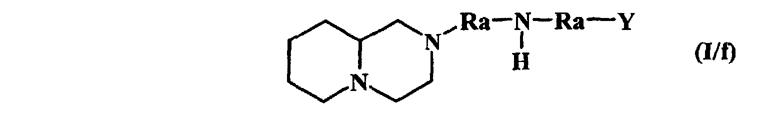
dans laquelle Ra et Y sont tels que définis dans la formule (I),
composés de formule (I/f) qui peuvent être soumis à l'action d'un composé de formule (X) tel que décrit précédemment, pour conduire aux composés de formule (I/g), cas particulier des composés de formule (I) : 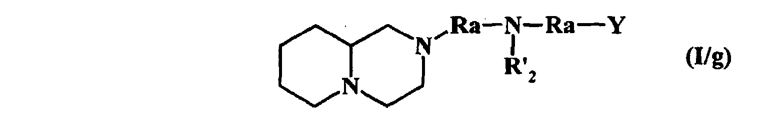 dans laquelle Ra, R'₂ et Y sont tels que décrits précédemment,
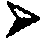 *soit avec un composé de formule (XII) :*
**Y-Ra-W**_{**3**}**-Ra-OTs (XII)**
dans laquelle Y et Ra sont tels que définis dans la formule (I) et W₃ représente un atome d'oxygène ou de soufre,
pour conduire aux composés de formule (I/h), cas particulier des composés de formule (I) : 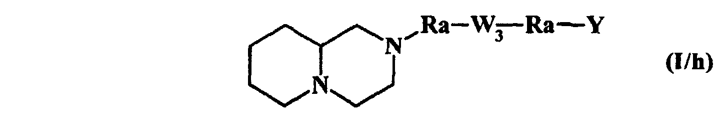 dans laquelle Ra, Y et W₃ sont tels que définis précédemment,
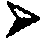 *soit avec un composés de formule (XIII) :* 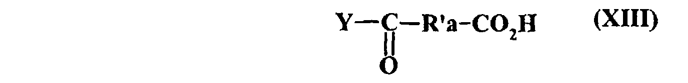 dans laquelle Y et R'a sont tels que définis dans la formule (I),
pour conduire aux composés de formule (XIV) : 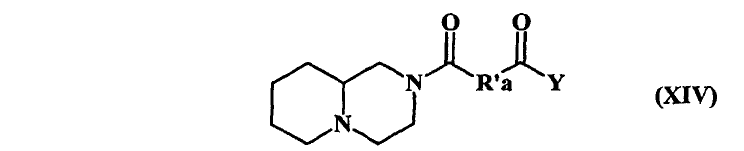 dans laquelle R'a et Y sont tels que définis précédemment,
composés de formule (XIV) qui sont placés en présence d'un agent réducteur, pour conduire aux composés de formule (I/i), cas particulier des composés de formule (I) : 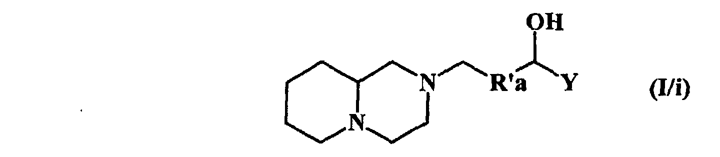 dans laquelle R'a et Y sont tels que définis dans la formule (I),
composés de formule (I/i), dans le cas où Y représente spécifiquement un groupement Y₂ tel que décrit précédemment, qui sont oxydés en présence de diméthylsulfoxyde, de triéthylamine et de chlorure d'oxalyle, pour conduire aux composés de formule (I/j), cas particulier des composés de formule (I) : 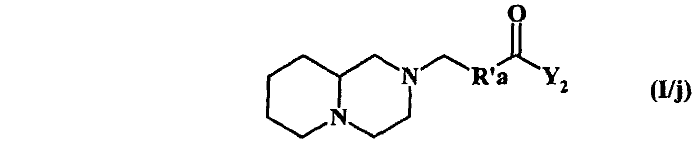 dans laquelle R'a et Y₂ sont tels que définis précédemment,
ou composé de formule (I/i), qui est mis à réagir avec un composé de formule (XV) :
**R'**_{**1**}**-Hal (XV)**
dans laquelle Hal représente un atome d'halogène et R'₁ prend les mêmes définitions que R₁ à l'exception de la définition atome d'hydrogène,
pour conduire aux composés de formule (I/k), cas particulier des composés de formule (I) : 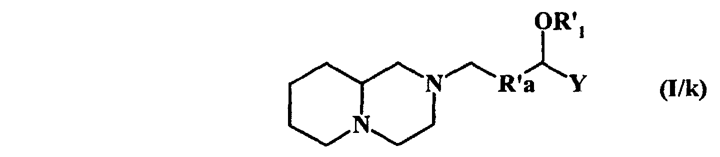 dans laquelle R'a, R'₁ et Y sont tels que définis précédemment,
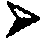 *soit avec un composés de formule (XVI) :*
**Hal-Ra-W**_{**2**}**H (XVI)**
dans laquelle Hal représente un atome d'halogène, Ra et W₂ sont tels que définis dans la formule (I)
pour conduire aux composés de formule (XVII) : 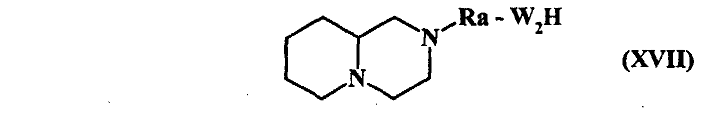 dans laquelle Ra et W₂ sont tels que définis précédemment,
composés de formule (XVII) qui sont traités par un composé de formule (XVIII) : 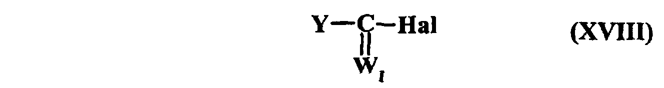 dans laquelle Hal représente un atome d'halogène, Y et W₁ sont tels que définis dans la formule (I) :
pour conduire aux composés de formule (I/l), cas particulier des composés de formule (I) : 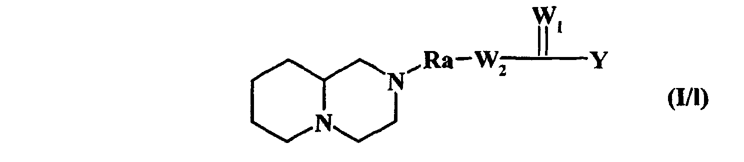 dans laquelle Ra, W₂, W₁ et Y sont tels que définis précédemment,
les composés de formule (I/a) à (I/l) formant l'ensemble des composés de l'invention, que l'on purifie le cas échéant, selon des techniques classiques de purification, dont on sépare, éventuellement, les isomères, selon une technique classique de séparation, et que l'on transforme, si on le souhaite en leurs sels d'addition à un acide ou à une base pharmaceutiquement acceptable.

14. Compositions pharmaceutiques contenant comme principe actif au moins un composé selon l'une quelconque des revendications 1 à 12, seul ou en combinaison avec un ou plusieurs excipients ou véhicules inertes, non toxiques, pharmaceutiquement acceptables.

15. Compositions pharmaceutiques selon la revendication 14 contenant au moins un principe actif selon l'une quelconque des revendications 1 à 12 utiles en tant qu'antagoniste des récepteurs histaminergiques centraux de type H3.

16. Compositions pharmaceutiques selon la revendication 14 contenant au moins un principe actif selon l'une quelconque des revendications 1 à 12 utiles, en tant que médicament, dans le traitement des déficits cognitifs associés au vieillissement cérébral et aux maladies neurodégénératives, ainsi que dans le traitement des troubles de l'humeur, des crises convulsives, du syndrome d'hyperactivité avec déficits attentionnels, de l'obésité et de la douleur.

17. Compositions pharmaceutiques selon la revendication 14 contenant au moins un principe actif selon l'une quelconque des revendications 1 à 12 utiles, en tant que médicament, dans le traitement des déficits cognitifs associés à la maladie d'Alzheimer, la maladie de Parkinson, la maladie de Pick, la maladie de Korsakoff, et les démences frontales et sous-corticales d'origines vasculaires ou autres.

## Claims

1. Compounds of formula (I) : 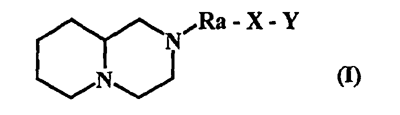 wherein :
• **Ra** represents a linear or branched (C₁-C₆)alkylene chain,
• **X** represents a group selected from W₁, -C(W₁)-W₂-, -W₂-C(W₁)-, -W₂-C(W₁)W₂-, -W₂-Ra- wherein Ra is as defined hereinbefore, and -CH(OR₁)- wherein :
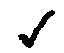 W₁ represents an oxygen atom, a sulphur atom, or a group of formula -NR₂ wherein R₂ represents a group selected from a hydrogen atom, linear or branched (C₁-C₆)-alkyl, aryl, aryl-(C₁-C₆)alkyl in which the alkyl moiety is linear or branched, and linear or branched (C₁-C₆)acyl,
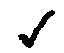 W₂ represents a group as defined for W₁,
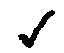 R₁ represents a group selected from a hydrogen atom and a linear or branched (C₁-C₆)alkyl group,
• when **Y** represents an aryl or heteroaryl group,
or
• **X** represents a group selected from a single bond, -C(W₁)-, -W₂-C(W₁)-, -W₂-Ra- and -CH(OR₁)- wherein W₁, W₂, Ra and R₁ are as defined hereinbefore,
• when **Y** represents a fused bicyclic group, of formula : 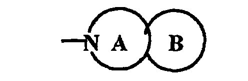 wherein :
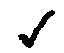 A represents a nitrogen-containing heterocycle containing from 4 to 7 ring members that is unsaturated or partially saturated and optionally contains a second hetero atom selected from oxygen, nitrogen and sulphur, and is optionally substituted by one or more groups selected from oxo and linear or branched (C₁-C₆)alkyl,
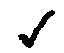 B represents a phenyl ring optionally substituted by one or more groups selected from halogen atoms and the groups nitro, cyano, hydroxy, linear or branched (C₁-C₆)alkoxy, linear or branched (C₁-C₆)alkyl, linear or branched trihalo-(C₁-C₆)-alkyl, linear or branched (C₁-C₆)acyl, linear or branched (C₁-C₆)acyloxy, carboxy, linear or branched (C₁-C₆)alkoxy-carbonyl, sulphanyl, linear or branched (C₁-C₆)-alkylsulphanyl, and amino optionally substituted by one or two identical or different groups selected from linear or branched (C₁-C₆)alkyl, aryl and aryl-(C₁-C₆)alkyl in which the alkyl moiety is linear or branched,
their enantiomers, diastereoisomers, and addition salts thereof with a pharmaceutically acceptable acid or base,
with the proviso that the compounds :
- 2-octahydro-2*H*-pyrido[1,2-a]pyrazin-2-yl-1-phenylethanol,
- 3-octahydro-2*H*-pyrido[1,2-a]pyrazin-2-ylpropyl-3,4,5-trimethoxybenzoate, and
- 2-octahydro-2*H*-pyrido[1,2-a]pyrazin-2-ylethyl-3,4,5-trimethoxybenzoate,
are not included in the compounds of the invention,
and it also being understood that :
• "aryl group" is understood to mean a monocyclic or bicyclic aromatic system, containing from 5 to 10 carbon atoms, optionally substituted by one or more identical or different groups each independently of the others selected from halogen atoms and the groups nitro, cyano, hydroxy, linear or branched (C₁-C₆)alkoxy, aryloxy, aryl-(C₁-C₆)-alkoxy in which the alkoxy moiety is linear or branched, linear or branched (C₁-C₆)alkyl, linear or branched (C₂-C₆)alkenyl, linear or branched (C₂-C₆)alkynyl, linear or branched trihalo-(C₁-C₆)alkyl, carboxy, linear or branched (C₁-C₆)alkoxy-carbonyl, linear or branched (C₁-C₆)acyl, linear or branched (C₁-C₆)acyloxy, sulphanyl, linear or branched (C₁-C₆)alkylsulphanyl, methylenedioxy, ethylenedioxy, amide-oxime and amino optionally substituted by one or two identical or different groups each independently of the other selected from linear or branched (C₁-C₆)alkyl, aryl, aryl-(C₁-C₆)alkyl in which the alkyl moiety is linear or branched, and linear or branched (C₁-C₆)acyl,
• "heteroaryl group" is understood to mean a monocyclic or bicyclic aromatic system, containing from 5 to 12 ring members, and containing within the same ring system from 1 to 3 identical or different hetero atoms each independently of the others selected from an oxygen atom, a nitrogen atom and a sulphur atom, each of the said systems being optionally substituted by one or more identical or different groups each independently of the others selected from the list of substituents described above in the case of an aryl group, and
• "isomer" is understood to include the optical isomers, enantiomers and diastereoisomers.

2. Compounds of formula (I) according to claim 1, **characterised in that** Ra represents a linear (C₂-C₅)alkylene chain, their isomers, and addition salts thereof with a pharmaceutically acceptable acid or base.

3. Compounds of formula (I) according to claim 1, **characterised in that** R₂ represents a hydrogen atom, a linear or branched (C₁-C₆)alkyl group, or a linear or branched (C₁-C₆)-acyl group, their isomers, and addition salts thereof with a pharmaceutically acceptable acid or base.

4. Compounds of formula (I) according to claim 1, **characterised in that** they represent compounds of formula (IA) : 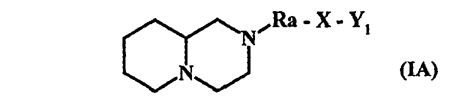 wherein :
• **Ra** represents a linear or branched (C₁-C₆)alkylene chain,
• **X** represents a group selected from W₁, -C(W₁)-W₂-, -W₂-C(W₁)-, -W₂-C(W₁)W₂-, -W₂-Ra- wherein Ra is as defined hereinbefore, and -CH(OR₁)- wherein :
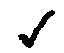 W₁ represents an oxygen atom, a sulphur atom or a group of formula -NR₂ wherein R₂ represents a group selected from a hydrogen atom, linear or branched (C₁-C₆)-alkyl, aryl, aryl-(C₁-C₆)alkyl in which the alkyl moiety is linear or branched, and linear or branched (C₁-C₆)acyl,
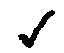 W₂ represents a group as defined for W₁,
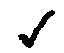 R₁ represents a group selected from a hydrogen atom and a linear or branched (C₁-C₆)alkyl group,
• **Y**_{**1**} represents an aryl or heteroaryl group,
their enantiomers, diastereoisomers, and addition salts thereof with a pharmaceutically acceptable acid or base.

5. Compounds of formula (I) according to claim 4, **characterised in that** X represents a group selected from an oxygen atom, and groups -C(W₁)-W₂-, -W₂-C(W₁)- and -N(R₂)-wherein W₁ represents an oxygen atom, W₂ represents an oxygen atom or a group -NR₂ and R₂ represents a group selected from a hydrogen atom, linear or branched (C₁-C₆)alkyl, aryl, aryl-(C₁-C₆)alkyl in which the alkyl moiety is linear or branched, and linear or branched (C₁-C₆)acyl, their isomers, and addition salts thereof with a pharmaceutically acceptable acid or base.

6. Compounds of formula (I) according to claim 4, **characterised in that** Y₁ represents a phenyl group optionally substituted by from 1 to 3 groups selected from halogen, cyano, hydroxy, linear or branched (C₁-C₆)alkoxy, aryloxy, aryl-(C₁-C₆)alkoxy in which the alkoxy moiety is linear or branched, linear or branched (C₁-C₆)alkyl, linear or branched trihalo-(C₁-C₆)-alkyl, methylenedioxy and ethylenedioxy, their isomers, and addition salts thereof with a pharmaceutically acceptable acid or base.

7. Compounds of formula (I) according to claim 1, **characterised in that** they represent compounds of formula (IB): 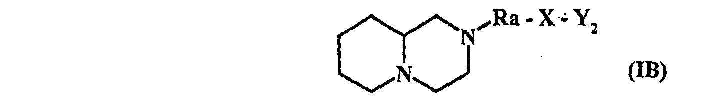 wherein :
**Ra** represents a linear or branched (C₁-C₆)alkylene chain,
• **X** represents a group selected from a single bond, -C(W₁)-, -W₂-C(W₁)-, -W₂-Ra- and -CH(OR₁)- wherein W₁, W₂, Ra and R₁ are as defined for formula (I),
• **Y**_{**2**} represents a group of formula : 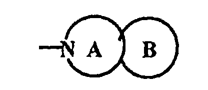
wherein A and B are as defined for formula (I),
their enantiomers, diastereoisomers, and addition salts thereof with a pharmaceutically acceptable acid or base.

8. Compounds of formula (I) according to claim 7, **characterised in that** Y₂ represents a group of formula: 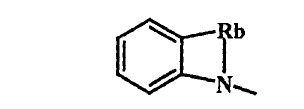 wherein Rb represents a saturated or unsaturated linear chain, containing 2 or 3 atoms selected from carbon, nitrogen and oxygen, and/or optionally containing a carbonyl group, their isomers, and addition salts thereof with a pharmaceutically acceptable acid or base.

9. Compounds of formula (I) according to claim 7, **characterised in that** Y₂ represents a group selected from 2,3-dihydro-1*H*-indol-1-yl, 1*H*-indol-1-yl, 1*H*-indazol-1-yl, 1*H*-benzimidazol-1-yl, 3,4-dihydro-2*H*-quinol-1-yl, 2,3-dihydro-1*H*-4-quinolon-1-yl, 3,4-dihydro-2*H*-quinoxalin-1-yl and 2,3-dihydro-4*H*-1,4-benzoxazin-4-yl, their isomers, and addition salts thereof with a pharmaceutically acceptable acid or base.

10. Compounds of formula (I) according to claim 7, **characterised in that** X represents a single bond, their isomers, and addition salts thereof with a pharmaceutically acceptable acid or base.

11. Compounds of formula (1) according to claim 1 which are :
- 2-[4-(2,3-dihydro-1*H*-indol-1-yl)butyl]octahydro-2*H*-pyrido[1,2-*a*]pyrazine,
- 2-[4-(3,4,5-trimethoxyphenoxy)butyl]octahydro-2*H*-pyrido[1,2-*a*]pyrazine,
- 2-[4-(3,4,5-trimethoxyphenoxy)butyl]octahydro-2*H*-pyrido[1,2-*a*]pyrazine, α enantiomer,
- 2-[4-(3,4,5-trimethoxyphenoxy)butyl]octahydro-2*H*-pyrido[1,2-*a*]pyrazine, β enantiomer,
- *N*-(4-octahydro-2*H*-pyrido[1,2-*a*]pyrazin-2-ylbutyl)-*N*-(3,4,5-trimethoxyphenyl)amine,
- *N*-(4-octahydro-2*H*-pyrido[1,2-*a*]pyrazin-2-ylbutyl)-*N*-(3,4,5-trimethoxyphenyl)amine, α enantiomer,
- *N*-(4-octahydro-2*H*-pyrido[1,2-*a*]pyrazin-2-ylbutyl)-*N*-(3,4,5-trimethoxyphenyl)amine, β enantiomer,
- *N*-(4-trifluorophenyl)-*N*-(4-octahydro-2*H*-pyrido[1,2-*a*]pyrazin-2-ylbutyl)amine,
- *N*-(3,4-dichloro-phenyl)-*N*-(4-octahydro-2*H*-pyrido[1,2-*a*]pyrazin-2-ylbutyl)amine,
- *N*-(3,5-dichloro-phenyl)-*N*-(4-octahydro-2*H*-pyrido[1,2-*a*]pyrazin-2-ylbutyl)amine,
- *N*-(2-chlorophenyl)-*N*-(4-octahydro-2*H*-pyrido[1,2-*a*]pyrazin-2-ylbutyl)amine,
- 2-[4-(3,4-dihydro-1(2*H*)-quinolyl)butyl]octahydro-2*H*-pyrido[1,2-*a*]pyrazine,
- 2-[4-(1*H*-benzimidazol-1-yl)butyl]octahydro-2*H*-pyrido[1,2-*a*]pyrazine,
- 2-[4-(1*H*-indol-1-yl)butyl]octahydro-2*H*-pyrido[1,2-*a*]pyrazine,
- 2-[4-(1*H*-indazol-1-yl)butyl]octahydro-2*H*-pyrido[1,2-*a*]pyrazine,
- 2-[4-(2,3-dihydro-1*H*-indol-1-yl)butyl]octahydro-2*H*-pyrido[1,2-*a*]pyrazine, α enantiomer,
- 2-[4-(2,3-dihydro-1*H*-indol-1-yl)butyl]octahydro-2*H*-pyrido[1,2-*a*]pyrazine, β enantiomer,
- 3-(4-octahydro-2*H*-pyrido[1,2-*a*]pyrazin-2-ylbutoxy)benzonitrile,
- 3-methoxy-4-(4-octahydro-2*H*-pyrido[1,2-*a*]pyrazin-2-ylbutoxy)benzonitrile,
- 2-[4-(2,3,4-trimethoxyphenoxy)butyl]octahydro-2*H*-pyrido[1,2-*a*]pyrazine,
their enantiomers, diastereoisomers, and addition salts thereof with a pharmaceutically acceptable acid or base.

12. Compounds of formula (I) according to claim 1 which are :
- 4-[(4-octahydro-2*H*-pyrido[1,2-*a*]pyrazin-2-ylbutyl)amino]benzonitrile,
- 4-(3-octahydro-2*H*-pyrido[1,2-*a*]pyrazin-2-ylpropoxy)benzonitrile,
- 4-(3-octahydro-2*H*-pyrido[1,2-*a*]pyrazin-2-ylpropoxy)benzonitrile, α enantiomer,
- 4-(3-octahydro-2*H*-pyrido[1,2-*a*]pyrazin-2-ylpropoxy)benzonitrile, β enantiomer,
- 4-(4-octahydro-2*H*-pyrido[1,2-*a*]pyrazin-2-ylbutoxy)benzonitrile,
- 4-(4-octahydro-2*H*-pyrido[1,2-*a*]pyrazin-2-ylbutoxy)benzonitrile, a enantiomer,
- 4-(4-octahydro-2*H*-pyrido[1,2-*a*]pyrazin-2-ylbutoxy)benzonitrile, β enantiomer,
- 4-(2-octahydro-2*H*-pyrido[1,2-*a*]pyrazin-2-ylethoxy)benzonitrile,
their enantiomers, diastereoisomers, and addition salts thereof with a pharmaceutically acceptable acid or base.

13. Process for the preparation of compounds of formula (I) according to claim 1, **characterised in that** there is used as starting material a compound of formula (II) : 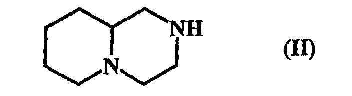 which compound of formula (II) is reacted, under basic conditions :
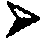 *with a compound of formula (III)* : 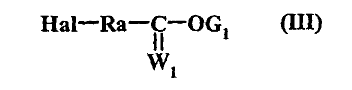 wherein Hal represents a halogen atom, Ra is as defined for formula (I) and G₁ represents a linear or branched (C₁-C₆)alkyl group,
to yield compounds of formula (IV) : 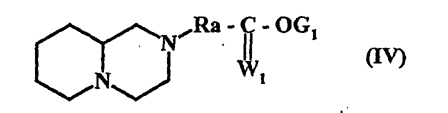 wherein Ra and G₁ are as defined hereinbefore,
which compounds of formula (IV) are hydrolysed and then reacted, in the presence of 1-(3-dimethylaminopropyl)-3-ethylcarbodiimide hydrochloride and a tertiary amine, with a compound of formula (V) :
**Y**_{**1**}**-W**_{**2**}**H (V)**
wherein Y₁ represents an aryl or heteroaryl group, and W₂ is as defined for formula (I),
to yield compounds of formula (I/a), a particular case of the compounds of formula (I) : 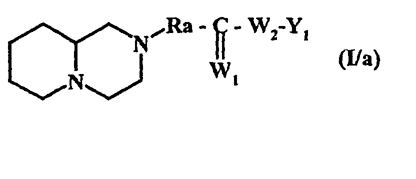 wherein Ra, W₁, W₂ and Y₁ are as defined hereinbefore,
which compounds of formula (I/a), in the particular case when W₁ represents an oxygen atom and Ra has the specific meaning R'a and represents a linear or branched (C₁-C₅)-alkylene chain, may be reduced selectively by the action of a conventional reducing agent used in organic synthesis, to yield compounds of formula (I/b), a particular case of the compounds of formula (I) : 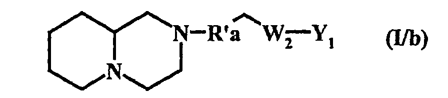 wherein R'a represents a linear or branched (C₁-C₅)alkylene chain, W₂ and Y₁ are as defined hereinbefore,
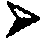 *or with a compound of formula (VI) :*
**Hal-Ra**_{**1**}**-CN (VI)**
wherein Hal represents a halogen atom, and Ra₁ represents a linear or branched (C₁-C₅)-alkyl group or a bond,
to yield compounds of formula (VII) : 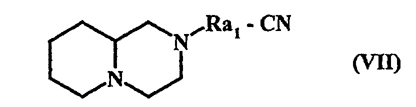 wherein Ra₁ is as defined hereinbefore,
the cyano function of which compounds of formula (VII) is reduced in accordance with conventional conditions to a primary amine, which compounds are then treated :
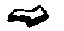 in ethanol, with a compound of formula (VIII) :
**Y**_{**1**}**-N=C=W**_{**3**} **(VIII)**
wherein Y₁ is as defined hereinbefore and W₃ represents an oxygen atom or a sulphur atom,
to yield compounds of formula (I/c), a particular case of the compounds of formula (I) : 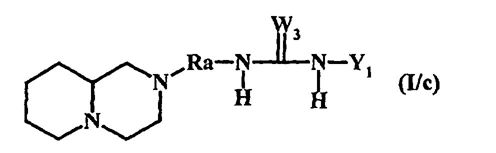 wherein Ra, Y₁ and W₃ are as defined hereinbefore,
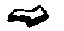 or, under coupling conditions, with a compound of formula (IX) : 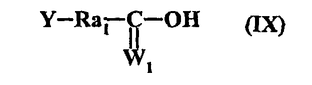 wherein Y and W₁ are as defined for formula (I) and Ra₁ is as defined hereinbefore,
to yield :
• in the case when Ra₁ represents a bond, compounds of formula (I/d), a particular case of the compounds of formula (I) : 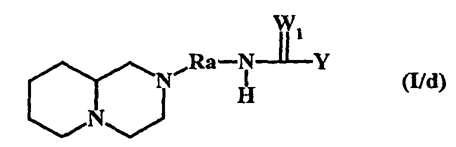 wherein Ra, W₁ and Y are as defined hereinbefore,
which compounds of formula (I/d) are treated in the presence of sodium hydride with a compound of formula (X) :
**R'**_{**2**}**-Hal (X)**
wherein Hal represents a halogen atom and R'₂ is as defined for R₂ in formula (I) with the exception of the definition of a hydrogen atom,
to yield compounds of formula (I/e), a particular case of the compounds of formula (I) : 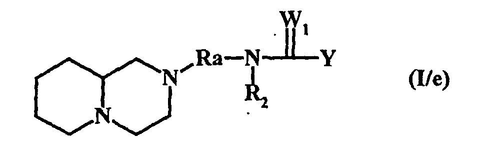 wherein Ra, R₂, W₁ and Y are as defined hereinbefore,
• or in the case when Ra₁ represents a linear or branched (C₁-C₅)alkylene chain, compounds of formula (XI) : 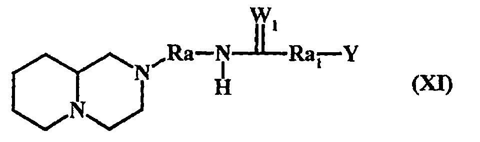 which compounds of formula (XI) are treated with a reducing agent conventionally used in organic synthesis, to yield compounds of formula (I/f), a particular case of the compounds of formula (I) : 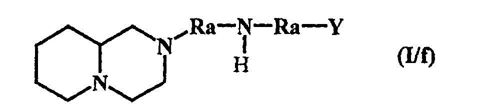
wherein Ra and Y are as defined for formula (I),
which compounds of formula (I/f) may be subjected to the action of a compound of formula (X) as described hereinbefore, to yield compounds of formula (I/g), a particular case of the compounds of formula (I) : 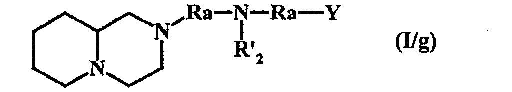 wherein Ra, R'₂ and Y are as defined hereinbefore,
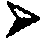 *or with a compound of formula (XII) :*
**Y-Ra-W**_{**3**}**-Ra-OTs (XII)**
wherein Y and Ra are as defined for formula (I) and W₃ represents an oxygen or sulphur atom,
to yield compounds of formula (I/h), a particular case of the compounds of formula (I) : 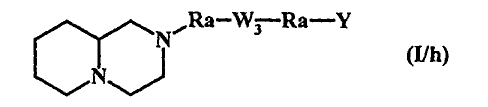 wherein Ra, Y and W₃ are as defined hereinbefore,
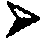 *or with a compound of formula (XIII) :* 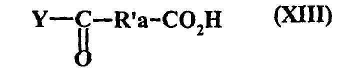 wherein Y and R'a are as defined for formula (I),
to yield compounds of formula (XIV): 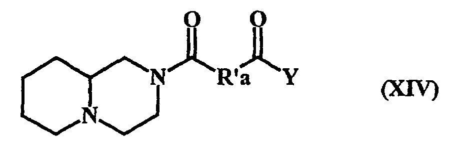 wherein R'a and Y are as defined hereinbefore,
which compounds of formula (XIV) are treated with a reducing agent to yield compounds of formula (I/i), a particular case of the compounds of formula (I) : 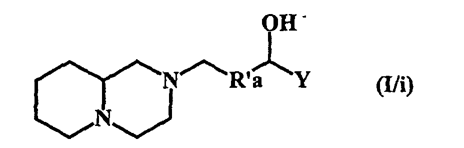 wherein R'a and Y are as defined for formula (I),
which compounds of formula (I/i), in the case when Y represents specifically a Y₂ group as described hereinbefore, are oxidised in the presence of dimethyl sulphoxide, triethylamine and oxalyl chloride, to yield compounds of formula (I/j), a particular case of the compounds of formula (I) : 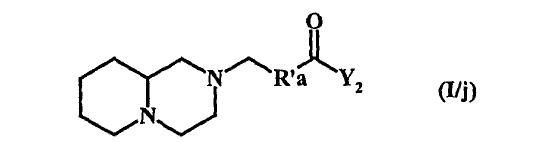 wherein R'a and Y₂ are as defined hereinbefore,
or which compound of formula (I/i) is reacted with a compound of formula (XV) :
**R'**_{**1**}**-Hal (XV)**
wherein Hal represents a halogen atom and R'₁ has the same meanings as R₁ with the exception of the definition of a hydrogen atom,
to yield compounds of formula (I/k), a particular case of the compounds of formula (I) : 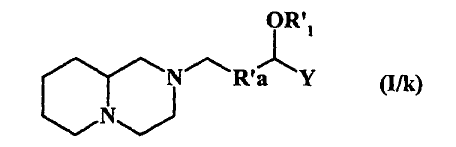 wherein R'a, R'₁ and Y are as defined hereinbefore,
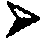 *or with a compound of formula (XVI) :*
**Hal-Ra-W**_{**2**}**H (XVI)**
wherein Hal represents a halogen atom, Ra and W₂ are as defined for formula (I),
to yield compounds of formula (XVII) : 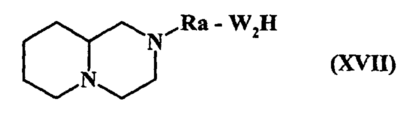 wherein Ra and W₂ are as defined hereinbefore,
which compounds of formula (XVII) are treated with a compound of formula (XVIII) : 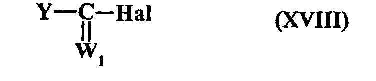 wherein Hal represents a halogen atom, Y and W₁ are as defined for formula (I) :
to yield compounds of formula (I/l), a particular case of the compounds of formula (I) : 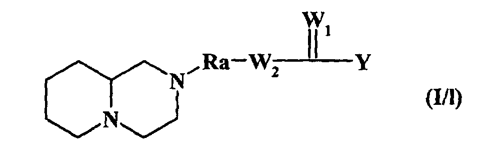 wherein Ra, W₂, W₁ and Y are as defined hereinbefore,
which compounds of formulae (I/a) to (I/l) constitute the totality of the compounds of the invention, are purified, if necessary, in accordance with conventional purification techniques, are separated, where appropriate, into their isomers in accordance with a conventional separation technique, and are converted, if desired, into addition salts thereof with a pharmaceutically acceptable acid or base.

14. Pharmaceutical compositions comprising as active ingredient at least one compound according to any one of claims 1 to 12, alone or in combination with one or more inert, non-toxic, pharmaceutically acceptable excipients or carriers.

15. Pharmaceutical compositions according to claim 14 comprising at least one active ingredient according to any one of claims 1 to 12 for use as antagonists of type H3 central histamine receptors.

16. Pharmaceutical compositions according to claim 14 comprising at least one active ingredient according to any one of claims I to 12 for use, as a medicament, in the treatment of cognitive deficiencies associated with cerebral ageing and with neurodegenerative diseases, and in the treatment of mood disorders, convulsive attacks, attention deficit hyperactivity syndrome, obesity and pain.

17. Pharmaceutical compositions according to claim 14 comprising at least one active ingredient according to any one of claims 1 to 12 for use, as a medicament, in the treatment of cognitive deficiencies associated with Alzheimer's disease, Parkinson's disease, Pick's disease, Korsakoff's disease, and frontal and sub-cortical dementia of vascular or other origin.

## Patentansprüche

1. Verbindungen der Formel (I): 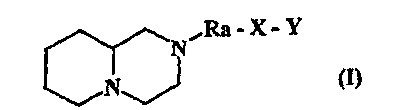 in der:
• **Ra** eine geradkettige oder verzweigte (C₁-C₆)-Alkylenkette darstellt,
• **X** eine Gruppe ausgewählt aus W₁, -C(W₁)-W₂-, -W₂-C(W₁)-, -W₂-C(W₁)W₂-, -W₂-Ra, worin Ra die oben angegebenen Bedeutungen besitzt, und -CH(OR₁)- bedeutet, worin:
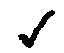 W₁ ein Sauerstoffatom, ein Schwefelatom oder eine Gruppe der Formel -NR₂ darstellt, worin R₂ eine Gruppe ausgewählt aus dem Wasserstoffatom, geradkettigen oder verzweigten (C₁-C₆)-Alkylgruppen, Arylgruppen, geradkettigen oder verzweigten Aryl-(C₁-C₆)-alkylgruppen und geradkettigen oder verzweigten (C₁-C₆)-Acylgruppen darstellt,
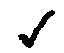 W₂ eine Gruppe bedeutet, wie sie für W₁ definiert worden ist,
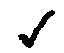 R₁ eine Gruppe ausgewählt aus dem Wasserstoffatom und geradkettigen oder verzweigten (C₁-C₆)-Alkylgruppen darstellt,
• wenn **Y** eine Aryl- oder Heteroarylgruppe darstellt,
oder
• **X** eine Gruppe ausgewählt aus der Einfachbindung, -C(W₁)-, -W₂-C(W₁)-, -W₂Ra- und -CH(OR₁-, worin W₁, W₂, Ra und R₁ die oben angegebenen Bedeutungen besitzen, darstellt,
• wenn **Y** eine kondensierte bicyclische Gruppe der Formel bedeutet: 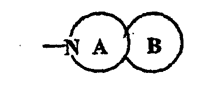 in der:
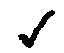 A einen ungesättigten oder teilweise gesättigten Stickstoff-haltigen Heterocyclus mit 4 bis 7 Kettengliedern bedeutet, der gegebenenfalls ein zweites Heteroatom ausgewählt aus Sauerstoff, Stickstoff und Schwefel enthält und gegebenenfalls durch eine oder mehrere Gruppen ausgewählt aus Oxo und geradkettigem oder verzweigtem (C₁-C₆)-Alkyl substituiert ist,
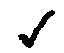 B einen Phenylring bedeutet, der gegebenenfalls substituiert ist durch eine oder mehrere Gruppen ausgewählt aus Halogenatomen, Nitrogruppen. Cyanogruppen, Hydroxygruppen, geradkettigen oder verzweigten (C₁-C₆)-Alkoxygruppen, geradkettigen oder verzweigten (C₁-C₆)-Alkylgruppen, geradkettigen oder verzweigten (C₁-C₆)-Trihalogenalkylgruppen, geradkettigen oder verzweigten (C₁-C₆)-Acylgruppen, geradkettigen oder verzweigten (C₁-C₆)-Acyloxygruppen, Carboxygruppen, geradkettigen oder verzweigten (C₁-C₆)-Alkoxycarbonylgruppen, Sulfanylgruppen, geradkettigen oder verzweigten (C₁-C₆)-Alkylsulfanylgruppen und Aminogruppen, die gegebenenfalls durch eine oder zwei gleichartige oder verschiedenartige Gruppen ausgewählt aus geradkettigem oder verzweigtem (C₁-C₆)-Alkyl, Aryl und geradkettigem oder verzweigtem Aryl-(C₁-C₆)-alkyl substituiert sind,
deren Enantiomere, Diastereoisomere sowie deren Additionssalze mit einer pharmazeutisch annehmbaren Säure oder Base,
mit der Maßgabe, daß die Verbindungen:
- 2-Octahydro-2*H*-pyrido[1,2-a]pyrazin-2-yl-1-phenylethanol,
- 3-Octahydro-2*H*-pyrido[1,2-a]pyrazin-2-yl-propyl-3,4,5-trimethoxybenzoat und
- 2-Octahydro-2*H*-pyrido[1,2-a]pyrazin-2-yl-ethyl-3,4,5-trimethoxybenzoat nicht Teil der Verbindungen der Erfindung sind,
mit der weiteren Maßgabe, daß:
• unter einer Arylgruppe ein monocyclisches oder bicyclisches aromatisches System mit 5 bis 10 Kohlenstoffatomen zu verstehen ist, das gegebenenfalls substituiert ist durch eine oder mehrere gleichartige oder verschiedene Gruppen, die unabhängig voneinander ausgewählt sind aus Halogenatomen, Nitrogruppen, Cyanogruppen, Hydroxygruppen, geradkettigen oder verzweigten (C₁-C₆)-Alkoxygruppen, Aryloxygruppen, geradkettigen oder verzweigten Aryl-(C₁-C₆)-alkoxygruppen, geradkettigen oder verzweigten (C₁-C₆)-Alkylgruppen, geradkettigen oder verzweigten (C₂-C₆)-Alkenylgruppen, gerädkettigen oder verzweigten (C₂-C₆)-Alkinylgruppen, geradkettigen oder verzweigten (C₁-C₆)-Trihalogenalkylgruppen, Carboxygruppen, geradkettigen oder verzweigten (C₁-C₆)-Alkoxycarbonylgruppen, geradkettigen oder verzweigten (C₁-C₆)-Acylgruppen, geradkettigen oder verzweigten (C₁-C₆)-Acyloxygruppen, Sulfanylgruppen, geradkettigen oder verzweigten (C₁-C₆)-Alkylsulfanylgruppen, Methylendioxygruppen, Ethylendioxygruppen, Amid-oximgruppen und Aminogruppen, die gegebenenfalls substituiert sind durch eine oder zwei gleichartige oder verschiedene Gruppen, die unabhängig voneinander ausgewählt sind aus geradkettigem oder verzweigtem (C₁-C₆)-Alkyl, Aryl, geradkettigem oder verzweigtem Aryl-(C₁-C₆)-alkyl und geradkettigem oder verzweigtem (C₁-C₆)-Acyl,
• man unter einer Heteroarylgruppe ein monocyclisches oder bicyclisches aromatisches System mit 5 bis 12 Kettengliedern versteht, das im Rahmen des cyclischen Systems 1 bis 3 gleichartige oder verschiedenartige Heteroatome aufweist, die unabhängig voneinander ausgewählt sind aus Sauerstoff-, Stickstoff- und Schwefelatomen, wobei jedes dieser Systeme gegebenenfalls durch eine oder mehrere, gleichartige oder verschiedenartige Gruppen substituiert ist, die unabhängig voneinander ausgewählt sind aus der oben beschriebenen Liste der Substituenten für die Arylgruppe,
• und man unter Isomeren die optischen Isomeren, die Enantiomeren und die Diastereoisomeren versteht.

2. Verbindungen der Formel (I) nach Anspruch 1, **dadurch gekennzeichnet, daß** Ra eine geradkettige oder verzweigte (C₂-C₅)-Alkylenkette bedeutet, deren Isomere sowie deren Additionssalze mit einer pharmazeutisch annehmbaren Säure oder Base.

3. Verbindungen der Formel (I) nach Anspruch 1, **dadurch gekennzeichnet, daß** R₂ ein Wasserstoffatom, eine geradkettige oder verzweigte (C₁-C₆)-Alkylgruppe oder eine geradkettige oder verzweigte (C₁-C₆)-Acylgruppe bedeutet, deren Isomere sowie deren Additionssalze mit einer pharmazeutisch annehmbaren Säure oder Base.

4. Verbindungen der Formel (I) nach Anspruch 1, **dadurch gekennzeichnet, daß** sie die Verbindungen der Formel (IA) darstellen: 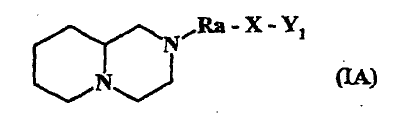 in der:
• **Ra** eine geradkettige oder verzweigte (C₁-C₆)-Alkylenkette bedeutet,
• **X** eine Gruppe ausgewählt aus W₁, -C(W₁)-W₂-, -W₂-C(W₁)-, -W₂-C(W₁)W₂-, -W₂-Ra, worin Ra die oben angegebenen Bedeutungen besitzt, und -CH(OR₁)- bedeutet, worin:
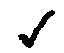 W₁ ein Sauerstoffatom, ein Schwefelatom oder eine Gruppe der Formel -NR₂ darstellt, worin R₂ eine Gruppe ausgewählt aus dem Wasserstoffatom, geradkettigen oder verzweigten (C₁-C₆)-Alkylgruppen, Arylgruppen, geradkettigen oder verzweigten Aryl-(C₁-C₆)-alkylgruppen und geradkettigen oder verzweigten (C₁-C₆)-Acylgruppen darstellt,
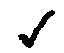 W₂ eine Gruppe bedeutet, wie sie für W₁ definiert worden ist,
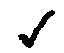 R₁ eine Gruppe ausgewählt aus dem Wasserstoffatom und geradkettigen oder verzweigten (C₁-C₆)-Alkylgruppen darstellt, und
• **Y**_{**1**} eine Aryl- oder Heteroarylgruppe bedeutet,
deren Enantiomere, Diastereoisomere sowie deren Additionssalze mit einer pharmazeutisch annehmbaren Säure oder Base.

5. Verbindungen der Formel (I) nach Anspruch 4, **dadurch gekennzeichnet, daß** X eine Gruppe ausgewählt aus dem Sauerstoffatom und den Gruppen -C(W₁)-W₂-, -W₂-C(W₁)- und -N(R₂)- bedeutet, worin W₁ ein Sauerstoffatom, W₂ ein Sauerstoffatom oder eine Gruppe -NR₂ und R₂ eine Gruppe ausgewählt aus dem Wasserstoffatom, geradkettigen oder verzweigten (C₁-C₆)-Alkylgruppen, Arylgruppen, geradkettigen oder verzweigten Aryl-(C₁-C₆)-alkylgruppen und geradkettigen oder verzweigten (C₁-C₆)-Acylgruppen darstellen, deren Isomere sowie deren Additionssalze mit einer pharmazeutisch annehmbaren Säure oder Base.

6. Verbindungen der Formel (I) nach Anspruch 4, **dadurch gekennzeichnet, daß** Y₁ eine Phenylgruppe bedeutet, die gegebenenfalls substituiert ist durch 1 bis 3 Gruppen ausgewählt aus Halogen, Cyano, Hydroxy, geradkettigem oder verzweigtem (C₁-C₆)-Alkoxy, Aryloxy, geradkettigem oder verzweigtem Aryl-(C₁-C₆)-alkoxy, geradkettigem oder verzweigtem (C₁-C₆)-Alkyl, geradkettigem oder verzweigtem (C₁-C₆)-Trihalogenalkyl, Methylendioxy und Ethylendioxy, deren Isomere sowie deren Additionssalze mit einer pharmazeutisch annehmbaren Säure oder Base.

7. Verbindungen der Formel (I) nach Anspruch 1, **dadurch gekennzeichnet, daß** sie die Verbindungen der Formel (IB) darstellen: 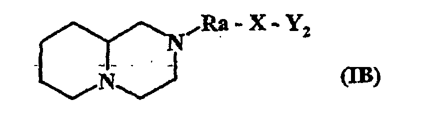 in der:
• **Ra** eine geradkettige oder verzweigte (C₁-C₆)-Alkylenkette bedeutet,
• **X** eine Gruppe ausgewählt aus der Einfachbindung, -C(W₁)-, -W₂-C(W₁)-, -W₂-Ra- und -CH(OR₁)- darstellt, worin W₁, W₂, Ra und R₁ die bezüglich der Formel (I) angegebenen Bedeutungen besitzen, und
• **Y**_{**2**} eine Gruppe der Formel: 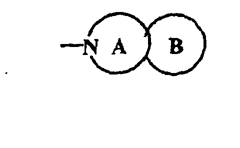 darstellt, in der A und B die bezüglich der Formel (I) angegebenen Bedeutungen besitzen,
deren Enantiomere, Diastereoisomere sowie deren Additionssalze mit einer pharmazeutisch annehmbaren Säure oder Base.

8. Verbindungen der Formel (I) nach Anspruch 7, **dadurch gekennzeichnet, daß** Y₂ eine Gruppe der Formel darstellt: 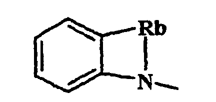 in der Rb eine geradkettige gesättigte oder ungesättigte lineare Kette bedeutet, die 2 oder 3 Atome ausgewählt aus Kohlenstoff, Stickstoff und Sauerstoff enthält und/oder die gegebenenfalls eine Carbonylgruppe enthält, deren Isomere sowie deren Additionssalze mit einer pharmazeutisch annehmbaren Säure oder Base.

9. Verbindungen der Formel (I) nach Anspruch 7, **dadurch gekennzeichnet, daß** Y₂ eine Gruppe ausgewählt aus 2,3-Dihydro-1*H*-indol-1-yl, 1*H*-Indol-1-yl, 1*H*-Indazol-1-yl, 1*H*-Benzimidazol-1-yl, 3,4-Dihydro-2*H*-chinolin-1-yl, 2,3-Dihydro-1*H*-4-chinolinon-1-yl, 3,4-Dihydro-2*H*-chinoxalin-1-yl und 2,3-Dihydro-4*H*-1,4-benzoxazin-4-yl darstellt, deren Isomere sowie deren Additionssalze mit einer pharmazeutisch annehmbaren Säure oder Base.

10. Verbindungen der Formel (I) nach Anspruch 7, **dadurch gekennzeichnet, daß** X eine Einfachbindung darstellt, deren Isomere sowie deren Additionssalze mit einer pharmazeutisch annehmbaren Säure oder Base.

11. Verbindungen der Formel (I) nach Anspruch 1, nämlich:
- 2-[4-(2,3-Dihydro-1*H*-indol-1-yl)-butyl]-octahydro-2*H*-pyrido[1,2-*a*]pyrazin,
- 2-[4-(3,4,5-Trimethoxyphenoxy)-butyl]-octahydro-2*H*-pyrido[1,2-*a*]pyrazin,
- 2-[4-(3,4,5-Trimethoxyphenoxy)-butyl]-octahydro-2*H*-pyrido[1,2-*a*]pyrazin, Enantiomeres α.
- 2-[4-(3,4,5-Trimethoxyphenoxy)-butyl]-octahydro-2*H*-pyrido[1,2-*a*]pyrazin, Enantiomeres β,
- N-(4-Octahydro-2*H*-pyrido[1,2-*a*]pyrazin-2-ylbutyl)-*N*-(3,4,5-trimethoxyphenyl)-amin,
- *N*-(4-Octahydro-2*H*-pyrido[1,2-*a*]pyrazin-2-ylbutyl)-*N*-(3,4,5-trimethoxyphenyl)-amin. Enantiomeres α,
- *N*-(4-Octahydro-2*H*-pyrido[1,2-*a*]pyrazin-2-ylbutyl)-*N*-(3,4,5-trimethoxyphenyl)-amino, Enantiomeres β,
- *N*-(4-Trifluorphenyl)-*N*-(4-octahydro-2*H*-pyrido[1,2-*a*]pyrazin-2-ylbutyl)-amin,
- *N*-[3,4-Dichlor-phenyl)-*N*-(4-octahydro-2*H*-pyrido[1,2-*a*]pyrazin-2-ylbutyl)-amin,
- *N*-(3,5-Dichlor-phenyl)-*N*-(4-octahydro-2*H*-pyrido[1,2-*a*]pyrazin-2-ylbutyl)-amin,
- *N*-(2-Chlorphenyl)-*N*-(4-octahydro-2*H*-pyrido[1,2-*a*]pyrazin-2-ylbutyl)-amin,
- 2-[4-(3,4-Dihydro-1(2*H*)-chinolinyl)-butyl]-octahydro-2*H*-pyrido[1,2-*a*]pyrazin,
- 2-[4-(1*H*-Benzimidazol-1-yl)-butyl]-octahydro-2*H*-pyrido[1,2-*a*]pyrazin,
- 2-[4-(1*H*-Indol-1-yl)-butyl]-octahydro-2*H*-pyrido[1,2-*a*]pyrazin,
- 2-[4-[1*H*-Indazol-1-yl)-butyl]-octahydro-2*H*-pyrido[1,2-*a*]pyrazin,
- 2-[4-(2,3-Dihydro-1*H*-indol-1-yl)-butyl]-octahydro-2*H*-pyrido[1,2-*a*]pyrazin, Enantiomeres α,
- 2-[4-(2,3-Dihydro-1H-indol-1-yl)-butyl)-octahydro-2*H*-pyrido[1,2-*a*]pyrazin, Enantiomeres β,
- 3-(4-Octahydro-2*H*-pyrido[1,2-*a*]pyrazin-2-ylbutoxy)-benzonitril,
- 3-Methoxy-4-(4-octahydro-2*H*-pyrido[1,2-*a*]pyrazin-2-ylbutoxy)-benzonitril,
- 2-[4-(2,3,4-Trimethoxyphenoxy)-butyl]-octahydro-2*H*-pyrido[1,2-*a*]pyrazin,
deren Enantiomere, Diastereoisomere sowie deren Additionssalze mit einer pharmazeutisch annehmbaren Säure oder Base.

12. Verbindungen der Formel (I) nach Anspruch 1, nämlich:
- 4-[(4-Octahydro-2*H*-pyrido[1,2-*a*]pyrazin-2-ylbutyl)-amino]-benzonitril,
- 4-(3-Octahydro-2*H*-pyrido[1,2-*a*]pyrazin-2-ylpropoxy)-benzonitril,
- 4-(3-Octahydro-2*H*-pyrido[1,2-*a*]pyrazin-2-ylpropoxy)-benzonitril, Enantiomeres α,
- 4-(3-Octahydro-2*H*-pyrido[1,2-*a*]pyrazin-2-ylpropoxy)-benzonitril, Enantiomeres β,
- 4-(4-Octahydro-2*H*-pyrido[1,2-*a*]pyrazin-2-ylbutoxy)-benzonitril,
- 4-(4-Octahydro-2*H*-pyrido[1,2-*a*]pyrazin-2-ylbutoxy)-benzonitril, Enantiomeres α,
- 4-(4-Octahydro-2*H*-pyrido[1,2-*a*]pyrazin-2-ylbutoxy)-benzonitril, Enantiomeres β,
- 4-(2-Octahydro-2*H*-pyrido[1,2-*a*]pyrazin-2-ylethoxy)-benzonitril,
deren Enantiomere, Diastereoisomere sowie deren Additionssalze mit einer pharmazeutisch annehmbaren Säure oder Base.

13. Verfahren zur Herstellung der Verbindungen der Formel (I) nach Anspruch 1, **dadurch gekennzeichnet, daß** man als Ausgangsprodukt eine Verbindung der Formel (II) verwendet: 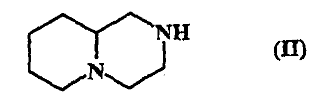 welche Verbindung der Formel (II) man unter basischen Bedingungen:
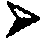 *entweder mit einer Verbindung der Formel (III)* umsetzt: 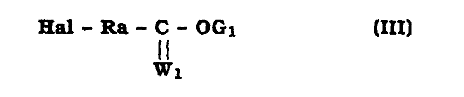 in der Hal ein Halogenatom bedeutet, Ra die bezüglich der Formel (I) angegebenen Bedeutungen besitzt und G₁ eine geradkettige oder verzweigte (C₁-C₆)-Alkylgruppe darstellt,
zur Bildung der Verbindungen der Formel (IV): 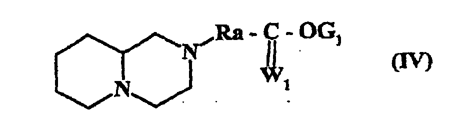 in der Ra und G₁ die oben angegebenen Bedeutungen besitzen,
welche Verbindungen der Formel (IV) verseift und dann in Gegenwart von 1-(3-Dimethylaminopropyl)-3-ethylcarbodiimid-Hydrochlorid und eines tertiären Amins mit einer Verbindung der Formel (V) umgesetzt werden:
**Y**_{**1**} **- W**_{**2**}**H (V)**
in der Y₁ eine Arylgruppe oder Heteroarylgruppe bedeutet und W₂ die bezüglich der Formel (I) angegebenen Bedeutungen besitzt,
zur Bildung der Verbindungen der Formel (I/a), einem Sonderfall der Verbindungen der Formel (I): 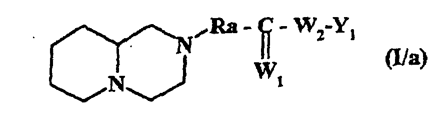 in der Ra, W₁, W₂ und Y₁ die oben angegebenen Bedeutungen besitzen,
welche Verbindungen der Formel (I/a) in dem besonderen Fall, da W₁ ein Sauerstoffatom bedeutet und Ra die besondere Definition R'a besitzt und eine geradkettige oder verzweigte (C₁-C₅)-Alkylenkette bedeutet, in selektiver Weise durch Einwirkung eines klassischen, in der organischen Synthese verwendeten Reduktionsmittels reduziert werden können zur Bildung der Verbindungen der Formel (I/b), einem Sonderfall der Verbindungen der Formel (I): 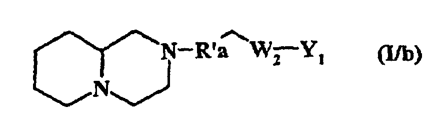 in der R'a eine geradkettige oder verzweigte (C₁-C₅)-Alkylenkette darstellt und W₂ und Y₁ die oben angegebenen Bedeutungen besitzen,
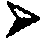 *oder mit einer Verbindung der Formel (VI)* umsetzt:
**Ha1 - Ra**_{**1**} **- CN (VI)**
in der Hal ein Halogenatom und Ra₁ eine geradkettige oder verzweigte (C₁-C₅)-Alkylgruppe oder eine Bindung bedeuten,
zur Bildung der Verbindungen der Formel (VII): 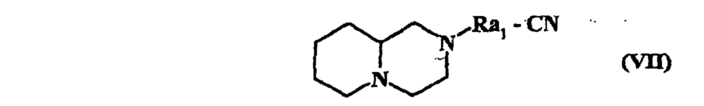 in der Ra₁ die oben angegebenen Bedeutungen besitzt,
bei welchen Verbindungen der Formel (VII) man die Cyanofunktion unter klassischen Bedingungen zu dem primären Amin reduziert und sie dann:
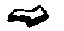 entweder in Ethanol in Gegenwart einer Verbindung der Formel (VIII) umsetzt:
**Y**_{**1**} **- N = C = W**_{**3**} **(VIII)**
in der Y₁ die oben angegebenen Bedeutungen besitzt und W₃ ein Sauerstoffatom oder ein Schwefelatom darstellt,
zur Bildung der Verbindungen der Formel (I/c), einem Sonderfall der Verbindungen der Formel (I): 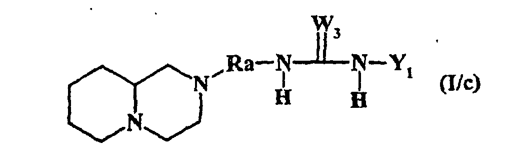 in der Ra, Y₁ und W₃ die oben angegebenen Bedeutungen besitzen,
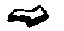 oder unter Kupplungsbedingungen mit einer Verbindung der Formel (IX) umsetzt: 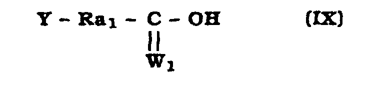 in der Y und W₁ die bezüglich der Formel (I) angegebenen Bedeutungen besitzen und Ra₁ die oben angegebenen Bedeutungen aufweist,
zur Bildung:
• in dem Fall, da Ra₁ eine Bindung darstellt, der Verbindungen der Formel (I/d), einem Sonderfall der Verbindungen der Formel (I): 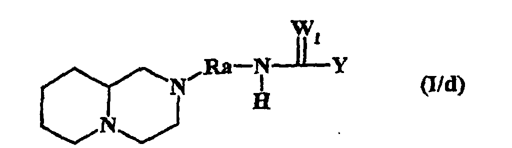 in der Ra, W₁ und Y die oben angegebenen Bedeutungen besitzen,
welche Verbindungen der Formel (I/d) man in Gegenwart von Natriumhydrid mit einer Verbindung der Formel (X) behandelt:
**R'**_{**2**} **- Hal (X)**
in der Hal ein Halogenatom darstellt und R'₂ die gleichen Bedeutungen besitzt wie R₂ in der Formel (I) mit Ausnahme der Bedeutung des Wasserstoffatoms,
zur Bildung der Verbindungen der Formel (I/e), einem Sonderfall der Verbindungen der Formel (I): 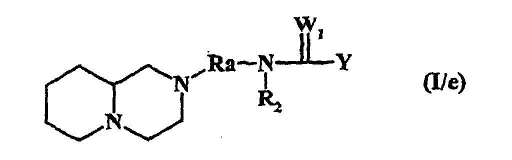 in der Ra, R₂, W₁ und Y die oben angegebenen Bedeutungen besitzen.
• oder in dem Fall, da Ra₁ eine geradkettige oder verzweigte (C₁-C₅)-Alkylenkette darstellt, der Verbindungen der Formel (XI): 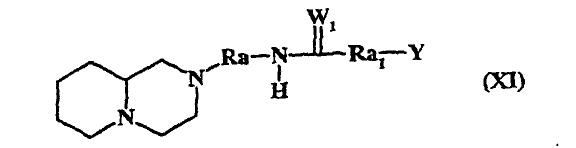 welche Verbindungen der Formel (XI) mit einem Reduktionsmittel, wie es in der klassischen organischen Synthese verwendet wird, behandelt werden zur Bildung der Verbindungen der Formel (I/f), einem Sonderfall der Verbindungen der Formel (I): 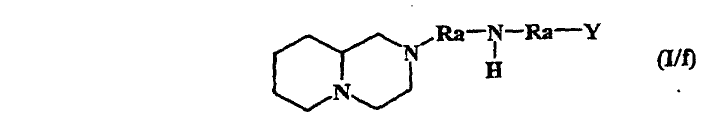 in der Ra und Y die bezüglich der Formel (I) angegebenen Bedeutungen besitzen,
welche Verbindungen der Formel (I/f) der Einwirkung einer Verbindung der Formel (X), wie sie oben definiert worden ist, unterworfen werden können zur Bildung der Verbindungen der Formel (I/g), einem Sonderfall der Verbindungen der Formel (I): 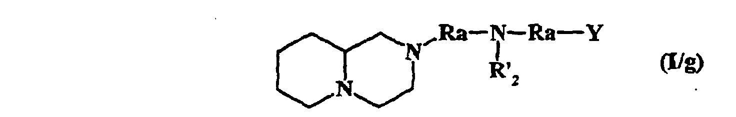 in der Ra, R'₂ und Y die oben angegebenen Bedeutungen besitzen,
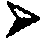 *oder mit einer Verbindung der Formel (XII)* umsetzt:
**Y - Ra - W**_{**3**} **- Ra - OTs (XII)**
in der Y und Ra die bezüglich der Formel (I) angegebenen Bedeutungen besitzen und W₃ ein Sauerstoffatom oder ein Schwefelatom darstellt,
zur Bildung der Verbindungen der Formel (I/h), einem Sonderfall der Verbindungen der Formel (I): 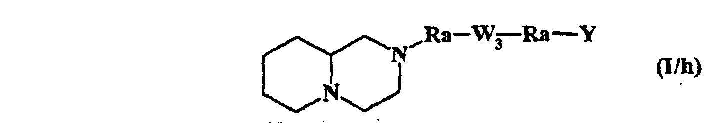 in der Ra, Y und W₃ die oben angegebenen Bedeutungen besitzen,
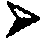 *oder mit einer Verbindung der Formel (XIII) umsetzt:* 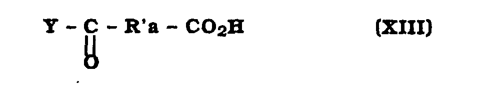 in der Y und R'a die bezüglich der Formel (I) angegebenen Bedeutungen besitzen,
zur Bildung der Verbindungen der Formel (XIV): 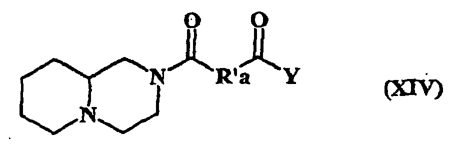 in der R'a und Y die oben angegebenen Bedeutungen besitzen,
welche Verbindungen der Formel (XIV) mit einem Reduktionsmittel behandelt werden zur Bildung der Verbindungen der Formel (I/i), einem Sonderfall der Verbindungen der Formel (I): 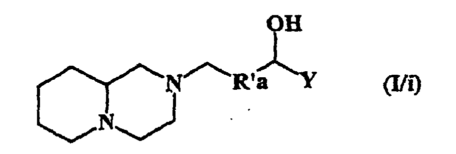 in der R'a und Y die bezüglich der Formel (I) angegebenen Bedeutungen besitzen,
welche Verbindungen der Formel (I/i) dann, wenn Y spezifisch eine Gruppe Y₂ darstellt, wie sie oben beschrieben worden ist, in Gegenwart von Dimethylsulfoxid, Triethylamin und Oxalylchlorid oxidiert werden zur Bildung der Verbindungen der Formel (I/j), einem Sonderfall der Verbindungen der Formel (I): 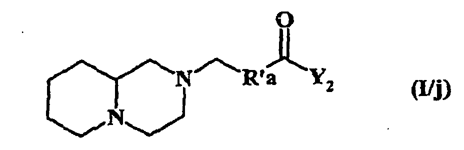 in der R'a und Y₂ die oben angegebenen Bedeutungen besitzen,
oder man die Verbindung der Formel (I/i) mit einer Verbindung der Formel (XV) umsetzt:
**R'**_{**1**} **- Hal (XV)**
in der Hal ein Halogenatom darstellt und R'₁ die Bedeutungen von R₁ mit Ausnahme der Definition des Wasserstoffatoms besitzt,
zur Bildung der Verbindungen der Formel (I/k), einem Sonderfall der Verbindungen der Formel (I): 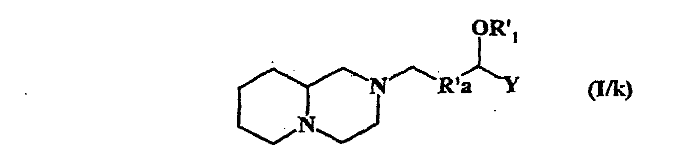 in der R'a, R'₁ und Y die oben angegebenen Bedeutungen besitzen,
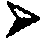 *oder mit einer Verbindung der Formel (XVI) umsetzt:*
**Hal - Ra - W**_{**2**}**H (XVI)**
in der Hal ein Halogenatom bedeutet und Ra und W₂ die bezüglich der Formel (I) angegebenen Bedeutungen besitzen,
zur Bildung der Verbindungen der Formel (XVII): 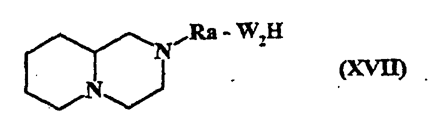 in der Ra und W₂ die oben angegebenen Bedeutungen besitzen,
welche Verbindungen der Formel (XVII) mit einer Verbindung der Formel (XVIII) behandelt werden: 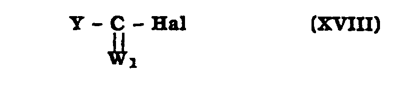 in der Hal ein Halogenatom darstellt und Y und W₁ die bezüglich der Formel (I) angegebenen Bedeutungen besitzen,
zur Bildung der Verbindungen der Formel (I/l), einem Sonderfall der Verbindungen der Formel (I): 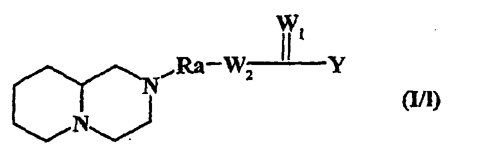 in der Ra, W₂, W₁ und Y die oben angegebenen Bedeutungen besitzen,
wobei die Verbindungen der Formeln (I/a) bis (I/l) die Gesamtheit der erfindungsgemäßen Verbindungen bilden, welche man gegebenenfalls mit Hilfe klassischer Reinigungsmethoden reinigt, gegebenenfalls mit Hilfe einer klassischen Trennungsmethode in ihre Isomeren trennt und gewünschtenfalls in ihre Additionssalze mit einer pharmazeutisch annehmbaren Säure oder Base umwandelt.

14. Pharmazeutische Zubereitungen enthaltend als Wirkstoff mindestens eine Verbindung nach einem der Ansprüche 1 bis 12 allein oder in Kombination mit einem oder mehreren inerten, nichttoxischen, pharmazeutisch annehmbaren Trägermaterialien oder Bindemitteln.

15. Pharmazeutische Zubereitungen nach Anspruch 14 enthaltend mindestens einen Wirkstoff nach einem der Ansprüche 1 bis 12 nützlich als Antagonisten der histaminergischen zentralen Rezeptoren des Typs H3.

16. Pharmazeutische Zubereitungen nach Anspruch 14 enthaltend mindestens einen Wirkstoff nach einem der Ansprüche 1 bis 12 nützlich als Arzneimittel zur Behandlung von Erkenntnismängeln, die mit dem Altern des Gehirns und mit neurodegenerativen Erkrankungen verknüpft sind, sowie zur Behandlung von Störungen des Gemüts, von Krampfkrisen, des Hyperaktivitätssyndroms mit Aufmerksamkeitsdefiziten, der Fettsucht und von Schmerzen.

17. Pharmazeutische Zubereitungen nach Anspruch 14 enthaltend mindestens einen Wirkstoff nach einem der Ansprüche 1 bis 12 nützlich als Arzneimittel bei der Behandlung von Erkenntnismängeln, die mit der Alzheimerschen Krankheit, der Parkinsonschen Krankheit, der Pickschen Krankheit, der Korsakoff-Krankheit, frontalen und subkortikalen Dementien mit vaskulärem oder andersartigem Ursprung verknüpft sind.
